(19) Europäisches Patentamt
European Patent Office
Office européen des brevets

(11) EP 2 128 136 A1

(12) EUROPEAN PATENT APPLICATION
published in accordance with Art. 153(4) EPC

(43) Date of publication:
**02.12.2009 Bulletin 2009/49**

(21) Application number: **07850498.2**

(22) Date of filing: **12.12.2007**

(51) Int Cl.:
***C07D 213/80*** (2006.01) ***A61K 31/455*** (2006.01)
***A61K 31/55*** (2006.01) ***A61P 3/06*** (2006.01)
***A61P 19/06*** (2006.01) ***A61P 43/00*** (2006.01)
***C07D 401/10*** (2006.01) ***C07D 413/10*** (2006.01)

(86) International application number:
**PCT/JP2007/073947**

(87) International publication number:
**WO 2008/072658 (19.06.2008 Gazette 2008/25)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **12.12.2006 JP 2006334021**

(71) Applicant: **Nippon Zoki Pharmaceutical Co., Ltd.**
**Osaka-shi**
**Osaka 541-0046 (JP)**

(72) Inventors:
- **MENJO, Nobuo**
**Kato-shi**
**Hyogo 673-1461 (JP)**
- **OOKUBO, Tomohiro**
**Kato-shi**
**Hyogo 673-1461 (JP)**
- **HASEGAWA, Taisuke**
**Kato-shi**
**Hyogo 673-1461 (JP)**
- **FURUKAWA, Kazuhito**
**Kato-shi**
**Hyogo 673-1461 (JP)**
- **FUJITA, Akihiro**
**Kato-shi**
**Hyogo 673-1461 (JP)**

(74) Representative: **HOFFMANN EITLE**
**Patent- und Rechtsanwälte**
**Arabellastrasse 4**
**81925 München (DE)**

# (54) 2-PHENYLNICOTINIC ACID DERIVATIVE

(57) The present invention is to provide the compounds useful as a treating or preventing agent for gout and hyperuricemia which are 2-phenylnicotinic acid derivatives having a uric acid lowering action due to an excellent xanthine oxidase inhibitory action. Since the 2-phenylnicotinic acid derivatives of the present invention exhibit a uric acid lowering action due to an excellent xanthine oxidase inhibitory action and also hypolipemic action, their utility is very high as a treating or preventive agent for gout and hyperuricemia which are often accompanied by hyperlipemia as a complication.

EP 2 128 136 A1

## Description

### [Technical Field]

The present invention relates to a novel 2-phonylnicotinic acid derivative and pharmaceutically-acceptable salt and hydrate thereof and also relates to a drug containing said compound as an effective ingredient.

[Background Art]

Gout is a disease which is specific to humans and often appears in adult males where hyperuricemia is a basal disease and acute arthritis, tophus, urinary calculus and renal interstitial tissue and blood vessel lesion caused by separation of urate are main symptoms. With regard to the treatment for gout, colchicine and nonsteroidal anti-inflammatory drug are used for onset of acute arthritis and, after remission of the attack, an improving therapy for hyperuricemia is conducted. Treating agents for hyperuricemia are roughly classified into a promoter for discharge of uric acid and an inhibitor for synthesis of uric acid and they are appropriately selected depending upon condition and degree of the disease. However, since the promoter for uric acid discharge decreases its action when renal function lowers, the inhibitor for uric acid synthesis is advantageously used for the patient where renal function lowers. The inhibitor for uric acid synthesis inhibits xanthine oxidase which is an enzyme participating in biosynthesis of uric acid and is an effective drug for the treatment of hyperuricemia and various diseases caused thereby. However, the inhibitor which is put to practical use in clinical parctice at present is allopurinol only.

On the other hand, patients suffering from hyperuricemia which is a basal disease for gout often have complications of lifestyle-related illness such as obesity, hypertension or hyperlipemia. Therefore, a way of thinking for a total control for reducing the risk factors for cardiovascular diseases grasping the gout as a lifestyle-related illness including the complications as such is now spreading in clinical site. Particularly in the patients suffering from hyperuricemia, complication with hyperlipemia is as very high as about 60% and death due to arteriosclerotic disease is increasing. Thus, according to the "Guideline for the management of hyperuricemia and gout" (2002) published by the Japanese Society of Gout and Nucleic Acid Metabolism, fenofibrate having a lowering action for uric acid level in serum is recommended as a drug therapy for the complicated hyperlipemia. However, since the hypouricemic effectof the fenofibrate is based on a promoting action for uric acid discharge, use of fenofibrate to patients where renal function lowers is awkward. Accordingly, there has been a brisk demand for a drug which is an inhibitor for xanthine oxidase which is able to be used at ease even for patients where renal function lowers and also has a hypolipemic action or particularly a lowering action for triglycerides.

Pyridine derivatives having the similar pharmacological action to the compound of the present invention are mentioned in the following documents but each and any of them is different from the compound of the present invention in terms of the position of carboxyl group substituted at a pyridine ring in the mother nucleus site. For example, in Non-Patent Document 1, it is mentioned that 2-phenylisonicotinic acid has a discharging action for uric acid and is useful for the treatment of hyperuricemia but there is no description therein for an inhibitory action on xanthine oxidase, an inhibitory action on uric acid synthesis and a hypolipemic action. In Patent Document 1, a 2-phenylpyridine derivative is mentioned as an inhibitor for xanthine oxidase but there is neither disclosure nor suggestion at all that it has a hypolipemic action as the compound of the present invention has.

Patent Document 1: Gazette of International Publication WO 2006/0223374
Non-Patent Document 1: Annali di Chimica Applicata, volume 21, pages 553 to 558 (1931).

### [Disclosure of the Invention]

### [Problems that the Invention is to Solve]

An object of the present invention is to provide a novel compound which has an excellent inhibitory action for xanthine oxidase together with a hypolipemic action and is useful as a treating or preventive agent for hyperuricemia and gout and also to provide a drug containing the same.

### [Means for Solving the Problems]

The present inventors have carried out intensive studies for finding a compound having not only an inhibitor action for xanthine oxidase but also a hypolipemic acid or, particularly, a lowering action on triglycerides. As a result, they have found that a 2-phenylnicotinic acid derivative represented by the following structure formula (I) is a compound

useful as a drug having a hypouricemic action due to an excellent inhibitory action on xanthine oxidase and also having a hypolipemic action whereby the present invention has been achieved.

**[Advantages of the Invention]**

Since the 2-phenylnicotinic acid derivative of the present invention has a hypouricezuic action due to an excellent inhibitory action for xanthine oxidase and also has a hypolipemic action, its usefulness is very high as a therapeutic or preventive agent for hyperuricemia or gout where hyperlipemia is often accompanied therewith as a complication.

**[Best Mode for Carrying Out the Invention]**

The present invention relates to a 2-phenylnicotinic acid derivative represented by the following formula (I) and pharmaceutically-acceptable salt and hydrate thereof and also relates to a drug containing said compound as an effective ingredient.

[chem. 1]

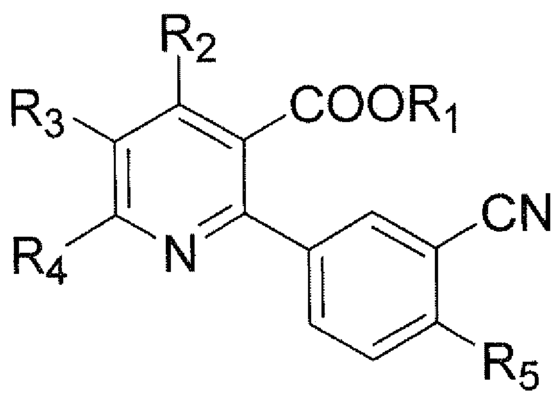

(I)

[In the formula, $R_1$, $R_2$ and $R_4$ are same or different and each is hydrogen or an alkyl group having 1 to 4 carbon(s); $R_3$ is hydrogen or halogen; $R_5$ is an azepanyl group, an amino group which is substituted with one or two alkyl group (s) having 1 to 4 carbon(s) or -O-X; and X is a substituent selected from the following (a) to (h).

(a) an alkyl group having 1 to 10 carbons(s),
(b) an alkyl group having 5 to 8 carbons and forming a saturated hydrocarbon ring having 3 to 6 carbons (which may have a phenyl group),
(c) an alkyl group having 1 to 4 carbon(s) which is substituted with a cycloalkyl group having 3 to 6 carbons,
(d) a phenyl-$C_{1-5}$ alkyl group which is optionally substituted with trifluoromethyl group, an alkyl group having 1 to 4 carbon(s), an alkoxy group having 1 to 4 carbon(s), halogen, methanesulfonyloxy group, nitro group, fluorophenyl group and/or hydroxyl group,
(e) a phenoxy-$C_{1-5}$ alkyl group which is optionally substituted with trifluoromethoxy group, phenoxy group and/or halogen,
(f) an oxazolyl-$C_{1-5}$ alkyl group which is substituted with an alkyl group having 1 to 4 carbon(s) and phenyl group,
(g) a benzoylamino-$C_{1-5}$ alkyl group which is substituted with trifluoromethoxy group or halogen and
(h) an amino alkyl group which is substituted with pyridyl group and an alkyl group having 1 to 4 carbon(s).

In the above-mentioned substituents for the formula (I), the alkyl group is a linear or branched alkyl group and the alkoxy group is a linear or branched alkoxy group. Examples of the alkyl group having 5 to 8 carbons and forming a saturated hydrocarbon ring having 3 to 6 carbons include 1-methylcyclopropylmethyl, 1-ethylcyclopentylmethyl and 1-mothylcyclopentylmethyl as well as those having a phenyl group such as 1-phenylethylcyclohexylmethyl.

Preferred compounds of the present invention are indicated as follows.

2-(3-Cyano-4-methoxyphenyl)nicotinic acid [Example 43]
2-(3-Cyano-4-isobutoxyphenyl)nicotinic acid [Example 44]

2-(3-Cyano-4-cyclopropylmethoxyphenyl)nicotinic acid [Example 45]
2-(3-Cyano-4-cyclobutylmethoxyphenyl)nicotinic acid [Example 46]
2-(3-Cyano-4-cyclohexylmethoxyphenyl)nicotinic acid [Example 47]
2-[3-Cyano-4-(2-cyclohexylethoxy)phenyl]nicotinic acid [Example 48]
2-(3-Cyano-4-hexyloxyphenyl)nicotinic acid [Example 49]
2-[3-Cyano-4-(4-trifluoromethylbenzyloxy)phenyl]nicotinic acid [Example 50]
2-(3-Cyano-4-phenethyloxyphenyl)nicotinic acid [Example 51]
2-[3-Cyano-4-(4-methylphenethyloxy)phenyl]meotimc acid [Example 52]
2-[3-Cyano-4-(4-methoxyphenethyloxy)phenyl]nicotinic acid [Example 53]
2-[4-(4-Chlorophenethyloxy)-3-cyanophenyl]nicotinic acid [Example 54]
2-{3-Cyano-4-[2-(4-trifluoromethylbenzyloxy)phenyl]nicotinic acid [Example 55]
2-[3-Cyano-4-(2-trifluoromethylphenyl)othoxy]phenyl}nicotinic acid [Example 56]
2-[3-Cyano-4-(2-fluoro-4-trifluoromethylphenethyloxy)phenyl]nicotinic acid [Example 57]
2-{3-Cyano-4-[2-(4'-fluorobiphenyl-4-yl)ethoxy]phenyl}nicotinic acid [Example 58]
2-{3-Cyano-4-[3-(4-trifluoromethylphenyl)propoxy]phenyl}nicotinate [Example 59]
2-{3-Cyano-4-[3-(2-fluorphenyl)propoxy]phenyl}nicotinic acid [Example 60]
2-{3-Cyano-4-[3-(4-fluorophenyl)propoxy]phenyl}nieotinie acid [Example 61]
2-[3-Cyano-4-(4-phenylbutoxy)phenyl]nicotinic acid [Example 62]
2-{3-Cyano-4-[4-(4-trifluoromethylphenyl)butoxy]phenyl}nicotinic acid [Example 63]
2-{3-Cyano-4-[4-(2-fluorophenyl)butoxy]phenyl}nicotinic acid [Example 64]
2-[3-Cyano-4-(3-phenoxypropoxy)phenyl]nicotinic acid [Example 65]
2-{3-Cyano-4-[3-(2-fluorophenoxy)propoxy]phenyl}nicotinic acid [Example 66]

2-{3-Cyano-4-[3-(3-fluorophenoxy)propoxy]phenyl}nicotinic acid [Example67]
2-{3-Cyano-4-[3-(4-fluorophenoxy)propoxy]phenyl}nicotinic acid [Example 68]
2-{4-[3-(2-Chloro-4-trifluoromethoxyphenoxy)propoxy]-3-cyanophenyl}nicotin ic acid [Example 69]
2-{4-[3-(2,6-Dichloro-4-trifluoromethoxyphenoxy)propoxy]-3-cyanophenyl}nic otinic acid [Example 70]
2-{3-Cyano-4-[3-(4-phenoxyphenoxy)propoxy]phenyl}nicotinic acid [Example 71]
2-[3-Cyano-4-(2,2-dimethylbutoxy)phenyl]nicotinic acid [Example 72]
2-[3-Cyano-4-(2,2-dimethylhexyloxy)phenyl]nicotinic acid [Example 73]
2-[3-Cyano-4-(1-methylcyclopropylmethoxy)phenyl]nicotinic acid [Example 74]
2-(3-Cyano-4-cyclopentylmethoxyphenyl)nicotinic acid [Example 75]
2-[3-Cyano-4-(1-ethylcyclopentylmethoxy)phenyl]nicotinic acid [Example 76]
2-[3-Cyano-4-(1-methylcyclohexylmethoxy)phenyl]nicotinic acid [Example 77]
2-[3-Cyano-4-(2-methyl-2-phenylpropoxy)phenyl]nicotinic acid [Example 78]
2-[3-Cyano-4-(1,1-dimethyl-2-phenylethoxy)phenyl]nicotinic acid [Example 79]
2-[3-Cyano-4-(2,2-dimethyl-3-phenylpropoxy)phenyl]nicotinic acid [Example 80]
2-[3-Cyano-4-(2,2-dimethyl-4-phenylbutoxy)phenyl]nicotinic acid [Example 81]
2-[3-Cyano-4-(1-phenylethylcyclohexylmethoxy)phenyl]nicotinic acid [Example 82]
2-[3-Cyano-4-(2,2-dimethylpropoxy)phenyl]nicotinic acid [Example 83]
2-[3-Cyano-4-(3,3-dimethylbutoxy)phenyl]nicotinic acid [Example 84]
2-{3-Cyano-4-[2-(4-methanesulfonyloxyphenyl)ethoxy]phenyl}nicotinic acid [Example 86]
2-[3-Cyano-4-[3-(3,5-di-tert-butyl-4-hydroxyphenyl)propoxy]phenylnicotinic acid [Example 88]
2-{3-Cyano-4-[4-(3,5-di-tert-butyl-4-hydroxyphenyl)butoxy]phenyl}nicotinic acid [Example 90]
2-{3-Cyano-4-[2-(4-trifluoromethylphenyl)ethoxy]phenyl}-6-methylnicotinic acid [Example 92]
2-{3-Cyano-4-[2-(4-trifluoromethylphenyl)ethoxy]phenyl}-4-methylnicotinic acid [Example 94]
2-{3-Cyano-4-[2-(5-methyl-2-phenyloxazol-4-yl)ethoxy]phenyl}nicotinic acid [Example 96]

2-{3-Cyano-4-[2-(methylpyridin-2-ylamino)ethoxy]phenyl}nicotinic acid [Example 98]
2-{4-[2-(2-Chlorophenyl)ethoxy]-3-cyanophenyl}nicotinic acid [Example 100]
2-[3-Cyano-4-(2-methylphenethyloxy)- phenyl]nicotinic acid [Example 102]
2-{3-Cyano-4-[2,2-dimethyl-3-(4-methoxyphenyl)propoxy]phenyl}nicotinic acid [Example 104]
2-{3-Cyano-4-[2,2-dimethyl-3-(4-trifluoromethylphenyl)propoxy]phenyl}nicot inic acid [Example 106]
2-{4-[2-(2-Nitrophenyl)ethoxy]-3-cyanophenyl}nicotinic acid [Example 108]

2-[3-Cyano-4-(2-methoxyphenethyloxy)phenyl]nicotinic acid [Example 110]
2-{3-Cyano-4-[2,2-dimethyl-3-(2-methoxyphenyl)propoxy]phenyl}mcotinic acid [Example 112]
2-{3-Cyano-4-[2-methyl-2-(4-trifluoromethylphenyl)propoxy]phenyl}nicotinic acid [Example 114]
2-[3-Cyano-4-(3-phenylpropoxy)phenyl]nicotinic acid [Example 116]
2-{3-Cyano-4-[3-(2-trifluoromethylphenyl)propoxy]phenyl}nicotinic acid [Example 118]
2-{3-Cyano-4-[3-(4-methoxyphenyl)propoxy]phenyl}nicotinic acid [Example 120]
2-{3-Cyano-4-[2-methyl-2-(4-methoxyphenyl)propoxy]phenyl}nicotinic acid [Example 122]
2-[3-Cyano-4-(4-t-butylphenethyloxy)phenyl]nicotinic acid [Example 124]
2-{3-Cyano-4-[4-(2-trifluoromethylphenyl)butoxy]phenyl}nicotinic acid [Example 126]
2-[3-Cyano-4-(3,4-dimethoxyphenethyloxy)phenyl]nicotinic acid [Example 128]
2-[3-Cyano-4-(2,5-dimethylphenethyloxy)phenyl]nicotinic acid [Example 130]
2-{3-Cyano-4-[2,2-dimethyl-3-(2-trifluoromethylphenyl)propoxy]phenyl}nicot inic acid [Example 32]
2-{3-Cyano-4-[4-(4-methoxyphenyl)butoxy]phenyl}nicotinic acid [Example 134]
2-[3-Cyano-4-(2,2-dimethylpropylamino)phenyl]nicotinic acid [Example 136]
2-(4-Azepan-1-yl-3-cyanophenyl)nicotinic acid [Example 138]
2-[3-Cyano-4-(2,2-dimethylpropoxy)phenyl]-5-fluoronicotinic acid [Example 140]
2-[4-(N-Methylbutylamino)-3-cyanophenyl]nicotinic acid [Example 142]

As hereunder, a general process for producing the compound of the present invention is shown. The compound of the present invention represented by the above formula (1) is able to be produced by a process mentioned below. There are also compounds which are produced by a process other than the following process and they are able to be produced by referring to Examples which will be mentioned later.

< Producing Process 1>

[chem. 2]

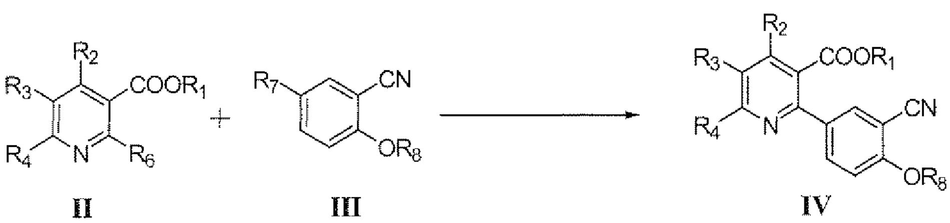

II III IV

[Scheme1]

$R_1$, $R_2$, $R_3$ and $R_4$ in the scheme 1 have the same meaning as mentioned above. $R_6$ is halogen; $R_7$ is $-B(OH)_2$ or $-B(OR_9)OR_{10}$; and $R_8$ is X which is mentioned already, hydrogen or a group used for protection of hydroxyl group such as methoxymethyl group. In the above formula, $R_9$ and $R_{10}$ are same or different and each is alkyl or alkylene where $R_9$ and $R_{10}$ are united.
The compound of the formula (II) and the compound of the formula (III) are used in the same amount or one of them is used excessively and they are made to react in a solvent inert to the reaction in the presence of a base and a palladium catalyst at room temperature or under heating to reflux usually for 1 to 24 hour(s) whereby the compound of the present invention or a material compound for producing the compound of the present invention is able to be produced.
As to the halogen represented by $R_6$, preferred ones are chlorine, bromine and iodine. Examples of the solvent include an aromatic hydrocarbon type solvent such as benzene, toluene or xylene; an ether type solvent such as tetrahydrofuran (THF), 1,4-dioxane, 1,2-dimethoxyethane (DME) or diethyl ether; a halogenated hydrocarbon type solvent such as dichloromethane, 1,2-dichloroethane or chloroform; and an alcohol type solvent such as methanol, ethanol or 2-propanol. Dimethylformamide (DMF), dimethyl sulfoxide (DMSO) or water may be used as well. The solvent is

appropriately selected depending, for example, upon the material compound. The solvent may be used solely or more than one solvent may be used by mixture.

As to the base, an inorganic base such as sodium hydroxide, potassium hydroxide, sodium carbonate, potassium carbonate or sodium hydrogen carbonate is preferred. Examples of a palladium catalyst include palladium acetate, tris(2-methylphenyl)phosphine, tetrakis(triphenylphosphine) palladium, dichlorobis(triphenylphosphine) palladium and palladium chloride-1,1'-bis(diphenylphosphino)ferrocene.

The compound (IV) produced by the above producing process 1 is able to be further induced into the compound of the present invention or a material compound for producing the compound of the present invention by subjecting to a common organic synthetic reaction such as hydrolyzing reaction, reducing reaction or acid decomposition reaction. For example, when $R_1$ is alkyl and $R_8$ is X, the compound of the present invention where $R_1$ is hydrogen is able to be produced by the reaction such as an acid or alkali hydrolyzing reaction. When $R_1$ is alkyl and $R_8$ is methoxymethyl group, the compound (V) in the Producing Process 2 is able to be produced by means of an acid decomposition reaction.

< Producing Process 2 >

[chem. 3]

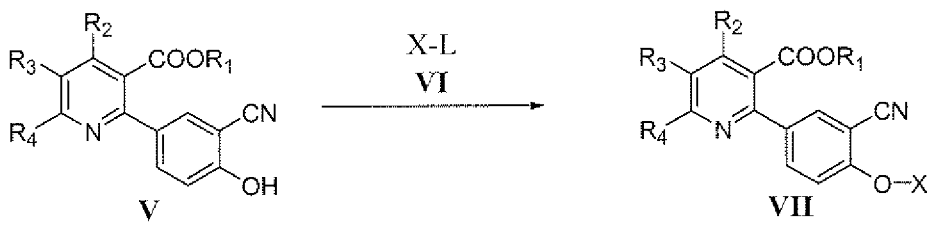


V

VII

[Scheme2]

$R_1$, $R_2$, $R_3$, $R_4$ and X in the scheme 2 have the same meanings as mentioned above. L is hydroxyl group or a leaving group which is easily substituted with hydroxyl group.

When the compound of the formula (VI) is condensed to the compound of the formula (V), the compound of the present invention or a material compound for producing the compound of the present invention is able to be produced.

Examples of a leaving group represented by L include halogen, methanesulfonyloxy group, p-toluenesulfonyloxy group and trifluoromethanesulfonyloxy group. The halogen has the same meaning as mentioned above. When L is a leaving group, it is carried out by such a means that the compound of the formula (V) and an alkylating agent of the formula (VI) are used in the same amount or the alkylating agent (VI) of them is used excessively and they are made to react in a solvent inert to the reaction in the presence of a base at room temperature or under heating to reflux usually for 1 to 24 hour(s). Examples of the solvent are the above-mentioned ones such as an aromatic hydrocarbon type solvent, an ether type solvent, a halogenated hydrocarbon type solvent, DMF or DMSO. The solvent is appropriately selected depending, for example, upon the material compound and may be used solely or two or more may be used by mixture. Examples of the base include an inorganic base such as sodium hydroxide, potassium hydroxide, sodium carbonate, potassium carbonate, sodium hydrogen carbonate or sodium acetate and an organic base such as aniline, pyridine, morpholine, piperidine, triethylamine, tributylamine, diisopropylethylamine, N-methylmorpholine or 4-dimethylaminopyridine.

When L is hydroxyl group, the compound (V) and an alkylating agent (VI) are used in the same amount or the alkylating agent (VI) is used excessively and they are made to react at room temperature or under heating to reflux usually for 1 hour to five days together with an azodicarboxylic acid derivative such as ethyl azodicarboxylate or 1,1'-(azodicarbonyl)dipiperidine and a phosphorus compound such as triphenyl phosphine or tributyl phosphine.

The compound (VII) produced by the above Production Process 2 is able to be induced into the compound of the present invention by a common organic synthetic reaction such as hydrolyzing reaction, reducing reaction or acid decomposition reaction. For example, when $R_1$ is alkyl, the compound of the present invention where $R_1$ is hydrogen is able to be produced by the reaction such as an acid or alkali hydrolyzing reaction.

When a pharmaceutically acceptable salt exists for the compound represented by the formula (I), said compound covers all of the salts as such and examples thereof include a salt with an inorganic acid such as hydrochloric acid, hydrobromic acid, hydroiodic acid, sulfuric acid, nitric acid or phosphoric acid and an acid addition salt with an organic acid such as formic acid, acetic acid, propionic acid, oxalic acid, malonic acid, succinic acid, fumaric acid, maleic acid,

lactic acid, malic acid, tartaric acid, citric acid, methanesulfonic acid, aspartic acid or glutamic acid. Depending upon the type of the substituent, there may be the case where a salt with a base is formed and examples thereof include a salt with an inorganic base including metal such as sodium, potassium, magnesium, calcium or aluminum, a salt with an organic base such as methylamine, ethylamine, ethanolamine, lysine or ornithine and an ammonium salt. When the product exists in a form of a stereoisomer such as cis-trans isomer, optical isomer or conformational isomer, a hydrate, a solvent or a metal complex compound, the present invention covers any of such stereoisomer, hydrate, solvate and complex compound.

The compound of the present invention is able to be made into a drug by combining with an appropriate pharmaceutical carrier or diluent. It is able to be made into a pharmaceutical preparation by any of usual means as an oral preparation such as tablets, capsules, powder or liquid and a parenteral preparation such as that for hypodermal, intramuscular, intrarectal or intranasal administration. In the prescription, the compound of the present invention may be used in a form of a pharmaceutically acceptable salt thereof wherein it is able to be used either solely or jointly by an appropriate combination. It may be made into a compounded agent with other pharmaceutically active ingredient.

As to an orally administering preparation, the compound *per se* or together with an appropriate additive such as a common excipient (e.g., lactose, mannitol, corn starch or potato starch) is able to be made into tablets, diluted powder, granules or capsules by means of an appropriate combination with a binder such as crystalline cellulose, cellulose derivative, acacia, corn starch or gelatin; a disintegrating agent such as corn starch, potato starch or carboxymethyl cellulose potassium; a lubricant such as talc or magnesium stearate; and others such as bulking agent, moisturizer, buffer, preservative or flavor.

It is also possible to make into other preparation than above which is optimum for the treatment depending upon the type of diseases or upon the patient such as a preparation for external application including injection, suppository, inhaling agent, aerosol, syrup, eye drop and ointment.

Although the advisable dose of the compound of the present invention varies depending upon a subject to be administered, dosage form, administering method, administering period, etc., said compound is usually able to be orally administered in an amount of 25 to 2,000 mg or, preferably, 50 to 1,000 mg a day either at a time or by dividing into several times to an adult for achieving the desired effect. In the case of a parenteral administration (such as injection preparation), its daily dose is preferred to be in a dose level of from 1/3 to 1/10 of each of the above doses.

[Examples]

Now the present invention will be specifically illustrated by way of the following examples although the present invention is not limited at all thereby. A process for producing a material compound used in Examples will be illustrated in Referential Examples. In drying an organic solvent used in the following Referential Examples and Examples, anhydrous sodium sulfate was used and, unless otherwise mentioned, evaporation of an organic solvent was carried out using a rotary evaporator *in vacuo*. Drying of the final product was conducted using a vacuum drier at 50˚C for 12 hours in the presence of phosphorus pentaoxide.

<Referential Example 1> 5-Bromo-2-hydroxybenzonitrile

To a solution of 5-bromosalicylaldehyde (80.0 g, 0.40 mol) in formic acid were added hydroxylamine hydrochloride (36.0 g, 0.52 mol) and sodium format (37.0 g, 0.52 mol) followed by stirring at 100˚C for 7 hours. Ethyl acetate was added to the residue prepared by evaporation of the solvent, the mixture was washed with water and then an organic layer was dried. Petroleum ether was added to the residue prepared by evaporation of the solvent and the crystals separated out therefrom were filtered to give the title compound (75.2 g, 95%).
$^1$H-NMR (DMSO-$d_6$) δ: 6.98 (d, J=8.9 Hz, 1H), 7.65 (dd, J=8.9, 2.4 Hz, 1H), 7.86 (d, J=2.4 Hz, 1H), 11.41 (s, 1H).

<Referential Example 2> 5-Bromo-2-methoxy- methoxybenzonitrile

A solution of 5-bromo-2-hydroxybenzonitrile (Referential Example 1) (54.1 g, 0.27 mol) in DMF (200 ml) was dropped into a solution of tert-butoxy potassium (40.0 g, 0.35 mol) in DMF (200 mL) at 0˚C followed by stirring at room temperature for 1 hour. Chloromethyl methyl ether (25 mL, 0.33 mol) was dropped thereinto at 0˚C followed by stirring at room temperature for 15 hours. The reaction solution was poured into ice water followed by extracting with ether. The organic layer was successively washed with a 5% aqueous solution of potassium hydroxide and a saturated saline solution and dried. Petroleum ether was added to the residue prepared by evaporation of the solvent and the crystals separated out therefrom were filtered to give the title compound (67.0 g, 86%).
$^1$H-NMR (DMSO-$d_6$) δ : 3.43 (s, 3H), 5.38 (s, 2H), 7.30 (d, J=9.1 Hz, 1H), 7.84 (dd, J=9.1, 2.4 Hz, 1H), 8.04 (d, J=2.4 Hz, 1H).

<Referential Example 3> 3-Cyano-4-methoxyphenylboronic acid

A solution of n-butyl lithium (1.6 mol/L hexane) (105 mL, 0.17 mol) was dropped into a solution of 5-bromo-2-methoxymethoxybenzonitrile (Referential Example 2) and triisopropyl borate (43 mL, 0.19 mol) in anhydrous THF (150 mL) in argon atmosphere at -80˚C during 1 hour and then the reaction solution was slowly returned to room temperature during 20 hours. A saturated aqueous solution of ammonium chloride was gradually added thereto followed by extracting with ethyl acetate. The organic layer was successively washed with a saturated ammonium chloride solution, water and a saturated saline solution and dried. Petroleum ether was added to the residue prepared by evaporation of the solvent and the crystals separated out therefrom were filtered to give the title compound (22.8 g, 89%).
$^1$H-NMR (DMSO-$d_6$) δ : 3.43 (s, 3H), 5.39 (s, 2H), 7.30 (d, J=8.6 Hz, 1H), 8.02 (dd, J=8.6, 1.2 Hz, 1H), 8.08 (d, J=1.2 Hz, 1H), 8.22 (s, 2H).

<Referential Example 4a> Ethyl 2-chloronicotinate

p-Toluenesulfonic acid monohydrate (1.2 g, 6.3 mmol) was added to a solution of 2-chloronicotinic acid (10.0 g, 64 mmol) in ethanol (200 mL) followed by heating to reflux for 24 hours. Ether was added to the residue prepared by evaporation of the solvent followed by successively washing with a 10% aqueous solution of sodium hydrogen carbonate and a saturated saline solution. The organic layer was dried and the residue prepared by evaporation of the solvent was purified by silica gel column chromatography (hexane/ethyl acetate = 5/1) to give the title compound (8.4 g, 72%) as an oily product.
$^1$H-NMR (DMSO-$d_6$) δ : 1.33 (t, J=7.2 Hz, 3H), 4.36 (q, J=7.2 Hz, 2H), 7.58 (dd, J=7.7, 4.8 Hz, 1H), 8.25 (dd, J=7.7, 1.8 Hz, 1H), 8.59 (dd, J=4.8, 1.8 Hz, 1H).

<Referential Example 4b> Benzyl 2-chloronicotinate

WSC.HCl (12.8 g, 66.8 mmol) and DMAP (0.78 g, 6.4 mmol) were added at 0˚C to a solution of 2-chloronicotinic acid (10.0 g, 63.3 mml) and benzyl alcohol (6.9 g, 63.6 mmol) in dichloromethane followed by stirring at room temperature for 22 hours. The reaction mixture was successively washed with water, a saturated aqueous solution of sodium hydrogen carbonate and a saturated saline solution and then an organic layer was dried. The solvent was evaporated therefrom to give the title compound (13.3 g, 85%) as an oily product.
$^1$H-NMR (DMSO-$d_6$) δ : 5.39 (s, 2H), 7.32-7.43 (m, 3H), 7.50 (d, J=7.6 Hz, 2H), 7.58 (dd, J=7.8, 5.1 Hz, 1H), 8.30 (dd, J=7.8, 2.2 Hz, 1H), 8.61 (dd, J=5.1, 2.2 Hz, 1H)

<Referential Example 4c> Ethyl 2-chloro-6-methyl- nicotinate

The same operation as in Referential Example 4a was conducted starting from 2-chloro-6-methylnicotinic acid (15.0 g, 87.4 mmol), p-toluenesulfonic acid monohydrate (3.3 g, 87.4 mmol) and ethanol (300 mL) to give the title compound (11.2 g, 64%) as an oily product.
$^1$H-NMR (DMSO-$d_6$) δ : 1.33 (t, J=7.3 Hz, 3H), 2.52 (s, 3H), 4.34 (q, J=7.3 Hz, 2H), 7.42 (d, J=7.9 Hz, 1H), 8.15 (d, J=7.9 Hz, 1H).

<Referential Example 4d> Ethyl 2-chloro-4-methyl- nicotinate

Step (1): A solution of ethyl 2-cyano-3-methyl- 2-butenoate (15.0 g, 95.0 mmol) and dimethoxymethyl dimethylamine (11.3 g, 95.0 mmol) in ethanol (100 mL) was heated to reflux for 24 hours. The residue prepared by evaporation of the solvent was purified by a silica gel column chromatography (hexane/ethyl acetate = 2/3) to give ethyl 2-eyano-5-dimethylamino-3-methylpenta-2,4-dienoate (15.2 g, 74%).
$^1$H-NMR (DMSO-$d_6$) δ : 1.19 (t, J=7.0 Hz, 3H), 2.23 (s, 3H), 2.92 (s, 3H), 3.33 (s, 3H), 4.09 (s, 3H), 4.09 (q, J=7.0 Hz, 2H), 6.98 (d, J=13.0 Hz, 1H), 7.79 (d, J=13.0 Hz, 1H).
Step (2): Hydrogen chloride gas was introduced for 15 minutes into a solution of ethyl 2-cyano-5-dmiethylammo-3-methylpenta-2,4-dienoate (10.0 g, 48.0 mmol) in ethanol (200 mL) at 0˚C followed by heating to reflux for 8 hours. Water was added to the residue prepared by concentrating the reaction solution and then the mixture was adjusted to pH 7 using triethylamine. The liberated organic layer was extracted with dichloromethane and dried and the solvent was evaporated therefrom. The resulting residue was purified by silica gel column chromatography (hexane/ethyl acetate = 7/1) to give the title compound (6.3 g, 65%) as an oily product.
$^1$H-NMR (DMSO-$d_6$) δ : 1.34 (t, J=7.1 Hz, 3H), 2.33 (s, 3H), 4.41 (q, J=7.1 Hz, 2H), 7.42 (d, J=5.1 Hz, 1H), 8.39 (d, J=5.1 Hz, 1H).

<Referential Example 5a> Ethyl 2-(3-cyano-4-methoxymethoxyphenyl)nicotinate

Palladium acetate (0.60 g, 2.7 mmol), tris(2-methylphenyl)phosphine (1.7 g, 5.5 mmol) and a 10% aqueous solution of sodium carbonate (93 mL, 87 mmol) were added to a solution of ethyl 2-chloronicotinate (Referential Example 4) (8.4 g, 45 mmol) and 3-cyano-4-methoxymethoxyphenylboronic acid (Referential Example 3) (12.2 g, 59 mmol) in DME (440 mL) in an argon atmosphere followed by stirring at 80˚C for 3 hours. Dichloromethane was added to the residue prepared by evaporation of the solvent followed by washing with water. The residue prepared by drying the organic layer and evaporating the solvent was purified by silica gel column chromatography (hexane/ethyl acetate = 3/2) to give the title compound (8.3 g, 58%) as crystals.
$^{1}$H-NMR (DMSO-$d_6$) $\delta$ : 1.11 (t, J=7.0 Hz, 3H), 3.47 (s, 3H), 4.19 (q, J=7.0 Hz, 2H), 5.45 (s, 2H), 7.42 (d, J=8.8 Hz, 1H), 7.55 (dd, J=7.8, 4.8 Hz, 1H), 7.78 (dd, J=8.8, 2.2 Hz, 1H), 7.87 (d, J=2.2 Hz, 1H), 8.21 (dd, J=7.8, 1.1 Hz, 1H), 8.81 (dd, J=4.8, 1.1 Hz, 1H).

<Referential Example 5b> Benzyl 2-(3-cyano-4-methoxymethoxyphenyl)nicotinate

The same operation as in Referential Example 5a was carried out starting from benzyl 2-chloronicotinate (Referential Example 4b) (6.0 g, 24.2 mmol), 3-cyano-4-methoxymethoxyphenylboronic acid (Referential Example 3) (6.0 g, 29.0 mmol), palladium acetate (0.33 g, 1.45 mmol), tris(2-methylphenyl)phosphine (0.9 g, 2.9 mmol), a 10% aqueous solution of sodium carbonate (46 mL, 43.9 mmol) and DME (230 mL) to give the title compound (1.2 g, 13%) as an oily product.
$^{1}$H-NMR (DMSO-$d_6$) $\delta$ : 3.46 (s, 3H), 5.20 (s, 2H), 5.42 (s, 2H), 7.19-7.21 (m, 2H), 7.29-7.34 (m, 4H), 7.55-7.56 (m, 1H), 7.70 (d, J=2.2 Hz, 1H), 7.85 (d, J=2.2 Hz, 1H), 8.26 (dd, J=8.1, 1.1 Hz, 1H), 8.80 (dd, J=4.8, 1.1 Hz, 1H).

<Referential Example 5c> Ethyl 2-(3-cyano-4-methoxymethoxyphenyl)-6-methylnicotinate

The same operation as in Referential Example 5a was carried out starting from ethyl 2-chloro-5-methylnlcotinate (Referential Example 4c) (7.0 g, 35.1 mmol), 3-cyano-4-methoxymethoxyphenylboronic acid (Referential Example 3) (9.4 g, 45.6 mmol), palladium acetate (0.5 g, 2.1 mmol), tris(2-methylphenyl)phosphine (1.3 g, 4.2 mmol), a 10% aqueous solution of sodium carbonate (67 mL, 63.2 mmol) and DME (330 mL) to give the title compound (8.2 g, 71%) as crystals.
$^{1}$H-NMR (DMSO-$d_6$) $\delta$ : 1.09 (t, J=6.9 Hz, 3H), 2.51 (s, 3H), 3.45 (s, 3H), 4.15 (q, J=6.9 Hz, 2H), 4.34 (q, J=7.3 Hz, 2H), 7.38-7.41 (m, 2H), 7.74 (dd, J=8.9, 2.2 Hz, 1H), 7.84 (d, J=2.2 Hz, 1H), 8.11 (d, J=8.1 Hz, 1H).

<Referential Example 5d> Ethyl 2-(3-cyano-4-methoxymethoxyphenyl)-4-methylnicotinate

The same operation as in Referential Example 5a was carried out starting from ethyl 2-chloro-4-methylnicotinate (Referential Example 4d) (6.3 g, 31.4 mmol), 3-cyano-4-methoxymethoxyphenylboronic acid (Referential Example 3) (7.8 g, 37.7 mmol), palladium acetate (0.42 g, 1.9 mmol), tris(2-methylphenyl)phosphine (1.1 g, 3.8 mmol), a 10% aqueous solution of sodium carbonate (60 mL, 56.5 mmol) and DME (300 mL) to give the title compound (5.5 g, 53%) as an oily product.
$^{1}$H-NMR (DMSO-$d_6$) $\delta$ : 1.10 (t, J=7.1 Hz, 3H), 2.37 (s, 3H), 3.46 (s, 3H), 4.21 (q, J=7.1 Hz, 2H), 5.44 (s, 2H), 7.40 (d, J=5.0 Hz, 1H), 7.45 (d, J=8.9 Hz, 1H), 7.79 (dd, J=8.9, 2.3 Hz, 1H), 7.83 (d, J=2.3 Hz, 1H), 8.61 (d, J=5.0 Hz, 1H).

<Referential Example 6a> Ethyl 2-(3-cyanohydroxy-phenyl)nicotinate hydrochloride

Into a solution of ethyl 2-(3-cyano-4-mothoxymethoxy-phenyl)nicotinate (Referential Example 5a) (8.0 g, 26 mmol) in dichloromethane (100 mL) was dropped 4 mol/L hydrogen chloride-dioxane (32 mL, 128 mmol) at room temperature followed by stirring for 14 hours. The crystals separated out therefrom were filtered and washed with diethyl ether to give the title compound (7.6 g, 97%).
$^{1}$H-NMR (DMSO-$d_6$) $\delta$ : 1.11 (t, J=7.2 Hz, 3H), 4.19 (q, J=7.2 Hz, 2H), 7.19 (d, J=8.7 Hz, 1H), 7.59 (dd, J=7.7, 4.9 Hz, 1H), 7.65 (dd, J=8.7, 2.2 Hz, 1H), 7.74 (d, J=2.2 Hz, 1H), 8.25 (d, J=7.7 Hz, 1H), 8.82 (d, J=4.9 Hz, 1H), 9.25-9.75 (brs, 2H), 11.50-11.75 (br, 2H).

<Referential Example 6b> Benzyl 2-(3-cyano-4-hydroxyphenyl)nicotinate hydrochloride

The same operation as in Referential Example 6a was carried out starting from benzyl 2-(3-cyano-4-methoxymethoxyphenyl)nicotinate (Referential Example 5b) (1.2 g, 3.2 mmol), 4 mol/L hydrogen chloride-dioxane (4.0 mL, 16.0 mmol) and dichloromethane (20 mL) to give the title compound (1.1 g, 94%) as crystals.
$^{1}$H-NMR (DMSO-$d_6$) $\delta$ : 5.20 (s, 2H), 7.06 (d, J=7.6 Hz, 1H), 7.20-7.21 (m, 2H), 7.32-7.34 (m, 3H), 7.55-7.57 (m, 2H),

7.73 (s, 1H), 8.24 (d, J=7.1 Hz, 1H), 8.80 (d, J=4.8 Hz, 1H), 11.25-11.30 (br, 1H), 11.50-11.60 (br, 1H).

<Referential Example 6c> Ethyl 2-(3-cyano-4-hydroxyphenyl)-6-methylnicotinate hydrochloride

The same operation as in Referential Example 6a was carried out starting from ethyl 2-(3-cyano-4-methoxymethoxyphenyl)-6-methylnicotinate (Referential Example 5c) (8.0 g, 24.5 mmol), 4 mol/L hydrogen chloride-dioxane (31 mL, 122.6 mmol) and dichloromethane (150 mL) to give the title compound (6.3 g, 81%) as crystals.
$^1$H-NMR (DMSO-$d_6$) δ : 1.10 (t, J=7.3 Hz, 3H), 2.64 (s, 3H), 4.16 (q, J=7.3 Hz, 2H), 7.19 (d, J=8.8 Hz, 1H), 7.54 (d, J=8.0 Hz, 1H), 7.63 (dd, J=8.8, 2.2 Hz, 1H), 7.77 (d, J=2.2 Hz, 1H), 8.27 (d, J=8.0 Hz, 1H), 9.25-9.50 (br, 1H), 11.60-11.75 (br, 1H).

<Referential Example 6d> Ethyl 2-(3-cyano-4-hydroxyphenyl)-4-methylnicotinate hydrochloride

The same operation as in Referential Example 6a was carried out starting from ethyl 2-(3-cyano-4-methoxymethoxyphenyl)-4-methylnicotinate (Referential Example 5d) (5.5 g, 16.7 mmol), 4 mol/L hydrogen chloride-dioxane (21 mL, 83.7 mmol) and dichloromethane (150 mL) to give the title compound (4.8 g, 90%) as crystals.
$^1$H-NMR (DMSO-$d_6$) δ : 1.09 (t, J=7.2 Hz, 3H), 2.42 (s, 3H), 4.21 (q, J=7.2 Hz, 2H), 7.23 (d, J=8.8 Hz, 1H), 7.54 (d, J=5.3 Hz, 1H), 7.67 (dd, J=8.8, 2.3 Hz, 1H), 7.75 (d, J=2.3 Hz, 1H), 8.67 (d, J=5.3 Hz, 1H), 11.75-11.85 (br, 1H).

<Referential Example 7a> Ethyl 2-[4-(3-chloropropoxy)-3-cyanophenyl]nicotinate

A suspension of ethyl 2-(3-cyano-4-hydroxyphenyl)- nicotinate hydrochloride (Referential Example 6a) (3.0 g, 9.8 mmol) in DMF (45 mL) was added to a suspension of potassium carbonate (3.1 g, 22.6 mmol) in DMF (25 mL) at 0˚C followed by stirring at room temperature for 1 hour. 1-Bromo-3-chloropropane (1.2 mL, 11.8 mmol) was added thereto at room temperature followed by stirring at 80˚C for 20 hours. The reaction mixture was poured into ice water and extracted with ethyl acetate. The organic layer was washed with water and dried and the solvent was evaporated to give the title compound (3.2 g, 95%) as an oily product.
$^1$H-NMR (DMSO-$d_6$) δ : 1.12 (t, J=7.3 Hz, 3H), 2.24-2.28 (m, 2H), 3.85 (t, J=6.5 Hz, 2H), 4.19 (q, J=7.3 Hz, 2H), 4.34 (t, J=5.9 Hz, 2H), 7.38 (d, J=8.9 Hz, 1H), 7.54 (dd, J=7.8, 4.6 Hz, 1H), 7.79 (dd, J=8.9, 2.3 Hz, 1H), 7.86 (d, J=2.3 Hz, 1H), 8.20 (dd, J=7.8, 1.4 Hz, 1H), 8.81 (dd, J=4.6, 1.4 Hz, 1H).

<Referential Example 7b> Ethyl 2-{3-cyano-4-[3-(3,5-di-tert-butyl-4-methoxymethoxyphenyl)propoxy]phenyl}-nicotinate

The same operation as in Referential Example 7a was carried out starting from ethyl 2-(3-cyano-4-hydroxyphenyl)nicotinate hydrochloride (Referential Example 6a) (2.1 g, 6.9 mmol), 3-(3,5-di-tert-butyl-4-methoxymethoxyphenyl)propyl methane- sulfonate (2.9 g, 7.6 mmol), potassium carbonate (2.2 g, 15.9 mmol) and DMF (120 mL) to give the title compound (3.4 g, 88%) as an oily product.
$^1$H-NMR (DMSO-$d_6$) δ : 1.11 (t, J=7.0 Hz, 3H), 1.35 (s, 18H), 2.05-2.09 (m, 2H), 2.73 (t, J=7.3 Hz, 2H), 3.53 (s, 3H), 4.16-4.20 (m, 4H), 4.82 (s, 2H), 7.11 (s, 2H), 7.31 (d, J=8.8 Hz, 1H), 7.54 (dd, J=7.9, 4.8 Hz, 1H), 7.76 (dd, J=8.8, 2.2 Hz, 1H), 7.86 (d, J=2.2 Hz, 1H), 8.19 (dd, J=7.9, 1.3 Hz, 1H), 8.80 (dd, J=4.8, 1.3 Hz, 1H).

<Referential Example 7c> Ethyl 2-{3-cyano-4-[4-(3,5-di-tert-butyl-4-methoxymethoxyphenyl)butoxy]phenyl}-nicotinate

The same operation as in Referential Example 7a was carried out starting from ethyl 2-(3-cyano-4-hydroxyphenyl)nicotinate hydrochloride (Referential Example 6a) (1.2 g, 3.9 mmol), 4-(3,5-di-tert-butyl-4-methoxymethoxyphenyl)butyl methane- sulfonate (1.7 g, 4.2 mmol), potassium carbonate (1.2 g, 8.9 mmol) and DMF (160 mL) to give the title compound (2.1 g, 93%) as an oily product.
$^1$H-NMR (DMSO-$d_6$) δ : 1.11 (t, J=7.2 Hz, 3H), 1.39 (s, 18H), 1.74-1.84 (m, 4H), 2.60 (d, J=7.4 Hz, 2H), 4.18 (q J=7.2 Hz, 2H), 4.24 (t, J=6.0 Hz, 2H), 7.10 (s, 2H), 7.32 (d, J=8.9 Hz, 1H), 7.53 (dd, J=7.8, 5.0 Hz, 1H), 7.77 (dd, J=8.9, 2.2 Hz, 1H), 7.83 (d, J=2.2 Hz, 1H), 8.19 (dd, J=7.8, 1.3 Hz, 1H), 8.80 (dd, J=5.0, 1.3 Hz, 1H).

<Referential Example 8a> 5-Bromo-2-(2,2-dimethyl-propoxy)benzonitrile

A solution of 5-bromo-2-hydroxybenzonitrile (Referential Example 1) (19.0 g, 96 mmol) in DMF (100 mL) was dropped into a solution of tert-butoxy potassium (14.1 g, 126 mmol) in DMF (100 mL) at 0˚C followed by stirring at room temperature for 1 hour. A solution of neopentyl iodide (25.0 g, 126 mmol) in DMF (100 mL) was dropped thereinto at 0˚C followed by stirring at 80˚C for 36 hours. The reaction solution was poured into ice water followed by extracting with ether. The organic layer was successively washed with a 5% aqueous solution of potassium hydroxide and a saturated

saline solution and dried. Petroleum ether was added to the residue prepared by evaporating the solvent and the crystals separated out therefrom were filtered to give the title compound (13.1 g, 71%).
$^1$H-NMR (DMSO-$d_6$) δ : 1.02 (s, 9H), 3.80 (s, 2H), 7.21 (d, J=9.1 Hz, 1H), 7.81 (dd, J=9.1, 2.2 Hz, 1H), 7.98 (d, J=2.2 Hz, 1H).

<Referential Example 8b> 5-Bromo-2-(3,3-dimethyl-butoxy)benzonitrile

A suspension of 5-bromo-2-hydroxybenzonitrile (Referential Example 1) (5.9 g, 30 mmol) in DMF (100 mL) was added to a suspension of potassium carbonate (4.1 g, 30 mmol) in DMF (100 mL) at 0˚C followed by stirring at room temperature for 1 hour. A solution of 3,3-dimethylbutyl methanesulfonate (5.5 g, 30.5 mmol) in DMF (100 mL) was added thereto at room temperature followed by stirring at 80˚C for 12 hours. The reaction solution was poured into ice water followed by extracting with ethyl acetate. The organic layer was washed with water and dried and the solvent was evaporated therefrom to give the title compound (5.8 g, 69%) as crystals.
$^1$H-NMR (DMSO-$d_6$) δ : 0.97 (s, 9H), 1.69 (t, J=7.0 Hz, 2H), 4.18 (t, J=7.0 Hz, 2H), 7.27 (d, J=9.1 Hz, 1H), 7.82 (dd, J=9.1, 2.5 Hz, 1H), 7.98 (d, J=2.5 Hz, 1H).

<Referential Example 8c> 5-Bromo-2-(2,2-dimethyl-propoxy)benzotrifluoride

Sodium hydride (60% oily) (46 g, 115.6 mmol) was gradually added to a solution of 5-bromo-2-chlorobenzotrifluoride (15.0 g, 57.8 mmol) and neopentyl alcohol (14.2 g, 115.6 mmol) in anhydrous DMF (150 mL) at 0˚C. After stirring at room temperature for 6 hours, the reaction mixture was poured into ice water followed by extracting with ethyl acetate. The organic layer was washed with a saturated saline solution and then dried. The residue prepared by evaporation of the solvent was purified by silica gel column chromatography (hexane) to give the title compound (12.6 g, 70%) as an oily product.
$^1$H-NMR (DMSO-$d_6$) δ : 1.02 (s, 9H), 3.75 (s, 2H), 7.21 (d, J=8.9 Hz, 1H), 7.73 (d, J=2.2 Hz, 1H), 7.79 (dd, J=8.9, 2.2 Hz, 1H).

<Referential Example 8d> 4-Bromo-1-(2,2-dimethyl-propoxy)-2-fluorobenzene

The same operation as in Referential Example 8a was carried out starting from 4-bromo-2-fluorophenol (8.0 g, 41.9 mmol), neopentyl iodide (6.7 mL, 50.3 mmol), tert-butoxy potassium (5.6 g, 50.3 mmol) and DMF (120 mL) to give the title compound (9.7 g, 71%) as an oily product.
$^1$H-NMR (DMSO-$d_6$) δ : 1.92 (s, 9H), 3.70 (s, 2H), 7.11-7.15 (m, 1H), 7.29-7.31 (m, 1H), 7.48-7.50 (m, 1H).

<Referential Example 9a> 5-Bromo-2-(2,2-dimethyl-butoxy)benzonitrile

Sodium hydride (60% oily) (2.0 g, 50.0 mmol) was gradually added to a solution of 5-bromo-2-fluorobenzonitrile (5.0 g, 25.0 mmol) and 2,2-dimethylbutan-1-ol (5.1 g, 50.0 mmol) in anhydrous DMF (100 mL) at 0˚C. After stirring at room temperature for 15 hours, the reaction mixture was poured into ice water followed by extracting with ethyl acetate. The organic layer was washed with a saturated saline solution and then dried. The residue prepared by evaporation of the solvent was purified by silica gel column chromatography (hexane/ethyl acetate = 50/1) to give the title compound (6.2 g, 88%) as an oily product.
The compounds of Referential Examples 9b to 9k shown in Table 1 were produced by the same operation as in Referential Example 9a starting from the corresponding alcohols respectively. Physical and chemical data of the compounds produced in Referential Examples 9a to 9k are shown in Table 1.
[Table 1.]

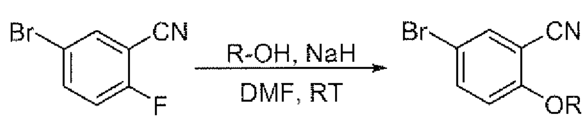


**9a-k**

(continued)

| Referential Example | R-OH | Physical and Chemical Data of 9a-k $^1$H-NMR (DMSO-$d_6$)( δ ) |
|---|---|---|
| **9a** | 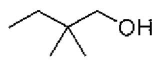 | **0.85 (t, *J*=7.6 Hz, 3H), 0.98 (s, 6H) 1.40 (q, *J*=7.6 Hz, 2H), 3.81 (s, 2H), 7.23 (d, *J*=9.1 Hz, 1H), 7.81 (dd, *J*=9.1, 2.3 Hz, 1H), 7.97 (d, *J*=2.3 Hz, 1H)** |
| **9b** | 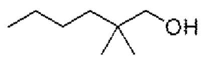 | **0.88 (t, *J*=6.5 Hz, 3H), 0.99 (s, 6H), 1.25-1.37 (m, 6H), 3.81 (s, 2H), 7.23 (d, *J*=9.0 Hz, 1H), 7.81 (dd, *J*=9.0, 2.1 Hz, 1H), 7.97 (d, *J*=2.1 Hz, 1H)** |
| **9c** | 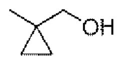 | **0.42-0.44 (m, 2H), 0.55-0.57 (m, 2H), 1.21 (s, 3H), 3.93 (s, 2H), 7.16 (d, *J*=8.9 Hz, 1H), 7.80 (d, *J*=8.9 Hz, 1H), 7.98 (d, *J*=2.0 Hz, 1H)** |
| **9d** | 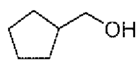 | **1.33-1.37 (m, 2H), 1.53-1.64 (m, 4H), 1.76-1.79 (m, 2H), 2.31-2.34 (m, 1H), 4.02 (d, *J*=6.8 Hz, 2H), 7.22 (d, *J*=9.1 Hz, 1H), 7.86 (dd, *J*=9.1, 2.6 Hz, 1H), 7.99 (d, *J*=2.6 Hz, 1H)** |
| **9e** | 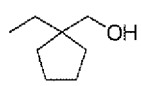 | **0.84 (t, *J*=7.5 Hz, 3H), 1.42-1.63 (m, 10H), 3.88 (s, 2H), 7.26 (d, *J*=9.1 Hz, 1H), 7.81 (dd, *J*=9.1, 2.4 Hz, 1H), 7.88 (d, *J*=2.4 Hz, 1H)** |
| **9f** | 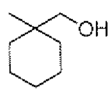 | **1.02 (s, 3H), 1.29-1.47 (m, 10H), 3.85 (s, 2H), 7.24 (d, *J*=9.0 Hz, 1H), 7.81 (d, *J*=9.0 Hz, 1H), 7.98 (s, 1H)** |
| **9g** | 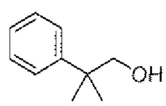 | **1.43 (s, 6H), 4.15 (s, 2H), 7.20-7.24 (m, 2H), 7.31-7.34 (m, 2H), 7.48-7.50 (m, 2H), 7.78 (d, *J*=8.3 Hz, 1H), 7.95 (s, 1H)** |
| **9h** | 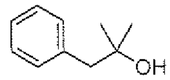 | **1.37 (s, 6H), 3.40 (s, 2H), 7.25-7.34 (m, 6H), 7.76 (d, *J*=9.6, 2.6 Hz, 1H), 8.00 (d, *J*=2.6 Hz, 1H)** |
| **9i** | 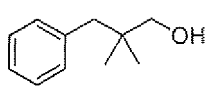 | **0.98 (s, 6H), 2.69 (s, 2H), 3.73 (s, 2H), 7.11 (d, *J*=7.2 Hz, 2H), 7.18-7.21 (m, 2H), 7.25-7.28 (m, 2H), 7.82 (d, *J*=9.0, 2.3 Hz, 1H), 8.04 (d, *J*=2.3 Hz, 1H)** |
| **9j** | 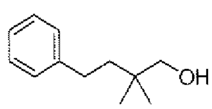 | **1.08 (s, 6H), 1.64-1.68 (m, 2H), 2.57-2.60 (m, 2H), 3.88 (s, 2H), 7.14-7.23 (m, 6H), 7.81 (dd, *J*=9.0, 2.3 Hz, 1H), 7.96 (d, *J*=2.3 Hz, 1H)** |
| **9k** | 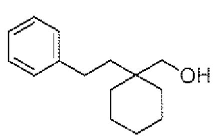 | **1.44-1.52 (m, 10H), 1.71-1.74 (m, 2H), 2.50-2.54 (m, 2H), 7.13-7.30 (m, 6H), 7.83 (dd, *J*=9.1, 2.5 Hz, 1H), 7.99 (d, *J*=2.5 Hz, 1H)** |

<Referential Example 10a> 4-(2,2-Dimethylpropoxy)-3-trifluoromethylphenylboronic acid

n-Butyl lithium (1.6 mol/L solution in hexane) (33 mL, 52.6 mmol) was dropped into a solution of 5-bromo-2-(2,2-dimethylpropoxy)benzotriluoride (Referential Example 8c) (12.6 g, 40.0 mmol) and triisopropyl borate (12 mL, 52.6 mmol) in anhydrous THF (150 mL) in an argon atmosphere at -80˚C during 30 minutes. After the dropping, the reaction solution was gradually returned to room temperature and then stirred for 2 hours just as it was. Diluted hydrochloric acid was gradually added thereto followed by stirring at room temperature for 0.5 hour. The reaction mixture was diluted with water and then extracted with ethyl acetate. The organic layer was successively washed with water and a saturated saline solution and dried and the solvent was evaporated therefrom. Petroleum ether was added to the residue and the crystals separated out therefrom were filtered to give the title compound (3.82 g, 34%).

$^1$H-NMR (DMSO-$d_6$) δ : 1.02 (s, 9H), 3.77 (s, 2H), 7.23 (d, J=8.3 Hz, 1H), 8.02-8.06 (m, 2H).

<Referential Example 10b> 4-(2,2-Dimethylpropoxy)-3-fluorophenylboronic acid

The same operation as in Referential Example 10a was carried out starting from 4-bromo-1-(2,2-dimethylpropoxy)-2-fluorobenzene (Referential Example 8d) (9.7 g, 37.1 mmol), triisopropyl borate (11.1 mL, 48.3 mmol), n-butyl lithium (1.6 mol/L solution in hexane) (30 mL, 48.3 mmol) and anhydrous THF (120 mL) to give the title compound (5.9 g, 70%).
$^1$H-NMR (DMSO-$d_6$) δ : 1.02 (s, 9H), 3.71 (s, 2H), 7.11-7.13 (m, 1H), 7.52-7.60 (m, 2H).

<Referential Example 10c> 3-Cyano-4-(2,2-dimethyl-butyloxy)phenylboronic acid

n-Butyl lithium (1.6 mol/L solution in hexane) (18 mL, 28.6 mmol) was dropped into a solution of 5-bromo-2-(2,2- dimethylpropoxy)benzonitrile (Referential Example 8a) (6.2 g, 22.0 mmol) and triisopropyl borate (7.6 mL, 28.6 mmol) in anhydrous THF (90 mL) in an argon atmosphere at -80˚C during 30 minutes. After the dropping, the reaction solution was gradually returned to room temperature and then stirred for 2 hours just as it was. Diluted hydrochloric acid was gradually added thereto followed by stirring at room temperature for 0.5 hour. The reaction mixture was diluted with water and then extracted with ethyl acetate. The organic layer was successively washed with water and a saturated saline solution followed by drying and the solvent was evaporated therefrom. Petroleum ether was added to the residue and the crystals separated out therefrom were filtered to give the title compound (3.1 g, 57%).
The compounds of Referential Examples 10d to 10o shown in Table 2 were produced by the same operation as in Referential Example 10c starting from the corresponding alcohols respectively. Physical and chemical data of the compounds produced in Referential Examples 10c to 10o are shown in Table 2.
[Table 2.]

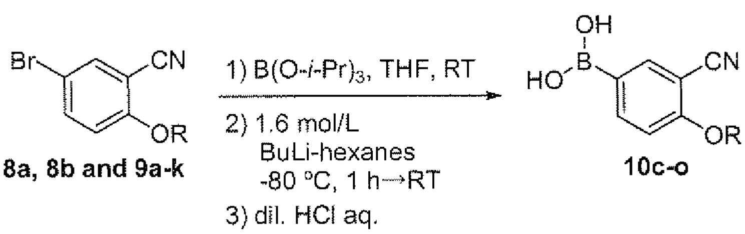


| Referential Example | R | Physical and Chemical Data of 10c-o<br>$^1$H-NMR (DMSO-$d_6$)( δ ) |
|---|---|---|
| **10c** | 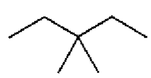 | **0.86 (t, *J*=7.6 Hz, 3H), 0.98 (s, 6H), 1.41 (q, *J*=7.6 Hz, 2H), 3.83 (s, 2H), 7.22 (d, *J*=8.3 Hz, 1H), 8.01-8.08 (m, 2H), 8.18 (s, 2H)** |
| **10d** | 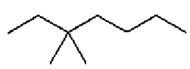 | **0.87 (t, *J*=7.6 Hz, 3H), 0.99 (s, 6H), 1.26-1.37 (m, 6H), 3.83 (s, 2H), 7.23 (d, *J*=8.3 Hz, 1H), 8.04-8.18 (m, 2H)** |
| **10e** | 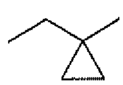 | **0.43 (t, *J*=5.0 Hz, 2H), 0.57 (t, *J*=5.0 Hz, 2H), 1.21 (s, 3H), 13.95 (s, 2H), 7.18 (d, *J*=8.5 Hz, 1H), 8.06-8.09 (m, 2H)** |
| **10f** | 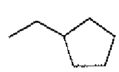 | **1.36-1.40 (m, 2H), 1.54-1.57 (m, 2H), 1.63-1.65 (m, 2H), 1.78-1.80 (m, 2H), 2.34-2.37 (m, 2H), 4.05 (d, *J*=6.8 Hz, 2H), 7.23 (d, *J*=9.0 Hz, 1H), 8.02-8.08 (m, 2H)** |
| **10g** | 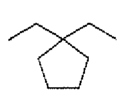 | **0.84 (t, *J*=7.3 Hz, 3H), 1.41-1.63 (m, 10H), 3.89 (s, 2H), 7.26 (d, *J*=8.4 Hz, 1H), 8.02-8.04 (m, 2H), 8.22 (s, 2H)** |

(continued)

| Referential Example | R | Physical and Chemical Data of 10c-o $^1$H-NMR (DMSO-$d_6$)( δ ) |
|---|---|---|
| **10h** | 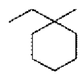 | **1.04 (s, 3H), 1.30-1.48 (m, 10H), 3.87 (s, 2H), 7.24 (d, *J*=8.5 Hz, 1H), 8.01-8.09 (m, 2H)** |
| **10i** | 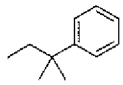 | **1.46 (s, 6H), 4.16 (s, 2H), 7.21-7.22 (m, 2H), 7.32-7.35 (m, 2H), 7.52 (d, *J*=7.8 Hz, 2H), 8.07-8.09 (m, 2H)** |
| **10j** | 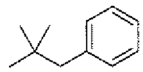 | **1.40 (s, 6H), 3.19 (s, 2H), 7.23-7.39 (m, 6H), 8.07-8.09 (m,2H)** |
| **10k** | 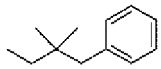 | **1.01 (s, 6H), 2.73 (s, 2H), 3.76 (s, 2H), 7.12-7.27 (m, 6H), 8.10-8.15 (m, 2H)** |
| **10l** | 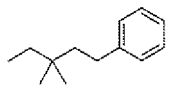 | **1.10 (s, 6H), 1.64-1.69 (m, 2H), 2.57-2.60 (m, 2H), 3.91 (s,1H), 7.13-7.27 (m, 6H), 8.02-8.11 (m, 2H)** |
| **10m** | 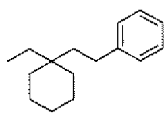 | **1.48-1.54 (m, 10H), 1.73-1.75 (m, 2H), 2.52-2.54 (m, 2H), 4.02 (s, 2H), 7.12-7.31 (m, 6H), 8.00-8.03 (m, 2H)** |
| **10n** | 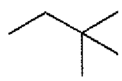 | **1.04 (s, 9H), 3.82 (s, 2H), 7.21 (d, *J*=9.1 Hz, 1H), 8.01-8.09 (m, 2H), 8.18 (s, 2H)** |
| **10o** | 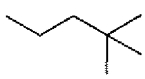 | **0.99 (s, 9H), 1.69-1.73 (m 2H), 4.19-4.23 (m, 2H), 7.27 (d, *J*=8.3 Hz, 1H), 8.08-8.12 (m, 2H)** |

<Example 1> Ethyl 2-(3-cyano-4-methoxyphenyl)- nicotinate

To a suspension of potassium carbonate (0.52 g, 3.8 mmol) in DMF (10 mL) was added a suspension of ethyl 2-(3-cyano-4-hydroxyphenyl)nicotinate hydrochloride (Referential Example 6a) (0.50 g, 1.6 mmol) in DMF (10 mL) at 0˚C followed by stirring at room temperature for 1 hour. After iodomethane (0.1. mL, 1.8 mmol) was added thereto at room temperature, the mixture was stirred for 3 hours just as it was. The reaction mixture was poured into ice water followed by extracting with ethyl acetate. The organic layer was washed with water and dried and the solvent was evaporated therefrom to give the title compound (0.45 g, 98%) as an oily product.

<Example 2> Ethyl 2-(3-cyano-4-isobutoxyphenyl)- nicotinate

To a suspension of potassium carbonate (1.0 g, 6.9 mmol) in DMF (10 mL) was added a suspension of ethyl 2-(3-cyano-4-hydroxyphenyl)nicotinate hydrochloride (Referential Example 6a) (0.90 g, 3.0 mmol) in DMF (20 mL) at 0˚C followed by stirring at room temperature for 1 hour. After isobutyl iodide (0.4 mL, 3.6 mmol) was added thereto at room temperature, the mixture was stirred for 20 hours at 80˚C. The reaction mixture was poured into ice water followed by extracting with ethyl acetate. The organic layer was washed with water and dried and the solvent was evaporated therefrom to give the title compound (0.53 g, 55%) as an oily product.
In accordance with the same method as in Example 1 or 2, the compounds of Examples 3 to 22 shown in Tables 3 to 5 were produced starting from the corresponding alkylating agents. Physical and chemical data of the compounds (ester substances of the compounds of the invention) produced in Examples 1 to 22 are shown in Tables 3 to 5.

[Table 3.]

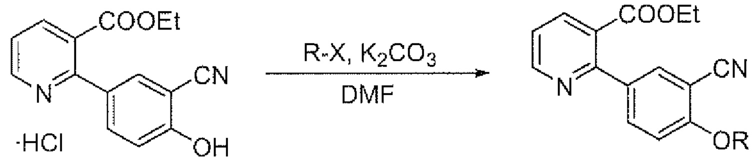


6 1-22

| Example | Alkylating agent R-X (X=I, Br, OMs) | $^1$H-NMR Spectral Data of 1-22 (δ, DMSO-$d_6$) |
|---|---|---|
| **1** | Me-I | **1.12 (t, *J*=7.2 Hz, 3H), 4.00 (s, 3H), 4.19 (q, *J*=7.2 Hz, 2H), 7.34 (d, *J*=8.7 Hz, 1H), 7.54 (dd, *J*=7.8, 4.6 Hz, 1H), 7.81 (dd, *J*=8.7, 2.2 Hz, 1H), 7.85 (d, *J*=2.2 Hz, 1H), 8.20 (dd, *J*=7.8, 1.4 Hz, 1H), 8.40 (d, *J*=4.6, 1.4 Hz, 1H)** |
| **2** | 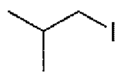 | **1.03 (d, *J*=6.7 Hz, 6H), 1.12 (t, *J*=7.3 Hz, 3H), 2.09-2.13 (m, 1H), 3.99 (d, *J*=6.4 Hz, 2H), 4.16 (q, *J*=7.3 Hz, 2H), 7.33 (d, *J*=8.8 Hz, 1H), 7.54 (dd, *J*=7.7, 4.6 Hz, 1H), 7.78 (dd, *J*=8.8, 1.5 Hz, 1H), 7.83 (d, *J*=1.5 Hz, 1H), 7.19 (d, *J*=7.7 Hz, 1H), 8.79 (d, *J*=4.6 Hz, 1H)** |
| **3** | 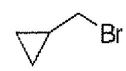 | **0.40-0.42 (m, 2H), 0.60-0.63 (m, 2H), 1.12 (t, *J*=7.0 Hz, 3H), 1.29-1.31 (m, 1H), 4.08 (d, *J*=6.9 Hz, 2H), 4.17 (q, *J*=7.0 Hz, 2H), 7.31 (d, *J*=8.8 Hz, 1H), 7.54 (dd, *J*=7.8, 4.6 Hz, 1H), 7.76 (dd, *J*=8.8, 2.5 Hz, 1H), 7.84 (d, *J*=2.5 Hz, 1H), 8.19 (dd, *J*=7.8, 1.5 Hz, 1H), 8.80 (dd, *J*=4.6, 1.5 Hz, 1H)** |
| **4** | 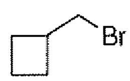 | **1.12 (t, *J*=7.0 Hz, 3H), 1.88-1.96 (m, 4H), 2.08-2.11 (m, 2H), 2.76-2.79 (m, 1H), 4.17-4.21 (m, 4H), 7.34 (d, *J*=8.9 Hz, 1H), 7.54 (dd, *J*=7.8, 4.8 Hz, 1H), 7.77 (dd, *J*=8.9, 2.2 Hz, 1H), 7.83 (d, *J*=2.2 Hz, 1H), 8.19 (dd, *J*=7.8, 1.2 Hz, 1H), 8.80 (dd, *J*=4.8, 1.2 Hz,1H)** |
| **5** | 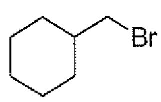 | **1.08-1.29 (m, 8H), 1.72-1.85 (m, 6H), 4.02 (d, *J*=6.1 Hz, 2H), 4.19 (q, *J*=6.9 Hz, 2H), 7.32 (d, *J*=8.9 Hz, 1H), 7.53 (dd, *J*=7.8, 5.1 Hz, 1H), 7.76 (dd, *J*=8.9, 2.2 Hz, 1H), 7.83 (d, *J*=2.2 Hz, 1H), 8.19 (dd, *J*=7.8, 1.9 Hz, 1H), 8.80 (dd, *J*=5.1, 1.9 Hz, 1H)** |
| **6** | 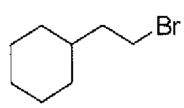 | **0.97-1.24 (m, 8H), 1.67-1.77 (m, 8H), 4.16-4.25 (m, 4H), 7.35 (d, *J*=8.8 Hz, 1H), 7.54 (dd, *J*=7.8, 4.8 Hz, 1H), 7.77 (d, *J*=8.8 Hz, 1H), 7.96 (s, 1H), 8.19 (dd, *J*=7.8, 1.6 Hz, 1H), 8.80 (dd, *J*=4.8, 1.6 Hz, 1H)** |

[Table 4.]

| Example | Alkylating agent R-X (X=I, Br, OMs) | $^1$H-NMR Spectral Data of 7-14 (δ, DMSO-$d_6$) |
|---|---|---|
| **7** | 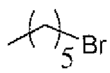 | 0.89 (t, *J*=6.9 Hz, 3H), 1.11 (t, *J*=7.3 Hz, 3H), 1.32-1.36 (m, 4H), 1.45-1.48 (m, 2H), 1.76-1.79 (m, 2H), 4.16-4.22 (m, 4H), 7.33 (d, *J*=8.8 Hz, 1H), 7.54 (dd, *J*=7.8, 5.0 Hz, 1H), 7.77 (d, *J*=8.8, 2.3 Hz, 1H), 7.83 (d, *J*=2.3 Hz, 1H), 8.19 (dd, *J*=7.8, 1.4 Hz, 1H), 8.80 (dd, *J*=5.0, 1.4 Hz, 1H) |
| **8** | 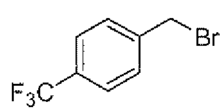 | 1.05 (t, *J*=7.0 Hz, 3H), 4.15 (q, *J*=7.0 Hz, 1H), 5.50 (s, 2H), 7.14 (d, *J*=8.9 Hz, 1H), 7.55 (dd, *J*=7.8, 5.0 Hz, 1H), 7.72-7.89 (m, 6H), 8.21 (dd, *J*=7.8, 1.4 Hz, 1H), 8.81 (dd, *J*=5.0, 1.4 Hz, 1H) |
| **9** | 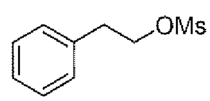 | 1.10 (t, *J*=6.9 Hz, 3H), 3.11 (t, *J*=6.7 Hz, 2H), 4.17 (q, *J*=6.9 Hz, 2H), 4.40 (t, *J*=6.7 Hz, 2H), 7.24-7.39 (m, 6H), 7.53 (dd, *J*=7.8, 5.0 Hz, 1H), 7.76 (d, *J*=8.8, 2.3 Hz, 1H), 7.83 (d, *J*=2.3 Hz, 1H), 8.19 (dd, *J*=7.8, 1.4 Hz, 1H), 8.79 (dd, *J*=5.0, 1.4 Hz, 1H) |
| **10** | 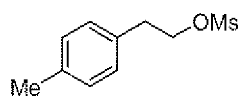 | 1.10 (t, *J*=6.9 Hz, 3H), 2.28 (s, 3H), 3.06 (t, *J*=6.6 Hz, 2H), 4.17 (q, *J*=6.9 Hz, 2H), 4.37 (t, *J*=6.6 Hz, 2H), 7.12 (d, *J*=7.7 Hz, 2H), 7.26 (d, *J*=7.7 Hz, 2H), 7.35 (d, *J*=8.8 Hz, 1H), 7.53 (dd, *J*=7.9, 4.8 Hz, 1H), 7.75 (d, *J*=8.8, 1.9 Hz, 1H), 7.82 (d, *J*=1.9 Hz, 1H), 8.18 (d, *J*=7.9 Hz, 1H), 8.79 (d, *J*=4.8 Hz, 1H) |
| **11** | 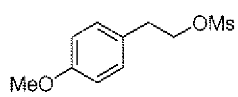 | 1.11 (t, *J*=7.1 Hz, 3H), 3.05 (t, *J*=6.7 Hz, 2H), 3.73 (s, 3H), 4.18 (q, *J*=7.1 Hz, 2H), 4.36 (t, *J*=6.7 Hz, 2H), 6.88 (d, *J*=8.5 Hz, 2H), 7.30 (d, *J*=8.5 Hz, 2H), 7.35 (d, *J*=8.8 Hz, 1H), 7.53 (dd, J=7.9, 4.8 Hz, 1H), 7.75 (d, *J*=8.8, 2.2 Hz, 1H), 7.83 (d, *J*=2.2 Hz, 1H), 8.19 (dd, *J*=7.9, 1.4 Hz, 1H), 8.79 (d, *J*=4.8, 1.4 Hz, 1H) |
| **12** | 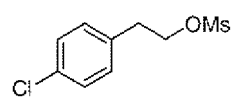 | 1.11 (t, *J*=7.1 Hz, 3H), 3.12 (t, *J*=6.6 Hz, 2H), 4.18 (q, *J*=7.1 Hz, 2H), 4.41 (t, *J*=7.1 Hz, 2H), 7.32-7.42 (m, 5H), 7.53 (dd, *J*=7.9, 4.4 Hz, 1H), 7.77 (d, *J*=8.8, 2.2 Hz, 1H), 7.83 (d, *J*=2.2 Hz, 1H), 8.19 (dd, *J*=7.9, 1.3 Hz, 1H), 8.79 (d, *J*=4.4, 1.3 Hz,1H) |
| **13** | 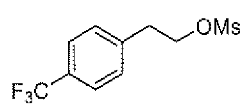 | 1.10 (t, *J*=7.1 Hz, 3H), 3.23 (t, *J*=6.4 Hz, 2H), 4.17 (q, *J*=7.1 Hz, 2H), 4.46 (t, *J*=6.4 Hz, 2H), 7.37 (d, *J*=8.9 Hz, 1H), 7.53 (dd, *J*=7.8, 4.7 Hz, 1H), 7.62 (d, *J*=8.0 Hz, 2H), 7.69 (d, *J*=8.0 Hz, 2H), 7.80 (dd, *J*=8.9, 2.2 Hz, 1H), 7.83 (d, *J*=2.2 Hz, 1H), 8.19 (dd, *J*=7.8, 1.7 Hz, 1H), 8.79 (dd, *J*=4.7, 1.7 Hz, 1H) |
| **14** | 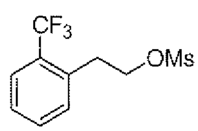 | 1.11 (t, *J*=7.2 Hz, 3H), 3.12 (t, *J*=6.6 Hz, 2H), 4.18 (q, *J*=7.2 Hz, 2H), 4.46 (t, *J*=6.6 Hz, 2H), 7.38 (d, *J*=8.8 Hz, 1H), 7.49-7.55 (m, 2H), 7.65-7.78 (m, 4H), 7.84 (d, *J*=2.2 Hz, 1H), 8.19 (dd, *J*=8.1, 1.2 Hz, 1H), 8.80 (dd, *J*=5.1, 1.2 Hz, 1H) |

[Table 5.]

| Example | Alkylating agent R-X (X=I, Br, OMs) | $^1$H-NMR Spectral Data of 15-22 (δ, DMSO-$d_6$) |
|---|---|---|
| **15** | 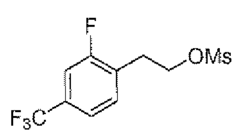 | 1.10 (t, *J*=7.2 Hz, 3H), 3.25 (t, *J*=6.3 Hz, 2H), 4.17 (q, *J*=7.2 Hz, 2H), 4.48 (t, *J*=6.3 Hz, 2H), 7.38 (d, *J*=8.8 Hz, 1H), 7.52-7.82 (m, 6H), 8.19 (d, *J*=7.9 Hz, 1H), 8.79 (d, *J*=4.5 Hz, 1H) |

(continued)

| Example | Alkylating agent R-X (X=I, Br, OMs) | $^1$H-NMR Spectral Data of 15-22 (δ, DMSO-$d_6$) |
|---|---|---|
| **16** | 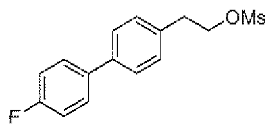 | **1.11 (t, *J*=7.0 Hz, 3H), 3.16 (t, *J*=6.7 Hz, 2H), 4.18 (q, *J*=7.0 Hz, 2H), 4.44 (t, *J*=6.7 Hz, 2H), 7.25-7.84 (m, 12H), 8.18 (d, *J*=1.4 Hz, 1H), 8.79 (d, *J*=4.6 Hz, 1H)** |
| **17** | 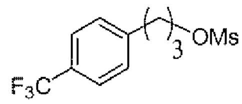 | **1.11 (t, *J*=7.2 Hz, 3H), 2.13-2.17 (m, 2H), 2.90 (t, *J*=6.2 Hz, 2H), 4.17-4.22 (m, 4H), 7.33 (d, *J*=8.8 Hz, 1H), 7.49 (d, *J*=8.0 Hz, 2H), 7.55 (dd, *J*=7.9, 5.0 Hz, 1H), 7.66 (d, *J*=8.0 Hz, 2H), 7.76 (d, *J*=8.8, 2.2 Hz, 1H), 7.86 (d, *J*=2.2 Hz, 1H), 8.20 (dd, *J*=7.9, 1.3 Hz, 1H), 8.81 (d, *J*=5.0, 1.3 Hz, 1H)** |
| **18** | 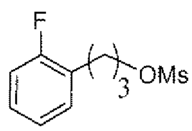 | **1.11 (t, *J*=7.0 Hz, 3H), 2.07-2.12 (m, 2H), 2.85 (t, *J*=7.6 Hz, 2H), 4.16-4.23 (m, 4H), 7.14-7.17 (m, 2H), 7.32-7.53 (m, 3H), 7.54 (dd, *J*=7.8, 5.0 Hz, 1H), 7.77 (dd, *J*=8.5, 2.1 Hz, 1H), 7.86 (d, *J*=2.1 Hz, 1H), 8.20 (dd, *J*=7.8, 1.3 Hz, 1H), 8.80 (d, *J*=5.0, 1.3 Hz, 1H)** |
| **19** | 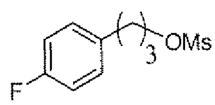 | **1.11 (t, *J*=7.1 Hz, 3H), 2.06-2.12 (m, 2H), 2.80 (t, *J*=7.4 Hz, 2H), 4.17-4.21 (m, 4H), 7.10-7.13 (m, 2H), 7.27-7.30 (m, 3H), 7.54 (dd, *J*=8.1, 4.7 Hz, 1H), 7.78 (dd, *J*=8.5, 2.2 Hz, 1H), 7.86 (d, *J*=2.2 Hz, 1H), 8.20 (dd, *J*=8.1, 1.5 Hz, 1H), 8.80 (d, *J*=4.7, 1.5 Hz, 1H)** |
| **20** | 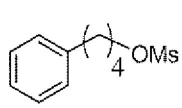 | **1.11 (t, *J*=7.2 Hz, 3H), 1.77-1.79 (m, 4H), 2.68 (t, *J*=7.0 Hz, 2H), 4.16-4.24 (m, 4H), 7.18-7.33 (m, 6H), 7.53 (dd, *J*=8.1, 4.5 Hz, 1H), 7.80 (dd, *J*=8.5, 2.1 Hz, 1H), 7.83 (d, *J*=2.1 Hz, 1H), 8.20 (dd, *J*=8.1, 1.2 Hz, 1H), 8.80 (d, *J*=4.5, 1.2 Hz, 1H)** |
| **21** | 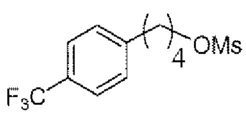 | **1.11 (t, *J*=7.0 Hz, 3H), 1.79-1.81 (m, 4H), 2.79 (t, *J*=7.0 Hz, 2H), 4.16-2.24 (m, 4H), 7.33 (d, *J*=8.9 Hz, 1H), 7.65 (d, *J*=7.9 Hz, 2H), 7.54 (dd, *J*=7.8, 4.9 Hz, 1H), 7.65 (d, *J*=7.9 Hz, 2H), 7.77 (dd, *J*=8.9, 2.0 Hz, 1H), 7.84 (d, *J*=2.0 Hz, 1H), 8.19 (d, *J*=7.8 Hz, 1H), 8.80 (d, *J*=4.9 Hz, 1H)** |
| **22** | 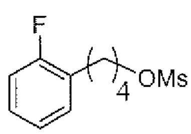 | **1.11 (t, *J*=6.9 Hz, 3H), 1.76-1.84 (m, 4H), 2.72 (t, *J*=7.4 Hz, 2H), 4.19 (q, *J*=6.9 Hz, 2H), 4.24 (d, *J*=5.8 Hz, 1H), 7.12-7.15 (m, 2H), 7.23-7.25 (m, 1H), 7.30-7.34 (m, 2H), 7.54 (dd, *J*=7.9, 4.8 Hz, 1H), 7.77 (d, *J*=8.6, 2.2 Hz, 1H), 7.84 (d, *J*=2.2 Hz, 1H), 8.19 (d, *J*=7,9, 1.4 Hz, 1H), 8.80 (d, *J*=4.8, 1.4 Hz, 1H)** |

<Example 23> Ethyl 2-[3-cyano-4-(3-phenoxypropoxy)-phenyl]nicotinate

Potassium carbonate (0.22 g, 1.6 mmol) was added at room temperature to a solution of ethyl 2-[4-(3-chloropropoxy)-3-cyanophenyl]nicotinate (Referential Example 7a) (0.5 g, 1.45 mmol) and phenol (0.13 mL, 1.45 mmol) in DMF (20 mL) and the mixture was stirred at 80˚C for 15 hours. The reaction mixture was poured into ice water followed by extracting with ethyl acetate and the organic layer was dried. The residue prepared by evaporation of the solvent was purified by silica gel column chromatography (benzene/ethyl acetate = 7/1) to give the title compound (37 g, 63%) as an oily product.
In accordance with the same method as in Example 23, the compounds of Examples 24 to 29 shown in Table 6 were produced starting from the corresponding phenols. Physical and chemical data of the compounds produced in Examples 23 to 29 are shown in Table 6.

[Table 6.]

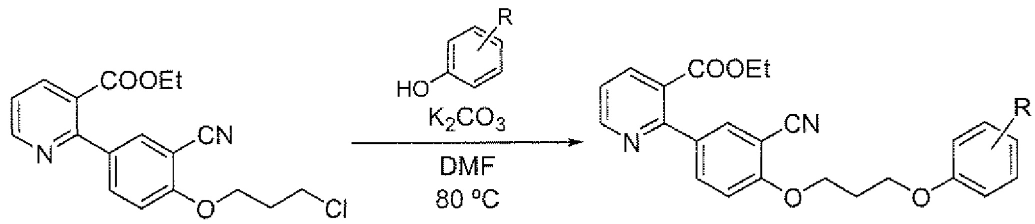


**7** → **23-29**

| Example | R | $^1$H-NMR Spectral Data of 23-29 (δ, DMSO-$d_6$) |
|---|---|---|
| **23** | **H** | **1.10 (t, *J*=7.3 Hz, 3H), 2.24-2.28 (m, 2H), 4.15-4.20 (m, 4H), 4.39 (t, *J*=6.1 Hz, 2H), 6.94-6.98 (m, 3H), 7.28-7.31 (m, 2H), 7.38 (d, *J*=8.8 Hz, 1H), 7.54 (dd, *J*=7.8, 4.6 Hz, 1H), 7.79 (dd, *J*=8.8, 2.2 Hz, 1H), 7.85 (d, *J*=2.2 Hz, 1H), 8.20 (dd, *J*=7.8, 1.4 Hz, 1H), 8.80 (dd, *J*=4.6, 1,4 Hz, 1H)** |
| **24** | **2-F** | **1.11 (t, *J*=7.0 Hz, 3H), 2.28-2.31 (m, 2H), 4.18 (q, *J*=7.0 Hz, 1H), 4.28 (t, *J*=6.2 Hz, 2H), 4.39 (t, *J*=6.1 Hz, 2H), 6.94-6.96 (m, 1H), 7.14-7.22 (m, 3H), 7.39 (d, *J*=8.8 Hz, 1H), 7.54 (dd, *J*=7.8, 5.0 Hz, 1H), 7.79 (dd, *J*=8.8, 2.2 Hz, 1H), 7.86 (d, *J*=2.2 Hz, 1H), 8.20 (dd, *J*=7.8, 1.3 Hz, 1H), 8.81 (dd, *J*=5.0, 1.3 Hz, 1H)** |
| **25** | **3-F** | **1.11 (t, *J*=6.8 Hz, 3H), 2.25-2.28 (m, 2H), 4.16-4.22 (m, 4H), 4.38 (t, *J*=6.1 Hz, 2H), 6.74-6.77 (m, 1H), 6.82-6.86 (m, 2H), 7.30-7.32 (m, 1H), 7.38 (d, *J*=8.8 Hz, 1H), 7.54 (dd, *J*=7.7, 5.0 Hz, 1H), 7.78 (dd, *J*=8.8, 2.1 Hz, 1H), 7.85 (d, *J*=2.1 Hz, 1H), 8.19 (d, *J*=7.7 Hz, 1H), 8.80 (d, *J*=5.0 Hz, 1H)** |
| **26** | **4-F** | **1.10 (t, *J*=7.3 Hz, 3H), 2.22-2.27 (m, 2H), 5.15-4.20 (m, 4H), 4.82 (t, *J*=5.0 Hz, 2H), 6.97-6.99 (m, 2H), 7.10-7.14 (m, 2H), 7.38 (d, *J*=8.9 Hz, 1H), 7.54 (dd, *J*=8.0, 4.7 Hz, 1H), 7.78 (dd, *J*=8.9, 2.2 Hz, 1H), 7.85 (d, *J*=2.2 Hz, 1H), 8.20 (d, *J*=8.0, 1.4 Hz, 1H), 8.80 (d, *J*=4.7, 1.4 Hz, 1H)** |
| **27** | **2-Cl, 4-$OCF_3$** | **1.10 (t, *J*=6.9 Hz, 3H), 2.29-2.32 (m, 2H), 4.18 (q, *J*=6.9 Hz, 2H), 4.32 (t, *J*=6.1 Hz, 2H), 4.42 (t, *J*=6.2 Hz, 2H), 7.31-7.38 (m, 3H), 7.54-7.57 (m, 2H), 7.78 (s, 1H), 7.86 (d, *J*=2.2 Hz, 1H), 8.20 (dd, *J*=7.9, 1.4 Hz, 1H), 8.81 (dd, *J*=4.8, 1.4 Hz, 1H)** |

(continued)

| Example | R | $^1$H-NMR Spectral Data of 23-29 (δ, DMSO-$d_6$) |
|---|---|---|
| **28** | **2,5-diCl, 4-$OCF_3$** | **1.11 (t, *J*=7.0 Hz, 3H), 2.30-2.33 (m, 2H), 4.18 (q, *J*=7.0 Hz, 2H), 4.24 (t, *J*=5.9 Hz, 2H), 4.46 (t, *J*=5.8 Hz, 2H), 7.40 (d, *J*=8.8 Hz, 1H), 7.55 (d, *J*=7.9, 4.8 Hz, 1H), 7.71 (s, 2H), 7.79 (dd, *J*=8.7, 2.1 Hz, 1H), 7.86 (d, *J*=2.1 Hz, 1H), 8.20 (dd, *J*=7.9, 1.5 Hz, 1H), 8.80 (dd, *J*=4.8, 1.5 Hz, 1H)** |
| **29** | **4-OPh** | **1.11 (t, *J*=7.1 Hz, 3H), 2.24-2.27 (m, 2H), 4.15-4.19 (m, 4H), 4.39 (t, *J*=6.1 Hz, 2H), 6.92 (d, *J*=8.0 Hz, 2H), 7.00-7.32 (m, 5H), 7.34-7.40 (m, 3H), 7.54 (dd, *J*=7.8, 4.9 Hz, 1H), 7.79 (dd, *J*=8.0, 2.1 Hz, 1H), 7.85 (d, *J*=2.1 Hz, 1H), 8.20 (d, *J*=7.8 Hz, 1H), 8.80 (d, *J*=4.9 Hz, 1H)** |

<Example 30> Ethyl 2-[3-cyano-4-(2,3-dimethylbutoxy)-phenyl]nicotinate

Palladium acetate (0.14 g, 0.6 mmol), tris(2-methylphenyl) phosphine (0.37 g, 1.7 mmol) and a 10% aqueous solution of sodium carbonate (21 mL, 18.2 mmol) were added in an argon atmosphere to a solution of ethyl 2-chloronicotinate (Referential Example 4a) (1.9 g, 10.1 mmol) and 3-cyano-4-(2,2-dimethylbutyloxy)phenyl boronic acid (Referential Example 12a) (3.0 g, 12.1 mmol) in DME (100 mL) and the mixture was stirred at 80˚C for 4 hours. To the residue prepared by evaporation of the solvent was added dichloromethane followed by washing with water. After the organic layer was dried, the residue prepared by evaporation of the solvent was purified by silica gel column chromatography (hexane/ethyl acetate = 7/3) to give the title compound (2.6 g, 72%) as an oily product.
In accordance with the same method as in Example 30, the compounds of Examples 31 to 42 shown in Tables 7 and 8 were produced starting from the corresponding phenylboronic acids. Physical and chemical data of the compounds produced in Examples 30 to 42 are shown in Tables 7 and 8.
[Table 7.]

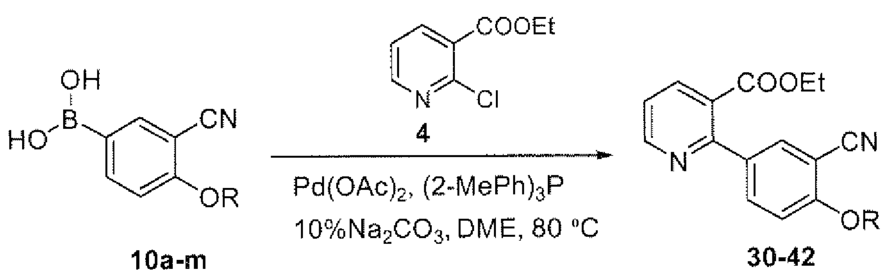


**10a-m** **4** **30-42**

| Example | R | $^1$H-NMR Spectral Data of 30-35 (δ, DMSO-$d_6$) |
|---|---|---|
| **30** | 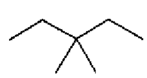 | **0.87 (t, *J*=7.4 Hz, 3H), 1.00 (s, 6H), 1.13 (t, *J*=6.9 Hz, 3H), 1.43 (q, *J*=7.4 Hz, 2H), 3.89 (s, 2H), 4.19 (q, *J*=6.9 Hz, 2H), 7.34 (d, *J*=8.9 Hz, 1H), 7.54 (dd, *J*=7.8, 4.7 Hz, 1H), 7.77 (dd, *J*=8.9, 2.2 Hz, 1H), 7.84 (d, *J*=2.2 Hz, 1H), 8.19 (d, *J*=7.8, 1.2 Hz,1H), 8.80 (dd, *J*=4.7, 1.2 Hz, 1H)** |

(continued)

| Example | R | $^1$H-NMR Spectral Data of 30-35 (δ, DMSO-$d_6$) |
|---|---|---|
| 31 | 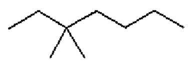 | 0.88 (t, *J*=6.5 Hz, 3H), 1.01 (s, 6H), 1.12 (t, *J*=7.1 Hz, 3H), 1.26-1.30 (m, 4H), 1.39 (t, *J*=8.1 Hz, 2H), 3.88 (s, 2H), 4.19 (q, *J*=7.1 Hz, 2H), 7.33 (d, *J*=8.8 Hz, 1H), 7.54 (dd, *J*=7.8, 4.9 Hz, 1H), 7.76 (dd, *J*=8.8, 2.1 Hz, 1H), 7.83 (d, *J*=2.1 Hz, 1H), 8.19 (d, *J*=7.8 Hz, 1H), 8.80 (dd, *J*=4.9 Hz, 1H) |
| 32 | 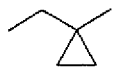 | 0.45 (t, *J*=4.1 Hz, 2H), 0.59 (t, *J*=4.1 Hz, 2H), 1.13 (t, *J*=7.0 Hz, 3H), 1.23 (s, 3H), 4.01 (s, 2H), 4.19 (q, *J*=7.0 Hz, 2H), 7.28 (d, *J*=8.8 Hz, 1H), 7.54 (dd, *J*=7.8, 4.8 Hz, 1H), 7.76 (dd, *J*=8.8, 2.2 Hz, 1H), 7.84 (d, *J*=2.2 Hz, 1H), 8.19 (dd, *J*=7.8, 1.3 Hz, 1H), 8.80 (dd, *J*=4.8, 1.3 Hz, 1H) |
| 33 | 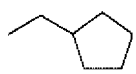 | 1.12 (t, *J*=7.0 Hz, 2H), 1.37-1.41 (m, 2H), 1.55-1.58 (m, 2H), 1.63-1.65 (m, 2H), 1.80-1.82 (m, 2H), 2.36-2.39 (m, 1H), 4.10 (d, *J*=6.8 Hz, 2H), 4.19 (q, *J*=7.0 Hz, 2H), 7.34 (d, *J*=8.8 Hz, 1H), 7.53 (dd, *J*=7.8, 4.8 Hz, 1H), 7.76 (dd, *J*=8.8, 2.0 Hz, 1H), 7.83 (d, *J*=2.0 Hz, 1H), 8.19 (d, *J*=7.8 Hz, 1H), 8.80 (d, *J*=4.8 Hz, 1H) |
| 34 | 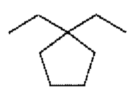 | 0.87 (t, *J*=7.4 Hz, 3H), 1.12 (t, *J*=7.2 Hz, 3H), 1.45-1.66 (m, 10H), 3.95 (s, 2H), 4.19 (q, *J*=7.2 Hz, 2H), 7.36 (d, *J*=8.8 Hz, 1H), 7.54 (dd, *J*=7.8, 5.0 Hz, 1H), 7.77 (dd, *J*=8.8, 2.1 Hz, 1H), 7.83 (d, *J*=2.1 Hz, 1H), 8.19 (dd, *J*=7.8, 1.6 Hz, 1H), 8.80 (dd, *J*=5.0, 1.6 Hz, 1H) |
| 35 | 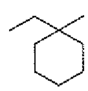 | 1.06 (s, 3H), 1.13 (t, *J*=7.1 Hz, 3H), 1.37-1.49 (m, 10H), 3.93 (s, 2H), 4.19 (q, *J*=7.1 Hz, 2H), 7.35 (d, *J*=8.8 Hz, 1H), 7.53 (dd, *J*=7.8, 5.0 Hz, 1H), 7.77 (dd, *J*=8.8, 2.2 Hz, 1H), 7.83 (d, *J*=2.2 Hz, 1H), 8.19 (dd, *J*=7.8, 1.4 Hz, 1H), 8.80 (dd, *J*=5.0, 1.4 Hz, 1H) |

[Table 8.]

| Example | R | $^1$H-NMR Spectral Data of 36-42 (δ, DMSO-$d_6$) |
|---|---|---|
| 36 | 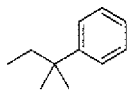 | 1.13 (t, *J*=6.9 Hz, 3H), 1.48 (s, 6H), 4.17-4.22 (m, 4H), 7.22-7.24 (m, 1H), 7.33-7.36 (m, 3H), 7.51-7.54 (m, 3H), 7.76 (d, *J*=8.6 Hz, 1H), 7.82 (d, *J*=2.0 Hz, 1H), 8.18 (dd, *J*=8.1, 1.2 Hz, 1H), 8.79 (dd, *J*=4.5, 1.2 Hz, 1H) |
| 37 | 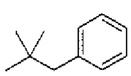 | 1.10 (t, *J*=7.2 Hz, 3H), 1.43 (s, 6H), 3.56 (s, 2H), 4.19 (q, *J*=7.2 Hz, 2H), 7.26-7.36 (m, 5H), 7.48 (d, *J*=8.8 Hz, 1H), 7.55 (dd, *J*=7.8, 4.6 Hz, 1H), 7.72 (dd, *J*=8.8, 2.3 Hz, 1H), 7.87 (d, *J*=2.3 Hz, 1H), 8.21 (dd, *J*=7.8, 1.4 Hz, 1H), 8.81 (dd, *J*=4.6, 1.4 Hz, 1H) |

(continued)

| Example | R | $^1$H-NMR Spectral Data of 36-42 (δ, DMSO-$d_6$) |
|---|---|---|
| **38** | 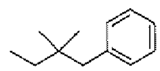 | **1.03 (s, 6H), 1.23 (t, *J*=7.1 Hz, 3H), 2.51 (s, 2H), 3.80 (s, 2H), 4.20 (q, *J*=7.1 Hz, 2H), 7.14-7.30 (m, 6H), 7.55 (dd, *J*=8.7, 2.0 Hz, 1H), 7.77 (dd, *J*=8.7, 2.0 Hz, 1H), 7.89 (d, *J*=2.0 Hz, 1H), 8.20 (d, *J*=7.8 Hz, 1H), 8.82 (d, *J*=4.9 Hz, 1H)** |
| **39** | 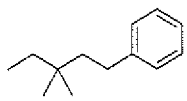 | **1.10-1.13 (m, 9H), 1.67-1.71 (m, 2H), 2.59-2.62 (m, 2H), 4.00 (s, 2H), 4.19 (q, *J*=6.9 Hz, 2H), 7.15-7.28 (m, 5H), 7.35 (d, *J*=8.9 Hz, 1H), 7.54 (dd, *J*=8.0, 4.7 Hz, 1H), 7.79 (dd, *J*=8.9, 2.2 Hz, 1H), 7.85 (d, *J*=2.2 Hz, 1H), 8.20 (dd, *J*=8.0, 1.2 Hz, 1H), 8.80 (dd, *J*=4.7, 1.2 Hz, 1H)** |
| **40** | 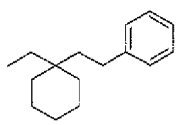 | **1.10 (t, *J*=7.2 Hz, 3H), 1.50-1.57 (m, 10H), 1.74-1.78 (m, 2H), 2.51-2.57 (m, 2H), 4.08 (s, 2H), 4.19 (q, *J*=6.9 Hz, 2H), 7.14-7.26 (m, 5H), 7.41 (d, *J*=8.8 Hz, 1H), 7.54 (dd, *J*=7.8, 4.7 Hz, 1H), 7.79 (dd, *J*=8.8, 2.2 Hz, 1H), 7.85 (d, *J*=2.2 Hz, 1H), 8.20 (dd, *J*=7.8, 1.4 Hz, 1H), 8.81 (dd, *J*=4.7, 1.4 Hz, 1H)** |
| **41** | 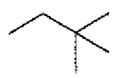 | **1.06 (s, 9H), 1.14 (t, *J*=7.1 Hz, 3H), 3.87 (s, 2H), 4.20 (q, *J*=7.1 Hz, 2H), 7.32 (d, *J*=8.8 Hz, 1H), 7.54 (dd, *J*=7.7, 4.9 Hz, 1H), 7.77 (dd, *J*=8.8, 1.5 Hz, 1H), 7.81 (d, *J*=1.5 Hz, 1H), 8.19 (d, *J*=7.7 Hz,1H), 8.80 (d, *J*=4.9 Hz, 1H)** |
| **42** | 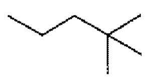 | **1.00 (s, 9H), 1.12 (t, *J*=7.0 Hz, 3H), 1.73 (d, *J*=6.8 Hz, 2H), 4.19 (q, *J*=7.0 Hz, 2H), 4.26 (t, *J*=6.8 Hz, 2H), 7.38 (d, *J*=8.9 Hz, 1H), 7.54 (dd, *J*=7.8, 4.7 Hz, 1H), 7.77 (dd, *J*=8.9, 2.2 Hz, 1H), 7.83 (d, *J*=2.2 Hz, 1H), 8.19 (dd, *J*=7.8, 1.2 Hz, 1H), 8.80 (d, *J*=4.7, 1.2 Hz, 1H)** |

<Example 43> 2-(3-Cyano-4-methoxyphenyl)nicotinic acid

To a solution of ethyl 2-(3-eyano-4-methoxyphenyl)- nicotinate (Example 1) (0.45 g, 1.6 mmol) in ethanol (20 mL) was added a 1 mol/L aqueous solution of sodium hydroxide (1.9 mL, 1.9 mmol) and the mixture was heated with stirring at 70˚C for 1 hour. Water was added to the residue prepared by evaporation of the solvent, the mixture was acidified with diluted hydrochloric acid and the crystals separated out therefrom were filtered to give the title compound (0.28 g, 69%).
In accordance with the same method as in Example 43, the compounds of Examples 44 to 84 shown in Tables 9 to 14 were produced starting from the corresponding esters. Physical and chemical data of the compounds produced in Examples 44 to 84 are shown in Tables 9 to 14.

[Table 9.]

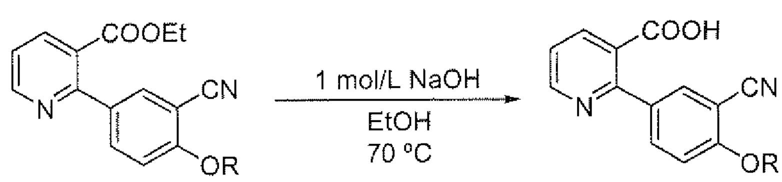

(continued)

| Example | R | Physical and Chemical Data of 43-48 | |
|---|---|---|---|
| | | Melting Point(˚C) | $^1$H-NMR (DMSO-$d_6$)(δ) |
| 43 | Me | 254-255 | $^1$H-NMR (DMSO-$d_6$) δ: 3.98 (s, 3H), 7.34 (d, *J*=8.5 Hz, 1H), 7.50 (dd, *J*=7.7, 4.9 Hz, 1H), 7.84-7.86 (m, 2H), 8.18 (d, *J*=7.7 Hz, 1H), 8.76 (d, *J*=4.9 Hz, 1H), 13.20-13.28 (br, 1H) |
| 44 | 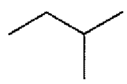 | 178-179 | 1.03 (d, *J*=6.7 Hz, 6H), 2.08-2.11 (m, 1H), 3.99 (d, *J*=6.5 Hz, 2H), 7.32 (d, *J*=8.8 Hz, 1H), 7.50 (dd, *J*=7.8, 4.7 Hz, 1H), 7.81-7.86 (m, 2H), 8.17 (dd, *J*=7.8, 1.4 Hz, 1H), 8.76 (dd, *J*=4.7, 1.4 Hz, 1H), 13.37 (s, 1H) |
| 45 | 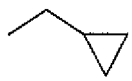 | 203-204 | 0.39-0.41 (m, 2H), 0.61-0.64 (m, 2H), 1.28-1.33 (m, 1H), 4.07 (d, *J*=7.0 Hz, 2H), 7.30 (d, *J*=8.8 Hz, 1H), 7.50 (dd, *J*=7.7, 4.5 Hz, 1H), 7.81 (d, *J*=8.8 Hz, 1H), 7.85 (s, 1H), 8.16 (d, *J*=7.7 Hz, 1H), 8.76 (d, *J*=4.5 Hz, 1H), 13.36 (s, 1H) |
| 46 | 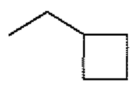 | 204-205 | 1.89-1.92 (m, 4H), 2.10-2.12 (m, 2H), 2.77-2.80 (m, 1H), 4.18 (d, *J*=6.4 Hz, 2H), 7.33 (d, *J*=8.8 Hz, 1H), 7.50 (dd, *J*=7.7, 4.9 Hz, 1H), 7.81-7.85 (m, 1H), 7.85 (s, 2H), 8.17 (d, *J*=7.7 Hz, 1H), 8.76 (d, *J*=4.9 Hz, 1H), 8.76 (d, *J*=4.9 Hz, 1H), 13.20-13.30 (br, 1H) |
| 47 | 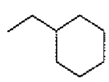 | 220-221 | 1.10-1.30 (m, 5H), 1.66-1.86 (m, 6H), 4.02 (d, *J*=6.0 Hz, 2H), 7.33 (d, *J*=8.8 Hz, 1H), 7.53 (dd, *J*=7.8, 4.9 Hz, 1H), 7.82 (dd, *J*=8.8, 2.2 Hz, 1H), 7.86 (d, *J*=2.2 Hz, 1H), 8.20 (d, *J*=7.8 Hz, 1H), 8.77 (d, *J*=4.9 Hz, 1H), 13.20-13.30 (br, 1H) |
| 48 | 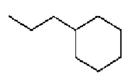 | 171-172 | 0.98-1.02 (m, 2H), 1.17-1.24 (m, 3H), 1.51-1.78 (m. 8H), 4.23 (t, *J*=6.6 Hz, 2H), 7.34 (d, *J*=8.8 Hz, 1H), 7.50 (dd, *J*=7.8, 5.0 Hz, 1H), 7.81-7.85 (m, 2H), 8.18 (d, *J*=7.8 Hz, 1H), 8.76 (d, *J*=5.0 Hz, 1H), 13.25-13.30 (br, 1H) |

[Table 10.]

| Example | R | Physical and Chemical Data of 49-56 | |
|---|---|---|---|
| | | Melting Point(˚C) | $^1$H-NMR(DMSO-$d_6$)(δ) |
| 49 | 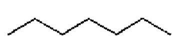 | 187-188 | 0.89 (t, *J*=7.0 Hz, 3H), 1.31-1.34 (m, 4H), 1.44-1.48 (m. 2H), 1.77-1.81 (m, 2H), 4.20 (t, *J*=6.4 Hz, 2H), 7.33 (d, *J*=8.8 Hz, 1H), 7.50 (dd, *J*=7.8, 4.8 Hz, 1H), 7.81.-7.85 (m, 2H), 8.16 (dd, *J*=7.8, 1.2 Hz, 1H), 8.76 (dd, *J*=4.8, 1.2 Hz, 1H), 13.40-13.50 (br, 1H) |
| 50 | 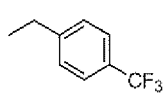 | 226-227 | 5.48 (s, 2H), 7.47 (d, *J*=8.9 Hz, 1H), 7.51 (dd, *J*=7.8, 4.9 Hz, 1H), 7.74 (d, *J*=8.1 Hz, 2H), 7.82-7.87 (m, 3H), 7.91 (d, *J*=1.9 Hz, 1H), 8.19 (d, *J*=7.8 Hz, 1H), 8.76 (d, *J*=4.9 Hz, 1H), 13.20-13.40 (br, 1H) |

(continued)

| Example | R | Physical and Chemical Data of 49-56 | |
|---|---|---|---|
| | | Melting Point(˚C) | $^1$H-NMR(DMSO-$d_6$)($\delta$) |
| **51** | 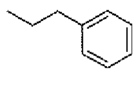 | **180-181** | **3.12 (t, *J*=6.7 Hz, 2H), 4.40 (t, *J*=6.7 Hz, 2H), 7.24-7.40 (m, 6H), 7.50 (dd, *J*=7.8, 4.9 Hz, 1H), 7.81 (d, *J*=8.8 Hz, 1H), 7.85 (d, *J*=2.2 Hz, 1H), 8.16 (dd, *J*=7.8, 1.3 Hz, 1H), 8.75 (dd, *J*=4.9, 1.3 Hz, 1H), 13.25-13.35 (br, 1H)** |
| **52** | 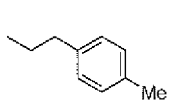 | **192-193** | **2.28 (s, 3H), 3.06 (t, *J*=6.6 Hz, 2H), 4.36 (t, *J*=6.6 Hz, 2H), 7.13 (d, *J*=7.6 Hz, 2H), 7.26 (d, *J*=7.6 Hz, 2H), 7.34 (d, *J*=8.0 Hz, 1H), 7.50 (dd, *J*=7.6, 4.9 Hz, 1H), 7.80 (d, *J*=8.0 Hz, 1H), 7.84 (s, 1H), 8.16 (d, *J*=7.6 Hz, 1H), 8.75 (d, *J*=4.9 Hz, 1H), 13.30-13.40 (br, 1H)** |
| **53** | 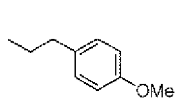 | **183-184** | **3.05 (t, *J*=6.7 Hz, 2H), 4.35 (t, *J*=6.7 Hz, 2H), 6.88 (d, *J*=8.5 Hz, 2H), 7.30 (d, *J*=8.5 Hz, 2H), 7.34 (d, *J*=8.8 Hz, 1H), 7.50 (dd, *J*=7.8, 4.7 Hz, 1H), 7.81 (dd, *J*=8.8, 2.2 Hz, 1H), 7.85 (d, *J*=2.2 Hz, 1H), 8.19 (dd, *J*=7.8, 1.6 Hz, 1H), 8.75 (dd, *J*=4.7, 1.6 Hz, 1H), 13.30-13.40 (br, 1H)** |
| **54** | 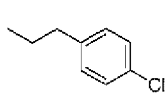 | **196-197** | **3.12 (t, *J*=6.5 Hz, 2H), 4.40 (t, *J*=6.5 Hz, 2H), 7.34-7.42 (m, 5H), 7.50 (dd, *J*=7.7, 4.7 Hz, 1H), 7.80-7.85 (m, 2H), 8.17 (d, *J*=7.9 Hz, 1H), 8.76 (d, *J*=4.7 Hz, 1H), 13.20-13.30 (br, 1H)** |
| **55** | 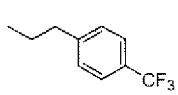 | **182-183** | **3.23 (t, *J*=6.1 Hz, 2H), 4.46 (t, *J*=6.1 Hz, 2H), 7.36 (d, *J*=8.8 Hz, 1H), 7.49-7.51 (m, 1H), 7.62 (d, *J*=7.8 Hz, 2H), 7.69 (d, *J*=7.8 Hz, 2H), 7.81-7.85 (m, 2H), 8.17 (d, *J*=7.8 Hz, 1H), 8.76 (d, *J*=4.2 Hz, 1H), 13.34 (s, 1H)** |
| **56** | 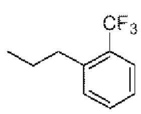 | **187-188** | **3.30 (t, *J*=6.5 Hz, 2H), 4.46 (t, *J*=6.5 Hz, 2H), 7.37 (d, *J*=8.9 Hz, 1H), 7.49-7.51 (m, 2H), 7.65-7.86 (m, 5H), 8.16 (d, *J*=7.7, 1.5 Hz, 1H), 8.76 (dd, *J*=5.0,1.5 Hz, 1H), 13.25-13.30 (br, 1H)** |

[Table 11.]

| Example | R | Physical and Chemical Data of 57-63 | |
|---|---|---|---|
| | | Melting Point(˚C) | $^1$H-NMR (DMSO-$d_6$)($\delta$) |
| **57** | 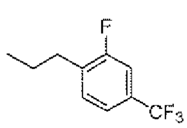 | **174-175** | **3.25 (t, *J*=6.0 Hz, 2H), 4.47 (t, *J*=6.5 Hz, 2H), 7.37 (d, *J*=8.8 Hz, 1H), 7.50 (dd, *J*=7.7, 4.9 Hz, 1H), 7.57 (d, *J*=7.9 Hz, 1H), 7.65 (d, *J*=9.9 Hz, 1H), 7.71 (d, *J*=7.9 Hz, 1H), 7.81-7.84 (m, 2H), 8.17 (d, *J*=7.7 Hz, 1H), 8.80 (d, *J*=4.9 Hz, 1H), 13.20-13.40 (br, 1H)** |
| **58** | 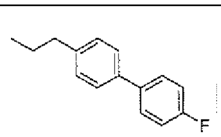 | **231-232** | **3.16 (t, *J*=6.1 Hz, 2H), 4.44 (t, *J*=6.1 Hz, 2H), 7.26-7.30 (m, 2H), 7.38 (d, *J*=8.9 Hz, 1H), 7.47-7.51 (m, 3H), 7.58-7.62 (m, 2H), 7.69-7.12 (m, 2H), 7.81-7.86 (m, 2H), 8.17 (d, *J*=7.7 Hz, 1H), 8.76 (d, *J*=4.1 Hz, 1H), 13.20-13.40 (br, 1H)** |
| **59** | 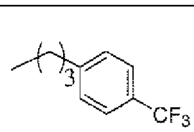 | **183-184** | **2.11-2.15 (m, 2H), 2.90 (t, *J*=7.3 Hz, 2H), 4.21 (t, *J*=5.9 Hz, 2H), 7.32 (d, *J*=8.8 Hz, 1H), 7.48-7.51 (m, 3H), 7.66 (d, *J*=7.8 Hz, 2H), 7.81 (d, *J*=8.8 Hz, 1H), 7.87 (s, 1H), 8.17 (d, *J*=7.6 Hz, 1H), 8.76 (d, *J*=4.7 Hz, 1H), 13.30 (s, 1H)** |

(continued)

| Example | R | Physical and Chemical Data of 57-63 | |
|---|---|---|---|
| | | Melting Point(˚C) | $^1$H-NMR (DMSO-$d_6$)(δ) |
| **60** | 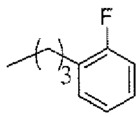 | **173-174** | **1.90-2.10 (m, 2H), 2.85 (t, *J*=7.1 Hz, 2H), 4.20-4.22 (m, 2H), 7.15-7.18 (m, 2H), 7.26-7.35 (m, 3H), 7.49-7.52 (m, 1H), 7.81-7.87 (m, 2H), 8.18 (d, *J*=7.4 Hz, 1H), 8.77 (d, *J*=3.1 Hz, 1H), 13.37 (s, 1H)** |
| **61** | 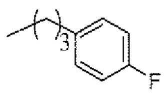 | **177-178** | **2.06-2.12 (m, 2H), 2.80 (t, *J*=7.5 Hz, 2H), 4.18 (t, *J*=6.1 Hz, 2H), 7.10-7.14 (m, 2H), 7.27-7.30 (m, 3H), 7.50-7.52 (m, 1H), 7.82 (d, *J*=7.2 Hz, 1H), 7.87 (d, *J*=2.1 Hz, 1H), 8.18 (dd, *J*=8.1, 1.3 Hz, 1H), 8.77 (dd, *J*=4.6, 1.3 Hz, 1H), 13.37 (s, 1H)** |
| **62** | 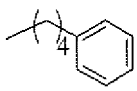 | **163-164** | **1.78-1.79 (m, 4H), 2.67-2.69 (m, 2H), 4.21-4.22 (m, 2H), 7.17-7.33 (m, 6H), 7.50 (dd, *J*=7.7, 4.7 Hz, 1H), 7.81-7.85 (m, 2H), 8.17 (d, *J*=7.7 Ilz, 1H), 8.76 (d, *J*=4.7 Hz, 1H), 13.36 (s, 1H)** |
| **63** | 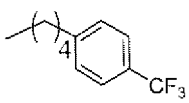 | **165-166** | **1.80-1.82 (m, 4H), 2.78-2.80 (m, 2H), 4.22-4.24 (m, 2H), 7.33 (d, *J*=8.7 Hz, 1H), 7.47-7.52 (m, 3H), 7.65 (d, *J*=7.7 Hz, 2H), 7.82-7.85 (m, 2H), 8.17 (d, *J*=7.7 Hz, 1H), 8.76 (d, *J*=4.2 Hz,1H) 13.20-13.40 (br, 1H)** |

[Table 12.]

| Example | R | Physical and Chemical Data of 64-70 | |
|---|---|---|---|
| | | Melting Point(˚C) | $^1$H-NMR (DMSO-$d_6$)( δ ) |
| **64** | 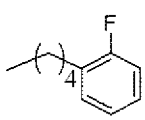 | **151-152** | **1.77-1.84 (m, 4H), 2.72 (t, *J*=7.3 Hz, 2H), 4.23 (t, *J*=5.7 Hz, 2H), 7.31-7.34 (m, 2H), 7.20-7.22 (m, 1H), 7.31-7.34 (m, 2H), 7.50 (dd, *J*=7.8, 4.8 Hz, 1H), 7.82-7.85 (m, 2H), 8.17 (dd, *J*=7.8, 1.3 Hz, 1H), 8.76 (dd, *J*=4.8, 1 .3 Hz, 1H), 13.37 (s, 1H)** |
| **65** | 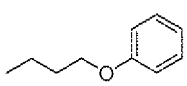 | **171-172** | **1.78-1.79 (m, 4H), 2.67-2.69 (m, 2H), 4.21-4.22 (m, 2H), 7.17-7.33 (m, 6H), 7.50 (dd, *J*=7.7, 4.7 Hz, 1H), 7.81-7.85 (m, 2H), 8.17 (d, *J*=7.7 Hz, 1H), 8.76 (d, *J*=4.7 Hz, 1H), 13.36 (s, 1H)** |
| **66** | 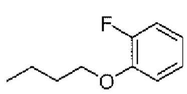 | **164-165** | **2.27-2.29 (m, 2H), 4.27 (t, *J*=6.1 Hz, 2H), 4.38 (t, *J*=5.9 Hz, 2H), 6.94-6.97 (m, 1H), 7.12-7.24 (m, 3H), 7.38 (d, *J*=8.8 Hz, 1H), 7.51 (dd, *J*=7.8, 4.9 Hz, 1H), 7.83-7.87 (m, 2H), 8.49 (d, *J*=7.8 Hz, 1H), 8.76 (d, *J*=4.9 Hz, 1H), 13.36 (s, 1H)** |
| **67** | 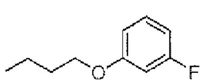 | **186-187** | **2.23-2.28 (m, 2H), 4.21 (t, *J*=6.3 Hz, 2H), 4.37 (t, *J*=6.1 Hz, 2H), 6.75-6.86 (m, 3H), 7.31-7.38 (m, 2H), 7.50 (dd, *J*=7.8, 4.8 Hz, 1H), 7.83-7.87 (m, 2H), 8.17 (dd, *J*=7.8, 1.2 Hz, 1H), 8.76 (dd, *J*=4.8, 1.2 Hz, 1H), 13.35 (s, 1H)** |
| **68** | 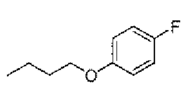 | **156-157** | **2.28-2.25 (m, 2H), 4.16 (t, *J*=6.3 Hz, 2H), 4.37 (t, *J*=6.1 Hz, 2H), 6.98-6.99 (m, 2H), 7.00-7.14 (m, 2H), 7.37 (d, *J*=8.9 Hz, 1H), 7.51 (dd, *J*=7.8, 4.6 Hz, 1H), 7.83-7.87 (m, 2H), 8.17 (dd, *J*=7.8, 1.6 Hz, 1H), 8.76 (dd, *J*=4.6, 1.6 Hz, 1H), 13.36 (s, 1H)** |

(continued)

| Example | R | Physical and Chemical Data of 64-70 | |
|---|---|---|---|
| | | Melting Point(˚C) | $^1$H-NMR (DMSO-$d_6$)( δ ) |
| 69 | 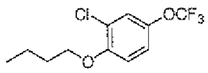 | 159-160 | 2.28-2.32 (m, 2H), 4.32 (t, *J*=6.1 Hz, 2H), 4.40 (t, *J*=6.1 Hz, 2H), 7.33-7.39 (m, 3H), 7.52-7.57 (m, 2H), 7.84-7.88 (m, 2H), 8.20 (dd, *J*=7.8, 1.3 Hz, 1H), 8.77 (dd, *J*=4.5, 1.3 Hz, 1H), 13.25-13.50 (br, 1H). |
| 70 | 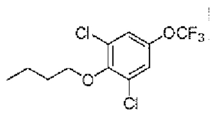 | 171-172 | 2.30-2.33 (m, 2H), 4.25 (t, *J*=5.2 Hz, 2H), 4.44 (t, *J*=4.9 Hz, 2H), 7.39 (d, *J*=8.6 Hz, 1H), 7.51-7.53 (m, 1H), 7.70 (s, 2H), 7.84-7.88 (m, 2H), 8.19 (d, *J*=7.7 Hz, 1H), 8.77 (d, *J*=4.1 Hz, 1H), 13.25-13.50 (br, 1H). |

[Table 13.]

| Example | R | Physical and Chemical Data of 71-77 | |
|---|---|---|---|
| | | Melting Point(˚C) | $^1$H-NMR (DMSO-$d_6$)( δ ) |
| 71 | 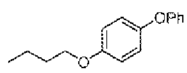 | 149-150 | 2.24-2.27 (m, 2H), 4.18 (t, *J*=5.9 Hz, 2H), 4.38 (t, *J*=5.6 Hz, 2H), 6.91-7.06 (m, 7H), 7.32-7.39 (m, 3H), 7.49-7.52 (m, 1H), 7.83-7.87 (m, 2H), 8.17 (d, *J*=7.4 Hz, 1H), 8.76 (d, *J*=4.2 Hz, 1H), 13.30-13.50 (br, 1H) |
| 72 | 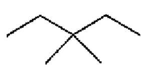 | 166-167 | 0.87 (t, *J*=7.5 Hz, 3H), 1.00 (s, 6H), 1.44 (q, *J*=7.5 Hz, 2H), 3.88 (s, 2H), 7.33 (d, *J*=8.9 Hz, 1H), 7.50 (dd, *J*=7.8, 4.7 Hz, 1H), 7.82 (dd, *J*=8.7, 2.1 Hz, 1H), 7.85 (d, *J*=2.1 Hz, 1H), 8.17 (d, *J*=7.8, 1.4 Hz, 1H), 8.76 (dd, *J*=4.7, 1.4 Hz, 1H), 13.30-13.40 (br, 1H) |
| 73 | 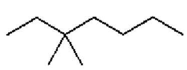 | 155-156 | 0.88 (t, *J*=6.5 Hz, 3H), 1.02 (s, 6H), 1.26-1.28 (m, 4H), 1.36-1.39 (m, 2H), 3.88 (s, 2H), 7.33 (d, *J*=8.6 Hz, 1H), 7.50 (dd, *J*=7.8, 4.7 Hz, 1H), 7.81-7.85 (m, 2H), 8.17 (d, *J*=7.8 Hz, 1H), 8.76 (d, *J*=4.7 Hz, 1H), 13.36 (s, 1H) |
| 74 | 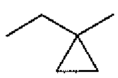 | 208-209 | 0.45 (t, *J*=4.3 Hz, 2H), 0.59 (t, *J*=4.3 Hz, 2H), 1.23 (s, 3H), 4.00 (s, 2H), 7.27 (d, *J*=8.9 Hz, 1H), 7.50 (dd, *J*=7.8, 5.0 Hz, 1H), 7.80 (dd, *J*=8.8, 2.2 Hz, 1H), 7.85 (d, *J*=2.2 Hz, 1H), 8.17 (dd, *J*=7.8, 1.6 Hz, 1H), 8.76 (dd, *J*=5.0, 1.6 Hz, 1H), 13.36 (s, 1H) |
| 75 | 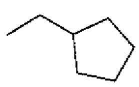 | 227-228 | : 1.38-1.40 (m, 2H), 1.55-1.58 (m, 2H), 1.64-1.66 (m, 2H), 1.79-1.82 (m, 2H), 2.36-2.39 (m, 1H), 4.09 (d, *J*=6.8 Hz, 2H), 7.33 (d, *J*=8.9 Hz, 1H), 7.50 (dd, *J*=7.8, 4.8 Hz, 1H), 7.80-7.85 (m, 2H), 8.17 (dd, *J*=7.8, 1.6 Hz, 1H), 8.76 (dd, *J*=4.8, 1.6 Hz, 1H), 13.36 (s, 1H) |
| 76 | 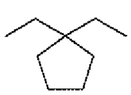 | 196-197 | 0.87 (t, *J*=7.4 Hz, 3H), 1.45-1.66 (m, 10H), 3.95 (s, 2H), 7.36 (d, *J*=8.8 Hz, 1H), 7.50 (dd, *J*=7.8, 4.8 Hz, 1H), 7.81-7.85 (m, 2H), 8.17 (dd, *J*=7.8, 1.1 Hz, 1H), 8.76 (dd, *J*=4.8, 1.1 Hz, 1H), 13.37 (s, 1H) |
| 77 | 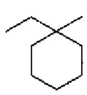 | 100-101 | 1.06 (s, 3H), 1.31-1.50 (m, 10H), 3.92 (s, 2H), 7.34 (d, *J*=8.8 Hz, 1H), 7.50 (dd, *J*=7.8, 4.8 Hz, 1H), 7.81-7.84 (m, 2H), 8.17 (dd, *J*=7.8, 1.2 Hz, 1H), 8.76 (d, *J*=4.8, 1.2 Hz, 1H), 13.36 (s, 1H) |

[Table 14.]

| Example | R | Physical and Chemical Data of 78-84 | |
|---|---|---|---|
| | | Melting Point(˚C) | $^1$H-NMR (DMSO-$d_6$)( δ ) |
| 78 | 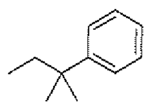 | 221-222 | 1.47 (s, 6H), 4.22 (m, 2H), 7.22-7.23 (m, 1H), 7.33-7.35 (m, 3H), 7.50-7.53 (m, 3H), 7.79-7.83 (m, 2H), 8.17 (d, *J*=7.6 Hz, 1H), 8.76 (d, *J*=3.9 Hz, 1H) |
| 79 | 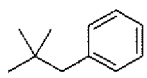 | 188-189 | 1.43 (s, 6H), 3.12 (s, 2H), 7.25-7.35 (m, 5H), 7.46-7.53 (m, 2H), 7.77 (d, *J*=8.8 Hz, 1H), 7.87 (s, 1H), 8.18 (d, *J*=7.8 Hz, 1H), 8.77 (d, *J*=4.6 Hz, 1H), 13.30-13.40 (br, 1H) |
| 80 | 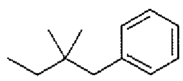 | 196-197 | 1.02 (s, 6H), 2.74 (s, 2H), 3.80 (s, 2H), 7.14-7.30 (m, 6H), 7.51 (dd, *J*=7.7, 5.0 Hz, 1H), 7.82 (d, *J*=8.4 Hz, 1H), 7.90 (d, *J*=1.4 Hz, 1H), 8.18 (d, *J*=7.7 Hz, 1H), 8.77 (d, *J*=5.0 Hz, 1H), 13.37 (s, 1H) |
| 81 | 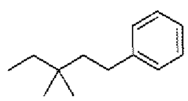 | 172-173 | 1.10 (s, 6H), 1.67-1.71 (m, 2H), 2.60-2.63 (m, 2H), 4.00 (s, 2H), 7.15-7.34 (m, 6H), 7.50 (d, *J*=7.7 Hz, 1H), 7.82-7.86 (m, 2H), 8.16 (d, *J*=7.7 Hz, 1H), 8.76 (d, *J*=4.8 Hz, 1H), 13.20-13.30 (br, 1H) |
| 82 | 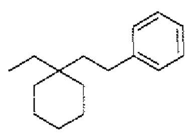 | 143-144 | 1.41-1.57 (m, 10H), 1.74-1.78 (m, 2H), 2.55-2.58 (m, 2H), 4.97 (s, 2H), 7.12-7.26 (m, 5H), 7.40 (d, *J*=8.8 Hz, 1H), 7.50 (dd, *J*=8.1, 4.8 Hz, 1H), 7.83-7.86 (m, 1H), 8.17 (dd, *J*=8.1 Hz, 1H), 8.76 (d, *J*=4.8 Hz, 1H), 13.30-13.40 (br, 1H) |
| 83 | 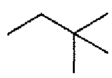 | 231-232 | 1.06 (s, 9H), 3.86 (s, 2H), 7.31 (d, *J*=8.3 Hz, 1H), 7.50 (dd, *J*=7.5, 4.7 Hz, 1H), 7.82 (d, *J*=8.3 Hz, 1H), 7.85 (s, 1H), 8.17 (d, *J*=7.5 Hz, 1H), 8.76 (d, *J*=4.7 Hz, 1H), 13.35 (s, 1H) |
| 84 | 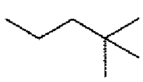 | 206-207 | 1.00 (s, 9H), 1.73 (t, *J*=6.9 Hz, 2H), 4.26 (t, *J*=6.9 Hz, 2H), 7.37 (d, *J*=8.7 Hz, 1H), 7.50 (dd, *J*=7.8, 4.7 Hz, 1H), 7.82-7.85 (m, 1H), 8.17 (dd, *J*=7.8, 1.3 Hz, , 1H), 8.76 (dd, *J*=4.7, 1.3 Hz, 1H), 13.20-13.30 (br, 1H) |

<Examples 85> Benzyl 2-{3-cyano-4-[2-(4-methane-sulfonyloxyphenyl)ethoxy]phenyl}nicotinate

The same operation as in Example 2 was carried out starting from benzyl 2-(3-cyano-4-hydroxyphenyl)nicotinate hydrochloride (Referential Example 6b) (1.0 g, 0.9 mmol), 4-(2-methanesulfonyloxyethyl)phenylmethane sulfonate (0.9 g, 3.0 mmol) and potassium carbonate (0.9 g, 6.8 mmol) to give the title compound (0.7 g, 49%) as crystals.
$^1$H-NMR (DMSO-$d_6$) δ : 3.16 (t, J=6.4 Hz, 2H), 3.36 (s, 3H), 4.36 (t, J=6.4 Hz, 2H), 5.17 (s, 2H), 7.16-7.21 (m, 6H), 7.33 (d, J=8.3 Hz, 2H), 7.51-7.56 (m, 3H), 7.67 (d, J=8.7 Hz, 1H), 7.79 (s, 1H), 8.24 (d, J=8.0 Hz, 1H), 8.79 (d, J=4.8 Hz, 1H).

<Example 86> 2-{3-Cyano-4-[2-(4-methanesulfonyloxy-phenyl)ethoxy]phenyl}nicotinic acid

In an argon atmosphere, 5% Pd/C (0.06 g) was added to a mixed solution of benzyl 2-{3-cyano-4-[2-(4-methane-sulfonyloxyphenyl)ethoxy]phenyl}nicotinate (Example 85) (0.6 g, 1.1 mmol) in methanol (40 mL) and DMF (40 mL) followed by stirring in a hydrogen atmosphere at room temperature for 20 hours. After the catalyst was filtered off, the solvent was evaporated to give the title compound (0.24 g, 48%) as crystals.
Mp. 85-86 ˚C. $^1$H-NMR (Acetone-$d_6$) δ : 3.25-3.29 (m, 5H), 4.49 (t, J=6.6 Hz, 2H), 7.32-7.35 (m, 3H), 7.54-7.58 (m, 3H), 7.87-7.89 (m, 2H), 8.29 (dd, J=8.0, 1.2 Hz, 1H), 8.80 (dd, J=4.4, 1.2 Hz, 1H).

<Example 87> Ethyl 2-[3-cyano-4-[3-(3,5-di-tert-butyl-3-hydroxyphenyl)propoxy]phenylnicotinate

Into a solution of ethyl 2-[3-cyano-4-[3-(3,5-di-tert-butyl-4-methoxymethoxyphenyl)propoxy]phenylnicotinate

(Referential Example 7b) (3.4 g, 6.1 mmol) in dichloromethane (80 mL) was dropped 4 mol/L hydrogen chloride-dioxane (7.8 mL, 31.0 mmol) at room temperature followed by stirring for 14 hours. Water was added to the reaction solution followed by neutralizing with triethylamine. The organic layer was washed with water and dried and the solvent was evaporated to give the title compound (3.1 g, 99%) as crystals.
$^1$H-NMR (DMSO-$d_6$) δ : 1.11 (t, J=7.2 Hz, 3H), 1.37 (s, 18H), 2.01-2.06 (m, 2H), 2.69 (t, J=7.3 Hz, 2H), 4.15-4.20 (m, 4H), 6.70 (s, 1H), 6.94 (s, 2H), 7.30 (d, J=8.9 Hz, 1H), 7.54 (dd, J=7.8, 4.9 Hz, 1H), 7.76 (dd, J=8.9, 2.1 Hz, 1H), 7.86 (d, J=2.1 Hz, 1H), 8.19 (dd, J=7.8, 1.3 Hz, 1H), 8.80 (dd, J=4.9, 1.3 Hz, 1H).

<Example 88> 2-[3-Cyano-4-[3-(3,5-di-tert-butyl-4-hydroxyphenyl)propoxy]phenylnicotinic acid

The same operation as in Example 43 was carried out starting from ethyl 2-[3-cyano-4-[3-(3,5-di-tert-butyl-3-hydroxyphenyl)propoxy]phenylnicotinate (Example 87) (3.1 g, 6.0 mmol), a 1 mol/L aqueous solution of sodium hydroxide (1.26 mL, 12.6 mmol) and ethanol (120 mL) to give the title compound (1.53 g, 52%) as crystals.

<Example 89> Ethyl 2-{3-cyano-4-[4-(3,5-di-tert-butyl-4-hydroxyphenyl)butoxy]phenyl}nicotinate

The same operation as in Example 87 was carried out starting from ethyl 2-{3-cyano-4-[4-(3,5-di-tert-butyl-4-methoxymethoxyphenyl)butoxy]phenyl}nicotinate (Referential Example 7c) (2.1 g,3.7 mmol), a 4 mol/L hydrogen chloride-dioxane (4.6 mL, 18.3 mmol) and dichloromethane (50 mL) to give the title compound (1.6 g, 75%) as crystals.
$^1$H-NMR (DMSO-$d_6$) δ : 1.10 (t, J=7.1 Hz, 3H), 1.37 (s, 18H), 1.71-1.76 (m, 2H), 1.79-1.83 (m, 2H), 2.55 (t, J=7.7 Hz, 2H), 4.18 (q, J=7.1 Hz, 2H), 4.23 (t, J=6.2 Hz, 2H), 6.68 (s, 1H), 7.31 (s, 2H), 7.32 (d, J=8.9 Hz, 1H), 7.53 (dd, J=6.7, 4.7 Hz, 1H), 7.77 (dd, J=8.9, 2.1 Hz, 1H), 7.83 (d, J=2.1 Hz, 1H), 8.19 (dd, J=6.7, 0.9 Hz, 1H), 8.80 (dd, J=4.7, 0.9 Hz, 1H).

<Example 90> 2-{3-Cyano-4-[4-(3,5-di-tert-butyl-4-hydroxyphenyl)butoxy]phenyl}nicotinic acid

The same operation as in Example 43 was carried out starting from ethyl 2-{3-cyano-4-[4-(3,5-di-tert-butyl-4-hydroxyphenyl)butoxy]phenyl}nicotinate (Example 89) (1.6 g, 3.1 mmol), a 1 mol/L aqueous solution of sodium hydroxide (6.5 mL, 6.5 mmol) and ethanol (70 mL) to give the title compound (0.67 g, 43%) as crystals.
Mp. 125-126 ˚C. $^1$H-NMR (DMSO-$d_6$) δ : 1.37 (s, 18H), 1.71-1.82 (m, 4H), 2.54-2.56 (m, 2H), 4.22-4.24 (m, 2H), 6.66 (s, 1H), 6.94 (s, 2H), 7.32 (d, J=8.2 Hz, 1H), 7.50 (dd, J=7.1, 4.7 Hz, 1H), 7.82-7.85 (m, 2H), 8.17 (d, J=7.1 Hz, 1H), 8.76 (dd, J=4.7 Hz, 1H), 13.35 (s, 1H).

<Example 91> Ethyl 2-{3-cyano-4-[2-(4-trifluoromethylphenyl)ethoxy]phenyl}-6-methylnicotinate

The same operation as in Example 1 was carried out starting from ethyl 2-(3-cyano-4-hydroxyphenyl)-6-methylnicotinate hydrochloride (Referential Example 6c) (3.0 g, 9.4 mmol), 4-trifluoromethylphenethyl methanesulfonate (3.0 g, 11.3 mmol), potassium carbonate (3.3 g, 23.5 mmol) and DMF (80 mL) to give the title compound (2.5 g, 58%) as an oily product.
$^1$H-NMR (DMSO-$d_6$) δ : 1.10 (t, J=7.0 Hz, 3H), 2.57 (s, 3H), 3.23 (t, J=6.0 Hz, 2H), 4.15 (q, J=7.0 Hz, 2H), 4.46 (t, J=6.0 Hz, 2H), 7.34-7.39 (m, 2H), 7.62 (d, J=7.8 Hz, 2H), 7.68-7.75 (m, 3H), 7.80 (s, 1H), 8.09 (d, J=8.0 Hz, 1H).

<Example 92> 2-{3-Cyano-4-[2-(4-trifluoromethylphenyl)ethoxy]phenyl}-6-methylnicotinic acid

The same operation as in Example 43 was carried out starting from ethyl 2-{3-cyano-4-[2-(4-trifluoromethylphenyl)ethoxy]phenyl}-6-methylnicotinate (Example 93) (2.5 g, 5.5 mmol), a 1 mol/L aqueous solution of sodium hydroxide (6.3 mL, 6.3 mmol) and ethanol (63 mL) to give the title compound (2.1 g, 90%) as crystals.
Mp. 174-175 ˚C. $^1$H-NMR (DMSO-$d_6$) δ : 2.55 (s, 3H), 3.23 (t, J=6.4 Hz, 2H), 4.45 (t, J=6.4 Hz, 2H), 7.33-7.35 (m, 2H), 7.62 (d, J=8.0 Hz, 2H), 7.69 (d, J=8.0 Hz, 2H), 7.77-7.81 (m, 2H), 8.08 (d, J=7.9 Hz, 1H), 13.25-13.40 (br, 1H).

<Example 93> Ethyl 2-{3-cyano-4-[2-(4-trifluoromethylphenyl)ethoxy]phenyl}-6-methylnicotinate

The same operation as in Example 2 was carried out starting from ethyl 2-(3-cyano-4-hydroxyphenyl)-4-methylnicotinate hydrochloride (Referential Example 6d) (1.0 g, 3.1 mmol), 4-trifluoromethylphenethyl methanesulfonate (1.0 g, 3.8 mmol), potassium carbonate (0.95 g, 6.9 mmol) and DMF (30 mL) to give the title compound (0.9 g, 63%) as an oily product.
$^1$H-NMR (DMSO-$d_6$) δ : 1.09 (t, J=7.3 Hz, 3H), 2.36 (s, 3H), 3.22 (t, J=6.4 Hz, 2H), 4.20 (q, J=7.3 Hz, 2H), 4.46 (t, J=6.4 Hz, 2H), 7.38-7.41 (m, 2H), 7.61 (d, J=8.1 Hz, 2H), 7.69 (d, J=8.1 Hz, 2H), 7.77-7.80 (m, 2H), 8.60 (d, J=5.1 Hz, 1H).

<Example 94> 2-{3-Cyano-4-[2-(4-trifluoromethylphenyl)ethoxy]phenyl}-4-methylnicotinic acid

The same operation as in Example 43 was carried out starting from ethyl 2-{3-cyano-4-[2-(4-trifluoromethylphenyl)ethoxy]phenyl}-4-methylnicotinate (Example 95) (0.9 g, 2.0 mmol), a 1 mol/L aqueous solution of sodium hydroxide (2.4 mL, 2.4 mmol) and ethanol (24 mL) to give the title compound (34 mg, 4%) as crystals.
Mp. 178-179 ˚C. $^1$H-NMR (DMSO-$d_6$) δ : 2.35 (s, 3H), 3.22 (t, J=6.6 Hz, 2H), 4.45 (t, J=6.6 Hz, 2H), 7.33 (d, J=4.9 Hz, 1H), 7.39 (d, J=8.5 Hz, 1H), 7.61 (d, J=7.8 Hz, 2H), 7.69 (d, J=7.8 Hz, 2H), 7.89-7.91 (m, 2H), 8.53 (d, J=4.9 Hz, 1H), 13.75-13.95 (br, 1H).

<Example 95> Ethyl 2-{3-cyano-4-[2-(5-methyl-2-phenyloxazol-4-yl)ethoxy]phenyl}nicotinate

The same operation as in Example 2 was carried out starting from ethyl 2-(3-cyano-4-hydroxyphenyl)-nicotinate (Referential Example 6a) (4.5 g, 15 mmol), 2-(5-methyl-2-phenyloxazol-4-yl)ethyl methanesulfonate (5.0 g, 18 mmol), potassium carbonate (4.7 g, 34 mmol) and DMF (210 mL) to give the title compound (6.0 g, 89%) as an oily product.
$^1$H-NMR (DMSO-$d_6$) δ : 1.11 (t, J=7.0 Hz, 3H), 2.41 (s, 3H), 3.03 (t, J=6.2 Hz, 2H), 4.17 (q, J=7.0 Hz, 2H), 4.45 (t, J=6.2 Hz, 2H), 7.38 (d, J=8.8 Hz, 1H), 7.49-7.54 (m, 4H), 7.77 (d, J=8.8 Hz, 1H), 7.83 (d, J=2.3 Hz, 1H), 7.92-7.94 (m, 2H), 8.19 (d, J=7.6, 1.4 Hz, 1H), 8.79 (d, J=5.0, 1.4 Hz, 1H).

<Example 96> 2-{3-Cyano-4-[2-(5-methyl-2-phenyloxazol-4-yl)ethoxy]phenyl}nicotinic acid

The same operation as in Example 43 was carried out starting from ethyl 2-{3-cyano-4-[2-(5-methyl-2-phenyloxazol-4-yl)ethoxy]phenyl}nicotinate (7.0 g, 15 mmol), a 1 mol/L aqueous solution of sodium hydroxide (19 mL, 19 mmol) and ethanol (200 mL) to give the title compound (3.9 g, 59%) as crystals.
Mp. 246-247 ˚C. $^1$H-NMR (DMSO-$d_6$) δ : 2.41 (s, 3H), 3.02 (t, J=6.0 Hz, 2H), 4.44 (t, J=6.0 Hz, 2H), 7.38 (d, J=8.8 Hz, 1H), 7.48-7.50 (m, 4H), 7.81-7.84 (m, 2H), 7.93 (d, J=8.0 Hz, 2H), 8.17 (d, J=8.0 Hz, 1H), 8.75 (d, J=4.7 Hz, 1H), 13.40 (s, 1H).

<Example 97> Ethyl 2-{3-cyano-4-[2-(methylpyridin-2-ylamino)ethoxy]phenyl}nicotinate

The same operation as in Referential Example 9a was carried out starting from ethyl 2-(3-cyano-4-fluorophenyl)-nicotinate (1.0 g, 3.7 mmol), 2-(N-methyl-N-pyridyl-2-yl- amino)ethanol (0.63 g, 3.7 mmol), sodium hydride (0.18 g, 4.4 mmol) and anhydrous DMF (10 mL) to give the title compound (0.33 g, 22%) as an oily product.
$^1$H-NMR (DMSO-$d_6$) δ : 1.10 (t, J=7.1 Hz, 3H), 3.14 (s, 3H), 3.99 (t, J=5.5 Hz, 2H), 4.17 (q, J=7.1 Hz, 2H), 4.38 (t, J=5.5 Hz, 2H), 6.59 (d, J=7.7 Hz, 1H), 6.68 (d, J=8.6 Hz, 1H), 7.38 (d, J=8.6 Hz, 1H), 7.52-7.54 (m, 2H), 7.75-7.77 (m, 1H), 7.84 (d, J=2.2 Hz, 1H), 8.09-8.10 (m, 1H), 8.19 (dd, J=7.7, 1.1 Hz, 1H), 8.80 (dd, J=4.6, 1.1 Hz, 1H)

<Example 98> 2-{3-Cyano-4-[2-(methylpyridin-2-yl-amino)ethoxy]phenyl}nicotinic acid

The same operation as in Example 43 was carried out starting from ethyl 2-{3-cyano-4-[2-(methylpyridin-2-yl-amino)ethoxy]phenyl}nicotmate (0.33 g, 0.82 mmol), a 1 mol/L aqueous solution of sodium hydroxide (0.98 mL, 0.98 mmol) and ethanol (10 mL) to give the title compound (0.07 g, 23%) as crystals.
$^1$H-NMR (DMSO-$d_6$) δ : 8.14 (s, 3H), 4.00 (t, J=5.3 Hz, 2H), 4.37 (t, J=5.3 Hz, 2H), 6.57-6.60 (m, 1H), 6.69 (d, J=8.6 Hz, 1H), 7.73 (d, J=8.9 Hz, 1H), 7.49-7.52 (m, 2H), 7.81 (dd, J=8.9, 2.0 Hz, 1H), 7.86 (d, J=2.0 Hz, 1H), 8.10 (d, J=3.7 Hz, 1H), 5.16 (d, J=7.0 Hz, 1H), 8.75 (d, J=3.4 Hz, 1H), 13.20-13.40 (br, 1H).

<Example 99> Ethyl 2-{4-[2-(2-chlorophenyl)ethoxy]-3-cyanophenyl}nicotinate

Into a solution of ethyl 2-(3-cyano-4-hydroxy-phenyl)nicotinate hydrochloride (Referential Example 6a) (1.0 g, 3.3 mmol), tert-butoxy potassium (0.37 g, 3.3 mmol) and triphenyl phosphine (0.92 g, 3.5 mmol) in THF was dropped a 2.2 mol/L DEAD-toluene solution (1.6 mL, 3.5 mmol) at room temperature and then 2-chlorophenethyl alcohol (0.55 g, 3.5 mmol) was added thereto. After stirring the mixture at room temperature for 12 hours, the solvent was evaporated therefrom *in vacuo* and the resulting residue was purified by silica gel column chromatography (dichloromethane-n-hexane-ethyl acetate = 2/2/1) to give the title compound (0.80 g, 51%) as an oily product.
$^1$H-NMR ($CDCl_3$) δ : 1.20 (t, J=7.1 Hz, 3H), 3.34 (t, J=6.8 Hz, 2H), 4.24 (q, J=7.1 Hz, 2H), 4.35 (t, J=6.8 Hz, 2H), 7.02 (d, J=8.8 Hz, 1H), 7.26-7.28 (m, 2H), 7.36-7.38 (m, 2H), 7.43-7.46 (m, 1H), 7.69-7.71 (m, 1H), 7.76 (d, J=2.3 Hz, 1H), 8.14 (dd, J=7.8, 1.7 Hz, 1H), 8.75 (dd, J=4.8, 1.7 Hz, 1H).

<Example 100> 2-{4-[2-(2-Chlorophenyl)ethoxy]-3-cyanophenyl}nicotinic acid

The same operation as in Example 43 was carried out starting from ethyl 2-{4-[2-(2-chlorophenyl)ethoxy]-3-cyano-phenyl}nicotinate (0.80 g, 2.0 mmol), sodium hydroxide (0.18 g, 4.5 mmol), water (5 mL) and ethanol (10 mL) to give the title compound (0.70 g, 90%) as crystals.
Mp. 187-188 ˚C. $^1$H-NMR (DMSO-$d_6$) δ : 3.25 (t, J=6.7 Hz, 2H), 4.42 (t, J=6.7 Hz, 2H), 7.29-7.32 (m, 2H), 7.37 (d, J=8.9 Hz, 1H), 7.45-7.53 (m, 3H), 7.80-7.84 (m, 2H), 7.80-7.84 (m, 1H), 8.74-8.75 (m, 1H), 13.20-13.40 (br, 1H).

<Example 101> Ethyl 2-[3-cyano-4-(2-methylphenethyl-oxy)phenyl] nicotinate

The same operation as in Example 1 was carried out starting from ethyl 2-(3-cyano-4-hydroxyphenyl)nicotinate hydrochloride (Referential Example 6a) (2.0 g, 6.6 mmol), 2-methylphenethyl methanesulfonate (1.7 g, 7.9 mmol), potassium carbonate (2.1 g, 15 mmol) and DMF (70 mL) to give the title compound (2.5 g, 99%) as an oily product.
$^1$H-NMR (DMSO-$d_6$) δ : 1.11 (t, J=7.1 Hz, 3H), 2.37 (s, 3H), 3.12 (t, J=6.8 Hz, 2H), 4.18 (q, J=7.1 Hz, 2H), 4.40 (t, J=6.8 Hz, 2H), 7.14-7.16 (m, 3H), 7.30-7.32 (m, 1H), 7.36 (d, J=8.9 Hz, 1H), 7.53 (dd, J=7.8, 4.9 Hz, 1H), 7.76 (dd, J=8.9, 2.3 Hz, 1H), 7.83 (d, J=2.3 Hz, 1H), 8.19 (dd, J=7.8, 1.6 Hz, 1H), 8.79 (dd, J=4.9, 1.6 Hz, 1H).

<Example 102> 2-[3-Cyano-4-(2-methylphenethyloxy)-phenyl]nicotinic acid

The same operation as in Example 43 was carried out starting from ethyl 2-[3-cyano-4-(2-methylphenethyl-oxy)-phenyl]nicotinate (2.5 g, 6.5 mmol), a 1 mol/L aqueous solution of sodium hydroxide (7.8 mL, 7.8 mmol) and ethanol (80 mL) to give the title compound (1.8 g, 76%) as crystals.
Mp. 179-180 ˚C. $^1$H-NMR (DMSO-$d_6$) δ : 2.38 (s, 3H), 3.12 (t, J=6.6 Hz, 2H), 4.39 (t, J=6.6 Hz, 2H) 7.15-7.18 (m, 3H), 7.31-7.37 (m, 2H), 7.49-7.51 (m, 1H), 7.81 (d, J=8.8 Hz, 1H), 8.84 (s, 1H), 8.16 (d, J=7.8 Hz, 1H), 8.75 (d, J=3.3 Hz, 1H), 13.30-13.50 (br, 1H).

<Example 103> Ethyl 2-{3-cyano-4-[2,2-dimethyl-3-(4-methoxyphenyl)propoxy]phenyl}nicotinate

The same operation as in Example 30 was carried out starting from ethyl 2-chloronicotinate hydrochloride (Example 4a) (2.5 g, 1.4 mmol), 3-cyano-4-[2,2-dimethyl-3-(4-methoxyphenyl)propoxy]phenylboronate (5.5 g, 16 mmol), palladium acetate (0.18 g, 0.81 mmol), tris(2-methylphenyl) phosphine (0.49 g, 1.6 mmol), a 10% aqueous solution of sodium carbonate (25.4 mL, 24 mmol) and ethylene glycol dimethyl ether (125 mL) to give the title compound (3.2 g, 54%) as an oily product.
$^1$H-NMR (DMSO-$d_6$) δ : 1.01 (s, 6H), 1.13 (t, J=7.1 Hz, 3H), 2.67 (s, 2H), 3.71 (s, 3H), 3.78 (s, 2H), 4.20 (q, J=7.1 Hz, 2H), 6.83 (d, J=8.4 Hz, 2H), 7.05 (d, J=8.4 Hz, 2H), 7.29 (d, J=8.8 Hz, 1H), 7.55 (dd, J=7.8, 4.8 Hz, 1H), 7.76 (dd, J=8.8, 2.0 Hz, 1H), 7.89 (d, J=2.0 Hz, 1H), 8.21 (d, J=7.8 Hz, 1H), 8.81 (d, J=4.8 Hz, 1H).

<Example 104> 2-{3-Cyano-4-[2,2-dimethyl-3-(4-methoxyphenyl)propoxy]phenyl}nicotinic acid

The same operation as in Example 43 was carried out starting from ethyl 2-{3-cyano-4-[2,2-dimethyl-3-(4-methoxyphenyl)propoxy]phenyl}nicotinate (3.2 g, 7.1 mmol), a 1 mol/L aqueous solution of sodium hydroxide (8.7 mL, 8.7 mmol) and ethanol (90 mL) to give the title compound (2.2 g, 75%) as crystals.
Mp. 197-198 ˚C. $^1$H-NMR (DMSO-$d_6$) δ: 1.00 (s, 6H), 2.66 (s, 2H), 3.71 (s, 2H), 3.78 (s, 3H), 6.84 (d, J=8.5 Hz, 2H), 7.05 (d, J=8.5 Hz, 2H), 7.24 (d, J=8.8 Hz, 1H), 7.52 (dd, J=7.8, 4.7 Hz, 1H), 7.82 (d, J=8.8, 2.3 Hz, 1H), 7.90 (d, J=2.3 Hz, 1H), 8.19 (dd, J=7.8, 1.6 Hz, 1H), 8.77 (dd, J=4.7, 1.6 Hz, 1H), 13.25-13.40 (br, 1H).

<Example 105> Ethyl 2-{3-cyano-4-[2,2-dimethyl-3-(4-trifluoromethylphenyl)propoxy]phenyl}nicotinate

The same operation as in Example 30 was carried out starting from ethyl 2-chloronicotinate (Referential Example 4a) (0.9 g, 4.8 mmol), 3-cyano-4-[2,2-dimethyl-3-(4-trifluoromethylphenyl)propoxy]phenylboronic acid (2.0 g, 5.3 mmol), palladium acetate (65 mg, 0.29 mmol), tris(2-methylphenyl) phosphine (0.18 g, 0.58 mmol), a 10% aqueous solution of sodium carbonate (9.1 mL, 8.7 mmol) and ethylene glycol dimethyl ether (45 mL) to give the title compound (1.6 g, 69%) as an oily product.
$^1$H-NMR (DMSO-$d_6$) δ: 1.04 (s, 6H), 1.13 (t, J=7.1 Hz, 3H), 2.84 (s, 2H), 3.82 (s, 2H), 4.20 (q, J=7.1 Hz, 2H), 7.32 (d, J=8.8 Hz, 1H), 7.38 (d, J=7.9 Hz, 2H), 7.54-7.56 (m, 1H), 7.65 (d, J=7.9 Hz, 2H), 7.76-7.78 (m, 1H), 7.89 (d, J=2.2 Hz, 1H), 8.21 (dd, J=8.4, 1.6 Hz, 1H), 8.81 (dd, J=4.8, 1.6 Hz, 1H).

<Example 106> 2-{3-Cyano-4-[2,2-dimethyl-3-(4-trifluoromethylphenyl)propoxy]phenyl}nicotinic acid

The same operation as in Example 43 was carried out starting from ethyl 2-{3-cyano-4-[2,2-dimethyl-3-(4-trifluoromethylphenyl)propoxy]phenyl}nicotinate (1.6 g, 3.4 mmol), a 1 mol/L aqueous solution of sodium hydroxide (4.0 mL, 4.0 mmol) and ethanol (40 mL) to give the title compound (0.9 g, 58%) as crystals.
Mp. 137-138 ˚C. $^1$H-NMR (DMSO-$d_6$) δ: 1.03 (s, 6H), 2.84 (s, 2H), 3.83 (s, 2H), 7.32 (d, J=8.8 Hz, 1H), 7.38 (d, J=7.9 Hz, 2H), 7.51 (dd, J=7.8, 4.9 Hz, 1H), 7.66 (d, J=7.9 Hz, 2H), 7.82 (dd, J=8.8, 2.1 Hz, 1H), 7.90 (d, J=2.1 Hz, 1H), 8.19 (d, J=7.8 Hz, 1H), 8.77 (d, J=4.9 Hz, 1H), 13.30-13.40 (br, 1H).

<Example 107> Ethyl 2-{4-[2-(2-nitrophenyl)ethoxy]-3-cyanophenyl}nicotinate

The same operation as in Example 99 was carried out starting from ethyl 2-(3-cyano-4-hydroxyphenyl)nicotinate hydrochloride (Example 6a) (1.0 g, 3.3 mmol), tert-butoxy potassium (0.37 g, 3.3 mmol), triphenyl phosphine (0.92 g, 35 mmol), a 2.2 mol/L DEAD-toluene solution (1.6 mL, 3.5 mmol), 2-nitrophenethyl alcohol (0.59 g, 3.5 mmol) and THF (10 mL) to give the title compound (0.72 g, 51%) as an oily product.
$^1$H -NMR (CDCl3) δ : 1.20 (t, J=7.1 Hz, 3H), 3.46 (t, J=5.9 Hz, 2H), 4.24 (q, J=7.1 Hz, 2H), 4.48 (t, J=5.9 Hz, 2H), 7.03 (d, J=8.8 Hz, 1H), 7.36 (dd, J=7.8, 4.8 Hz, 1H), 7.43-7.45 (m, 1H), 7.63-7.65 (m, 1H), 7.68-7.70 (m, 2H), 7.76 (d, J=2.2 Hz, 1H), 8.0 (dd, J=8.2, 1.0 Hz, 1H), 8.15 (dd, J=7.8, 1.7 Hz, 1H), 8.45 (dd, J=4.8, 1.7 Hz, 1H).

<Example 108> 2-{4-[2-(2-Nitrophenyl)ethoxy]-3-cyanophenyl}nicotinic acid

The same operation as in Example 43 was carried out starting from ethyl 2-{4-[2-(2-nitrophenyl)ethoxy]-3-cyano-phenyl}nicotinate (0.40 g, 0.95 mol), sodium hydroxide (0.10 g, 2.5 mmol), water (5 mL) and ethanol (10 mL) to give the title compound (0.19 g, 51%) as crystals.
Mp. 216-217 ˚C. $^1$H-NMR (DMSO-$d_6$) δ : 3.39 (t, J=6.5 Hz, 2H), 4.50 (t, J=6.5 Hz, 2H), 7.37 (d, J=8.8 Hz, 1H), 7.46-7.48 (m, 1H), 7.53-7.54 (m, 1H), 7.70-7.71 (m, 2H), 7.81-7.86 (m, 2H), 8.02 (d, J=8.2 Hz, 1H), 8.15-8.16 (m, 1H), 8.73 (d, J=4.4 Hz, 1H), 13.50-13.60 (br, 1H),

<Example 109> Ethyl 2-[3-cyano-4-(2-methoxyphenethyl-oxy)phenyl]nicotinate

The same operation as in Example 1 was carried out starting from ethyl 2-(3-cyano-4-hydroxyphenyl)nicotinate hydrochloride (Referential Example 6a) (2.0 g, 6.6 mmol), 2-methoxyphenethyl methanesulfonate (1.8 g, 7.9 mmol), potassium carbonate (2.1 g, 15 mmol) and DMF (30 mL) to give the title compound (1.3 g, 50%) as an oily product.
$^1$H-NMR (DMSO-$d_6$) δ : 1.12 (t, J=6.9 Hz, 3H), 3.09 (t, J=6.6 Hz, 2H), 3.82 (s, 3H), 4.19 (q, J=6.9 Hz, 2H), 4.36 (t, J=6.6 Hz, 2H), 6.90-6.92 (m, 1H), 7.00 (d, J=8.2 Hz, 1H), 7.23-7.25 (m, 1H), 7.30 (d, J=7.4 Hz, 1H), 7.37 (d, J=8.9 Hz, 1H), 7.53 (dd, J=7.8, 5.0 Hz, 1H), 7.77 (dd, J=8.9, 2.0 Hz, 1H), 7.83 (d, J=2.0 Hz, 1H), 8.18 (dd, J=7.8, 1.4 Hz, 1H), 8.80 (dd, J=5.0, 1.4 Hz, 1H).

<Example 110> 2-[3-Cyano-4-(2-methoxyphenethyloxy)-phenyl]nicotinic acid

The same operation as in Example 43 was carried out starting from ethyl 2-[3-cyano-4-(2-methoxyphenethyloxy)-phenyl]nicotinate (0.52 g, 1.4 mmol), a 1 mol/L aqueous solution of sodium hydroxide (1.6 mL, 1.6 mmol) and ethanol (20 mL) to give the title compound (0.36 g, 74%) as crystals.
Mp. 189-190 ˚C. $^1$H-NMR (DMSO-$d_6$) δ : 3.10 (t, J=6.9 Hz, 2H), 3.82 (s, 3H), 4.35 (t, J=6.9 Hz, 2H), 6.89-6.91 (m, 1H), 7.00 (d, J=8.2 Hz, 1H), 7.23-7.25 (m, 1H), 7.30 (d, J=7.3 Hz, 1H), 7.37 (d, J=8.7 Hz, 1H), 7.50 (dd, J=7.7, 4.7 Hz, 1H), 7.81 (m, 2H), 8.17 (dd, J=7.7, 1.3 Hz, 1H), 8.76 (dd, J=4.7, 1.3 Hz, 1H), 13.30-13.50 (br, 1H).

<Example 111> Ethyl 2-{3-cyano-4-[2,2-dimethyl-3-(2-methoxyphenyl)propoxy]phenyl}mcotinate

The same operation as in Example 30 was carried out starting from ethyl 2-chloronicotinate (Example 4a) (1.2 g, 6.7 mmol), 2-{3-cyano-4-[2,2-dimethyl-3-(2-methoxyphenyl)-propoxy]phenylboronic acid (2.5 g, 7.4 mmol), palladium acetate (90 mg, 74 mmol), tris(2-methylphenyl) phosphine (0.24 g, 0.80 mmol), a 10% aqueous solution of sodium carbonate (12.7 mL, 12 mmol) and ethylene glycol dimethyl ether (63 mL) to give the title compound (0.98 g, 56%) as an oily product.
$^1$H-NMR (DMSO-$d_6$) δ : 1.01 (s, 6H), 1.13 (t, J=7.2 Hz, 3H), 2.75 (s, 2H), 3.65 (s, 3H), 3.81 (s, 2H), 4.19 (q, J=7.2 Hz, 2H), 6.83-6.85 (m, 1H), 6.95 (d, J=8.2 Hz, 1H), 7.04-7.06 (m, 1H), 7.18-7.20 (m, 1H), 7.27 (d, J=8.8 Hz, 1H), 7.54 (dd, J-7.8, 4.7 Hz, 1H), 7.77 (dd, J=8.8, 2.2 Hz, 1H), 7.87 (d, J=2.2 Hz, 1H), 8.20 (dd, J=7.8, 1.6 Hz, 1H), 8.81 (d, J=4.7, 1.6 Hz, 1H).

<Example 112> 2-{3-Cyano-4-[2,2-dimethyl-3-(2-methoxyphenyl)propoxy]phenyl}nicotinic acid

The same operation as in Example 43 was carried out starting from ethyl 2-{3-cyano-4 [2,2-dimethyl-3-(2-methoxyphenyl)propoxy]phenyl}nicotinate (1.6 g, 3.6 mmol), a 1 mol/L aqueous solution of sodium hydroxide (4.3 mL, 4.3 mmol) and ethanol (43 mL) to give the title compound (1.1 g, 70%) as crystals.
Mp. 164-165 °C. $^1$H-NMR (DMSO-$d_6$) δ : 1.02 (s, 6H), 2.75 (s, 2H), 3.61 (s, 3H), 3.80 (s, 2H), 6.84-6.86 (m, 1H), 6.94 (d, J=8.2 Hz, 1H), 7.06-7.08 (m, 1H), 7.17-7.19 (m, 1H), 7.26 (d, J=8.9 Hz, 1H), 7.49-7.52 (m, 1H), 7.82 (d, J=8.9 Hz, 1H), 7.88 (d, J=2.2 Hz, 1H), 8.18 (dd, J=7.9, 1.5 Hz, 1H), 8.77 (dd, J=4.6, 1.5 Hz, 1H), 13.30 (s, 1H).

<Example 113> Ethyl 2-{3-cyano-4-[2-methyl-2-(4-trifluoromethylphenyl)propoxy]phenyl}nicotinate

The same operation as in Example 30 was carried out starting from ethyl 2-chloronicotinate (Referential Example 4a) (1.0 g, 5.4 mmol), 3-cyano-4-[2-methyl-2-(4-trifluoromethylphenyl)propoxy]phenylboronic acid (2.1 g, 5.9 mmol), palladium acetate (73 mg, 0.32 mmol), tris(2-methylphenyl) phosphine (0.19 g, 0.64 mmol), a 10% aqueous solution of sodium carbonate (10.2 mL, 9.7 mmol) and ethylene glycol dimethyl ether (50 mL) to give the title compound (1.1 g, 45%) as an oily product.
$^1$H-NMR (DMSO-$d_6$) δ : 1.11 (t, J=7.0 Hz, 3H), 1.49 (s, 6H), 4.18 (q, J=7.0 Hz, 2H), 4.29 (s, 2H), 7.36 (d, J=8.8 Hz, 1H), 7.53-7.55 (m, 1H), 7.68-7.71 (m, 2H), 7.75-7.77 (m, 3H), 7.81 (d, J=2.2 Hz, 1H), 8.19 (dd, J=7.8, 1.5 Hz, 1H), 8.79 (dd, J=4.9, 1.5 Hz, 1H).

<Example 114> 2-{3-Cyano-4-[2-methyl-2-(4-trifluoromethylphenyl)propoxy]phenyl}nicotinic acid

The same operation as in Example 43 was carried out starting from ethyl 2-{3-cyano-4-[2-methyl-2-(4-trifluoromethylphenyl)propoxy]phenyl}nicotinate (1.1 g, 2.4 mmol), a 1 mol/L aqueous solution of sodium hydroxide (2.8 mL, 2.8 mmol) and ethanol (30 mL) to give the title compound (0.8 g, 77%) as crystals.
Mp. 186-187 °C. $^1$H-NMR (DMSO-$d_6$) δ : 1.49 (s, 6H), 4.28 (s, 2H), 7.35 (d, J=8.6 Hz, 1H), 7.49-7.51 (m, 1H), 7.70 (d, J=7.9 Hz, 2H), 7.75 (d, J=7.9 Hz, 2H), 7.80-7.82 (m, 2H), 8.16 (d, J=7.8 Hz, 1H), 8.75 (d, J=4.7 Hz, 1H), 13.30-13.40 (br, 1H).

<Example 115> Ethyl 2-[3-cyano-4-(3-phenylpropoxy)-phenyl]nicotinate

The same operation as in Example 2 was carried out starting from ethyl 2-(3-cyano-4-hydroxyphenyl)nicotinate hydrochloride (Example 6a) (2.5 g, 8.2 mmol), 3-phenylpropyl methanesulfonate (2.1 g, 9.8 mmol), potassium carbonate (2.6 g, 19 mmol) and DMF (100 mL) to give the title product (3.0 g, 95%) as an oily product.
$^1$H-NMR(DMSO-$d_6$) δ : 1.11 (t, J=7.2 Hz, 3H), 2.08-2.12 (m, 2H), 2.81 (t, J=7.3 Hz, 2H), 4.16-4.21 (m, 4H), 7.20-7.32 (m, 6H), 7.54 (dd, J=7.8, 4.7 Hz, 1H), 7.76 (dd, J=8.9, 2.0 Hz, 1H), 7.86 (d, J=2.0 Hz, 1H), 8.19 (dd, J=7.8, 1.4 Hz, 1H), 8.80 (dd, J=4.7, 1.4 Hz, 1H).

<Example 116> 2-[3-Cyano-4-(3-phenylpropoxy)phenyl]-nicotinic acid

The same operation as in Example 43 was carried out starting from ethyl 2-[3-cyano-4-(3-phenylpropoxy) phenyl]-nicotinate (3.0 g, 7.8 mmol), a 1 mol/L aqueous solution of sodium hydroxide (9.3 mL, 9.3 mmol) and ethanol (90 mL) to give the title compound (2.1 g, 76%) as crystals.
Mp.183-184 °C. $^1$H-NMR (DMSO-$d_6$) δ : 2.07-2.13 (m, 2H), 2.80 (t, J=7.5 Hz, 2H), 4.19 (t, J=6.2 Hz, 2H), 7.20-7.30 (m, 6H), 7.51 (dd, J=7.8, 4.8 Hz, 1H), 7.81 (dd, J=8.7, 1.7 Hz, 1H), 7.87 (d, J=1.7 Hz, 1H), 8.17 (d, J=7.8 Hz, 1H), 8.76 (d, J=4.8 Hz, 1H), 13.25-13.40 (br, 1H).

<Example 117> Ethyl 2-{3-cyano-4-[3-(2-trifluoromethylphenyl)propoxy]phenyl}nicotinate

The same operation as in Example 2 was carried out starting from ethyl 2-(3-cyano-4-hydroxyphenyl)nicotinate hydrochloride (Example 6a) (1.0 g, 3.3 mmol), 3-(2-trifluoro-methylphenyl)propyl methanesulfonate (1.1. g, 3.9 mmol), potassium carbonate (1.0 g, 7.3 mmol) and DMF (70 mL) to give the title product (1.4 g, 95%) as an oily product.
$^1$H-NMR (DMSO-$d_6$) δ : 1.11 (t, J=7.1 Hz, 3H), 2.11-2.15 (m, 2H), 3.00 (t, J=7.4 Hz, 2H), 4.19 (q, J=7.1 Hz, 2H), 4.29 (t, J=5.9 Hz, 2H), 7.34 (d, J=8.8 Hz, 1H), 7.42-7.44 (m, 1H), 7.55-7.57 (m, 2H), 7.62-7.64 (m, 1H), 7.69 (d, J=7.8 Hz, 1H), 7.78 (dd, J=8.8, 2.0 Hz, 1H), 7.86 (d, J=2.0 Hz, 1H), 8.20 (d, J=7.8 Hz, 1H), 8.81 (d, J=4.5 Hz, 1H).

<Example 118> 2-{3-Cyano-4-[3-(2-trifluoromethylphenyl)propoxy]phenyl}nicotinic acid

The same operation as in Example 43 was carried out starting from ethyl 2-{3-cyano-4-[3-(2-trifluoromethylphenyl)propoxy]phenyl}nicotinate (2.5 g, 5.6 mmol), a 1 mol/L aqueous solution of sodium hydroxide (6.7 mL, 6.7 mmol) and ethanol (70 mL) to give the title compound (1.8 g, 76%) as crystals.
Mp.163-164 ˚C. $^1$H-NTMR (DMSO-$d_6$) δ : 2.09-2.14 (m, 2H), 2.99 (t, J=7.5 Hz, 2H), 4.28 (t, J=5.9 Hz, 2H), 7.34 (d, J=8.9 Hz, 1H), 7.42-7.44 (m, 1H), 7.50-7.52 (m, 1H), 7.55-7.57 (m, 1H), 7.62-7.64 (m, 1H), 7.69 (d, J=7.9 Hz, 1H), 7.81-7.84 (m, 1H), 7.87 (d, J=2.2 Hz, 1H), 8.16-8.18 (m, 1H), 8.77 (dd, J=4.7, 1.2 Hz, 1H), 13.30-13.40 (br, 1H).

<Example 119> Ethyl 2-{3-cyano-4-[3-(4-methoxyphenyl)-propoxy]phenyl}nicotinate

The same operation as in Example 2 was carried out starting from ethyl 2-(3-cyano-4-hydroxyphenyl)nicotinate hydrochloride (Referential Example 6a) (2.0 g, 6.6 mmol), 3-(4-methoxyphenyl)propoxy methanesulfonate (1.6 g, 7.9 mmol), potassium carbonate (2.1 g, 15.2 mmol) and DMF (100 mL) to give the title product (2.0 g, 76%) as an oily product.
$^1$H-NMR (DMSO-$d_6$) δ : 1.10 (t, J=7.1 Hz, 3H), 2.03-2.07 (m, 2H), 2.74 (t, J=7.4 Hz, 2H), 3.72 (s, 3H), 4.15-4.20 (m, 4H), 6.86 (d, J=8.2 Hz, 2H), 7.15 (d, J=8.2 Hz, 2H), 7.30 (d, J=8.9 Hz, 1H), 7.52-7.55 (m, 1H), 7.75-7.77 (m, 1H), 7.86 (d, J=1.1 Hz, 1H), 8.16 (d, J=7.8 Hz, 1H), 8.80 (d, J=4.7 Hz, 1H).

<Example 120> 2-{3-Cyano-4-[3-(4-methoxyphenyl)-propoxy]phenyl}nicotinic acid

The same operation as in Example 43 was carried out starting from ethyl 2-{3-cyano-4-[3-(4-methoxyphenyl)-propoxy]phenyl}nicotinate (2.1 g, 5.0 mmol), a 1 mol/L aqueous solution of sodium hydroxide (6.0 mL, 6.0 mmol) and ethanol (60 mL) to give the title compound (1.3 g, 68%) as crystals.
Mp. 179-180 ˚C. $^1$H-NMR (DMSO-$d_6$) δ : 2.05-2.09 (m, 2H), 2.74 (t, J=7.3 Hz, 2H), 4.17 (t, J=6.0 Hz, 2H), 6.86 (d, J=8.3 Hz, 2H), 7.16 (d, J=8.3 Hz, 2H), 7.30 (d, J=8.8 Hz, 1H), 7.51 (dd, J=7.5, 4.9 Hz, 1H), 7.81 (d, J=8.8 Hz, 1H), 7.87 (s, 1H), 8.17 (d, J=7.5 Hz, 1H), 8.76 (d, J=4.9 Hz, 1H), 13.30-13.40 (br, 1H).

<Example 121> Ethyl 2-{3-cyano-4-[2-methyl-2-(4-methoxyphenyl)propoxy]phenyl}nicotinate

The same operation as in Referential Example 5a was carried out starting from ethyl 2-chloronicotinate (Referential Example 4a) (1.2 g, 6.7 mmol), 3-cyano- 4-[2-methyl-2-(4-methoxyphenyl)propoxy]phenylboronic acid (2.4 g, 7.4 mmol), palladium acetate (90 mg, 0.40 mmol), tris(2-methylphenyl) phosphine (0.24 g, 0.80 mmol), a 10% aqueous solution of sodium carbonate (1.3 mL, 13 mmol) and ethylene glycol dimethyl ether (65 mL) to give the title compound (2.0 g, 74%) as an oily product.
$^1$H-NMR (DMSO-$d_6$) δ : 1.12 (t, J=7.2 Hz, 3H), 1.44 (s, 6H), 3.74 (s, 2H), 4.16-4.20 (m, 4H), 6.89 (s, J=8.5 Hz, 2H), 7.33 (d, J=8.9 Hz, 1H), 7.43 (d, J=8.5 Hz, 2H), 7.53 (dd, J=7.7, 5.0 Hz, 1H), 7.74 (d, J=8.9 Hz, 1H), 7.82 (s, 1H), 8.18 (d, J=7.7 Hz, 1H), 8.79 (d, J=5.0 Hz, 1H)

<Example 122> 2-{3-Cyano-4-[2-methyl-2-(4-methoxyphenyl)propoxy]phenyl}nicotinic acid

The same operation as in Example 43 was carried out starting from ethyl 2-{3-cyano-4-[2-methyl-2-(4-methoxyphenyl)propoxy]phenyl}nicotinate (2.1 g, 4.8 mmol), a 1 mol/L aqueous solution of sodium hydroxide (5.8 mL, 5.8 mmol) and ethanol (60 mL) to give the title compound (1.6 g, 82%) as crystals.
Mp. 183-184 ˚C. $^1$H-NMR (DMSO-$d_6$) δ : 1.44 (s, 6H), 3.74 (s, 3H), 4.16 (s, 2H), 6.89 (d, J=8.8 Hz, 2H), 7.32 (d, J=8.8 Hz, 1H), 7.43 (d, J=8.8 Hz, 2H), 7.50 (dd, J=7.8, 4.8 Hz, 1H), 7.79 (dd, J=8.8, 2.1 Hz, 1H), 7.83 (d, J=2.1 Hz, 1H), 8.17 (d, J=7.8 Hz, 1H), 8.75 (d, J=4.8 Hz, 1H), 13.30-13.40 (br, 1H).

<Example 123> Ethyl 2-[3-cyano-4-(4-tert-butyl-phenethyloxy)phenyl]nicotinate

The same operation as in Example 2 was carried out starting from ethyl 2-(3-cyano-4-hydroxyphenyl)nicotinate hydrochloride (Referential Example 6a) (2.0 g, 6.6 mmol), 4-tert-butylphenethyl methanesulfonate (2.0 g, 7.9 mmol), potassium carbonate (2.1 g, 15 mmol) and DMF (100 mL) to give the title product (1.5 g, 55%) as an oily product.
$^1$H-NMR (DMSO-$d_6$) δ : 1.11 (t, J=7.2 Hz, 3H), 1.27 (s, 9H), 3.07 (t, J=6.6 Hz, 2H), 4.18 (q, J=7.2 Hz, 2H), 4.38 (t, J=6.6 Hz, 2H), 7.29-7.37 (m, 5H), 7.53-7.55 (m, 1H), 7.71 (d, J=8.5 Hz, 1H), 7.84 (s, 1H), 8.18-8.20 (m, 1H), 8.79-8.80 (m, 1H).

<Example 124> 2-[3-Cyano-4-(4-tert-butylphenethyloxy)-phenyl]nicotinic acid

The same operation as in Example 43 was carried out starting from ethyl 2-[3-cyano-4-(4-tert-butylphenethyloxy)- phenyl]nicotinate (1.5 g, 3.6 mmol), a 1 mol/L aqueous solution of sodium hydroxide (4.3 mL, 4.3 mmol) and ethanol (40 mL) to give the title compound (0.73 g, 50%) as crystals.
Mp. 185-186 ˚C. $^1$H-NMR (DMSO-$d_6$) δ : 1.27 (s, 9H), 3.07 (t, J=6.7 Hz, 2H), 4.37 (t, J=6.7 Hz, 2H), 7.30-7.35 (m, 5H), 7.50 (dd, J=7.6, 4.9 Hz, 1H), 7.79-7.82 (m, 1H), 7.85 (s, 1H), 8.16 (d, J=7.6 Hz, 1H), 8.75 (d, J=4.9 Hz, 1H), 13.20-13.40 (br, 1H).

<Example 125> Ethyl 2-{3-cyano-4-[4-(2-trifluoromethylphenyl)butoxy]phenyl}nicotinic acid

The same operation as in Example 2 was carried out starting from ethyl 2-(3-cyano-4-hydroxyphenyl)nicotinate hydrochloride (Referential Example 6a) (2.0 g, 6.6 mmol), 4-(2-trifluoromethylphenyl)butyl methanesulfonate (2.3 g, 7.9 mmol), potassium carbonate (2.1 g, 15 mmol) and DMF (100 mL) to give the title product (2.8 g, 93%) as an oily product.
$^1$H-NMR (DMSO-$d_6$) δ 1.11 (t, J=7.3 Hz, 3H), 1.78-1.83 (m, 2H), 1.86-1.90 (m, 2H), 2.85 (t, J=7.8 Hz, 2H), 4.18 (q, J=7.3 Hz, 2H), 4.26 (t, J=5.9 Hz, 2H), 7.34 (d, J=8.8 Hz, 1H), 7.40-7.42 (m, 1H), 7.52-7.55 (m, 2H), 7.60-7.62 (m, 1H), 7.78 (d, J=7.9 Hz, 1H), 7.78 (dd, J=8.8, 1.9 Hz, 1H), 7.84 (d, J=1.9 Hz, 1H), 8.20 (d, J=7.6 Hz, 1H), 8.81 (d, J=4.5 Hz, 1H).

<Example 126> 2-{3-Cyano-4-[4-(2-trifluoromethylphenyl)butoxy]phenyl}nicotinic acid

The same operation as in Example 43 was carried out starting from ethyl 2-{3-cyano-4-[4-(2-trifluoromethylphenyl)butoxy]phenyl}nicotinate (2.8 g, 6.0 mmol), a 1 mol/L aqueous solution of sodium hydroxide (7.3 mL, 7.3 mmol) and ethanol (70 mL) to give the title compound (2.3 g, 86%) as crystals.
Mp. 134-135 ˚C. $^1$H-NMR (DMSO-$d_6$) δ : 1.79-1.81 (m, 2H), 1.86-1.88 (m, 2H), 2.84 (t, J=7.5 Hz, 2H), 4.24 (t, J=5.6 Hz, 2H), 7.33 (d, J=8.9 Hz, 1H), 7.41-7.43 (m, 1H), 7.49-7.53 (m, 2H), 7.61-7.62 (m, 1H), 7.68 (d, J=7.8 Hz, 1H), 7.82-7.85 (m, 2H), 8.17 (d, J=8.0 Hz, 1H), 8.76 (d, J=2.8 Hz, 1H), 13.37 (s, 1H).

<Example 127> Ethyl 2-[3-cyano-4-(3,4-dimethoxy-phenethyloxy)phenyl]nicotinate

The same operation as in Example 2 was carried out starting from ethyl 2-(3-cyano-4-hydroxyphenyl)nicotinate hydrochloride (Referential Example 6a) (1.5 g, 4.9 mmol), 3,4-dimethoxyphenethyl methanesulfonate (1.5 g, 5.9 mmol), potassium carbonate (1.6 g, 11 mmol) and DMF (100 mL) to give the title product (2.1 g, 99%) as an oily product.
$^1$H-NMR (DMSO-$d_6$) δ : 1.11 (t, J=7.2 Hz, 3H), 3.05 (t, J=6.6 Hz, 2H), 3.73 (s, 3H), 3.78 (s, 3H), 4.18 (q, J=7.2 Hz, 2H), 4.37 (t, J=6.6 Hz, 2H), 6.88-6.90 (m, 2H), 7.00 (s, 1H), 7.35 (d, J=8.9 Hz, 1H), 7.53 (dd, J=7.9, 4.6 Hz, 1H), 7.77 (dd, J=8.9, 2.3 Hz, 1H), 7.84 (d, J=2.3 Hz, 1H), 8.19 (dd, J=7.9, 1.6 Hz, 1H), 8.80 (dd, J=4.6, 1.6 Hz, 1H).

<Example 128> 2-[3-Cyano-4-(3,4-dimethoxyphenethyl-oxy)phenyl]nicotinic acid

The same operation as in Example 43 was carried out starting from ethyl 2-[3-cyano-4-(3,4-dimethoxyphenethyloxy)phenyl]nicotinate (2.1 g, 4.9 mmol), a 1 mol/L aqueous solution of sodium hydroxide (5.8 mL, 5.8 mmol) and ethanol (60 mL) to give the title compound (1.34 g, 74%) as crystals.
Mp. 146-147 ˚C. $^1$H-NMR (DMSO-$d_6$) δ : 3.04 (t, J=6.3 Hz, 2H), 3.72 (s, 3H), 3.77 (s, 3H), 4.36 (t, J=6.3 Hz, 2H), 6.85-6.87 (m, 2H), 7.00 (s, 1H), 7.35 (d, J=8.8 Hz, 1H), 7.56 (dd, J=7.7, 4.8 Hz, 1H), 7.81 (d, J=8.8 Hz, 1H), 7.86 (s, 1H), 8.17 (d, J=7.7 Hz, 1H), 8.76 (d, J=4.8 Hz, 1H), 13.30-13.40 (br, 1H).

<Example 129> Ethyl 2-[3-cyano-4-(2,5-dimethyl-phenethyloxy)phenyl]nicotinate

The same operation as in Example 2 was carried out starting from ethyl 2-(3-cyano-4-hydroxyphenyl)nicotinate hydrochloride (Referential Example 6a) (2.0 g, 6.6 mmol), 2,5-dimethylphenethyl methanesulfonate (1.8 g, 7.9 mmol), potassium carbonate (2.1 g, 15 mmol) and DMF (70 mL) to give the title product (2.5 g, 96%) as an oily product.
$^1$H-NMR (DMSO-$d_6$) δ : 1.11 (t, J=7.2 Hz, 3H), 2.25 (s, 3H), 2.31 (s, 3H), 3.07 (t, J=6.7 Hz, 2H), 4.18 (q, J=7.2 Hz, 2H), 4.37 (t, J=6.7 Hz, 2H), 6.94 (d, J=7.4 Hz, 1H), 7.05 (d, J=7.4 Hz, 1H), 7.14 (s, 1H), 7.36 (d, J=8.8 Hz, 1H), 7.53 (dd, J=7.8, 4.7 Hz, 1H), 7.76 (dd, J=8.8, 2.3 Hz, 1H), 7.83 (d, J=2.3 Hz, 1H), 8.19 (dd, J=7.8, 1.6 Hz, 1H), 8.79 (dd, J=4.7, 1.6 Hz, 1H).

<Example 130> 2-[3-Cyano-4-(2,5-dimethylphenethyloxy)-phenyl]nicotinic acid

The same operation as in Example 43 was carried out starting from ethyl 2-[3-cyano-4-(2,5-dimethylphenethyloxy)-phenyl]nicotinate (2.5 g, 6.3 mmol), a 1 mol/L aqueous solution of sodium hydroxide (7.6 mL, 7.6 mmol) and ethanol (70 mL) to give the title compound (2.0 g, 84%) as crystals.
Mp. 193-194 ˚C. $^1$H-NMR (DMSO-$d_6$) δ : 2.25 (s, 3H), 2.32 (s, 3H), 3.07 (t, J-6.7 Hz, 2H), 4.36 (t, J=6.7 Hz, 2H), 6.95

(d, J=8.1 Hz, 1H), 7.06 (d, J=8.1 Hz, 1H), 7.15 (s, 1H), 7.37 (d, J=8.8 Hz, 1H), 7.50 (dd, J=7.8, 4.8 Hz, 1H), 7.80 (d, J=8.8 Hz, 1H), 7.45 (s, 1H), 8.1.6 (dd, J=7.8, 1.5 Hz, 1H), 8.25 (dd, J=4.8, 1.5 Hz, 1H), 13.30-14.40 (br, 1H).

<Example 131> Ethyl 2-{3-cyano-4-[2,2-dimethyl-3-(2-trifluoromethylphenyl)propoxy]phenyl}nicotinate

The same operation as in Example 5a was carried out starting from ethyl 2-chloronicotinate (Referential Example 4a) (0.9 g, 4.9 mmol), 3-cyano-4-[2,2-dimethyl-3-(2-trifluoromethylphenyl)propoxy]phenylboronic acid (2.1 g, 5.4 mmol), palladium acetate (67 mg, 0.30 mmol), tris(2-methylphenyl) phosphine (0.18 g, 0.6 mmol), a 10% aqueous solution of sodium carbonate (9.2 mL, 8.9 mmol) and ethylene glycol dimethyl ether (45 mL) to give the title compound (1.4 g, 62%) as an oily product.
$^1$H-NMR (DMSO-$d_6$) δ : 1.03 (s, 6H), 1.13 (t, J=7.1 Hz, 3H), 2.99 (s, 2H), 3.97 (s, 2H), 4.19 (q, J=7.1 Hz, 2H), 7.37 (d, J=8.8 Hz, 1H), 7.43-7.46 (m, 2H), 7.54-7.59 (m, 2H), 7.72 (d, J=7.8 Hz, 1H), 7.78-7.80 (m, 1H), 7.88 (d, J=1.9 Hz, 1H), 8.21 (d, J=7.7 Hz, 1H), 8.81 (d, J=3.7 Hz, 1H).

<Example 132> 2-{3-Cyano-4-[2,2-dimethyl-3-(2-trifluoromethylphenyl)propoxy]phenyl}nicotinic acid

The same operation as in Example 43 was carried out starting from ethyl 2-{3-cyano-4-[2,2-dimethyl-3-(2-trifluoromethylphenyl)propoxy]phenyl}nicotinate (2.5 g, 5.6 mmol), a 1 mol/L aqueous solution of sodium hydroxide (6.7 mL, 6.7 mmol) and ethanol (70 mL) to give the title compound (1.8 g, 76%) as crystals.
Mp. 149-150 °C. $^1$H-NMR (DMSO-$d_6$) δ : 1.03 (s, 6H), 3.00 (s, 2H), 3.97 (s, 2H), 7.36 (d, J=8.8 Hz, 1H), 7.46-7.52 (m, 3H), 7.59-7.63 (m, 1H), 7.72 (d, J=7.7 Hz, 1H), 7.85 (d, J=8.6 Hz, 1H), 7.89 (s, 1H), 8.19 (d, J=7.6 Hz, 1H), 8.78 (d, J=4.0 Hz, 1H), 13.25-13.40 (br, 1H).

<Example 133> Ethyl 2-{3-cyano-4-[4-(4-methoxyphenyl)-butoxy]phenyl}nicotinate

The same operation as in Example 2 was carried out starting from ethyl 2-(3-cyano-4-hydroxyphenyl)nicotinate hydrochloride (Referential Example 6a) (1.5 g, 4.9 mmol), 4-(4-methoxypheny)butyl methanesulfonate (1.5 g, 5.9 mmol), potassium carbonate (1.6 g, 11 mmol) and DMF (50 mL) to give the title product (1.8 g, 86%) as an oily product.
$^1$H-NMR (DMSO-$d_6$) δ : 1.11 (t, J=7.2 Hz, 3H), 1.73-1.78 (m, 4H), 2.61 (t, J=7.3 Hz, 2H), 3.72 (s, 3H), 4.16-4.23 (m, 4H), 6.85 (d, J=8.8 Hz, 2H), 7.32 (d, J=8.8 Hz, 2H), 7.32 (d, J=8.8 Hz, 1H), 7.54 (dd, J=7.7, 4.6 Hz, 1H), 7.77 (dd, J=8.8, 1.8 Hz, 1H), 7.84 (d, J=1.8 Hz, 1H), 8.19 (d, J=7.7 Hz, 1H), 8.80 (d, J=4.6 Hz, 1H).

<Example 134> 2-{3-Cyano-4-[4-(4-methoxyphenyl)-butoxy]phenyl}nicotinic acid

The same operation as in Example 43 was carried out starting from ethyl 2-{3-cyano-4-[4-(4-methoxyphenyl)-butoxy]phenyl}nicotinate (1.8 g, 4.2 mmol), a 1 mol/L aqueous solution of sodium hydroxide (5.1 mL, 5.1 mmol) and ethanol (50 mL) to give the title compound (1.4 g, 83%) as crystals.
Mp. 148-149 °C. $^1$H-NMR (DMSO-$d_6$) δ : 1.75-1.80 (m, 4H), 2.62 (t, J=7.1 Hz, 2H), 3.72 (s, 3H), 4.22 (t, J=5.5 Hz, 2H), 6.85 (d, J=8.2 Hz, 2H), 7.15 (d, J=8.2 Hz, 2H), 7.32 (d, J=8.8 Hz, 1H), 7.50 (dd, J=7.5, 4.9 Hz, 1H), 7.81-7.85 (m, 2H), 8.17 (d, J=7.5 Hz, 1H), 8.76 (d, J=4.9 Hz, 1H), 13.30-13.40 (br, 1H).

<Example 135> Ethyl 2-[3-cyano-4-(2,2-dimethylpropyl-amino)phenyl]nicotinate

Neopentylamine (2.2 mL, 19 mmol) was added to a solution of ethyl 2-(3-cyano-4-fluorophenyl)nicotinate (2.0 g, 7.4 mmol) in DMSO (20 mL) followed by stirring at 40°C for 24 hours. The reaction mixture was poured into ice water and extracted with ethyl acetate. The organic layer was washed with water and dried, the solvent was evaporated therefrom *in vacuo* and the resulting residue was purified by silica gel column chromatography (hexane-ethyl acetate = 4/1) to give the title compound (1.6 g, 66%) as an oily product.
$^1$H-NMR (DMSO-$d_6$) δ : 0.94 (s, 9H), 1.13 (t, J=7.0 Hz, 3H), 3.12 (d, J=6.5 Hz, 2H), 4.19 (q, J=7.0 Hz, 2H), 6.07 (t, J=6.5 Hz, 1H), 7.01 (d, J=9.0 Hz, 1H), 7.43 (dd, J=7.7, 4.8 Hz, 1H), 7.54 (d, J=9.0 Hz, 1H), 7.59 (s, 1H), 8.08 (d, J=7.7 Hz, 1H), 8.73 (d, J=4.8 Hz, 1H).

<Example 136> 2-[3-Cyano-4-(2,2-dimethylpropylamino)-phenyl]nicotinic acid

The same operation as in Example 43 was carried out starting from ethyl 2-[3-cyano-4-(2,2-dimethylpropylamino)- phenyl]nicotinate (1.7 g, 5.0 mmol), a 1 mol/L aqueous solution of sodium hydroxide (5.9 mL, 5.9 mmol) and ethanol (60 mL) to give the title compound (1.0 g, 66%) as crystals.
Mp. 203-204 °C. $^1$H-NMR (DMSO-$d_6$) δ : 0.94 (s, 9H), 3.11 (d, J=5.4 Hz, 2H), 6.01 (t, J=5.4 Hz, 1H), 7.01 (d, J=9.0 Hz,

1H), 7.40 (dd, J=7.7, 4.8 Hz, 1H), 7.61-7.63 (m, 2H), 8.06 (d, J=7.7 Hz, 1H), 8.70 (d, J=4.8 Hz, 1H), 13.20-13.30 (br, 1H).

<Example 137> Ethyl 2-(4-azepan-1-yl-3-cyanophenyl)-nicotinate

The same operation as in Example 135 was carried out starting from ethyl 2-(3-cyano-4-fluorophenyl)nlcotinate (2.0 g, 7.4 mmol), hexamethyleneimine (2.1 mL, 19 mmol) and DMSO (10 mL) to give the title compound (2.5 g, 96%) as an oily product.
$^1$H-NMR (DMSO-$d_6$) δ : = 1.16 (t, J=7.2 Hz, 3H), 1.54-1.56 (m, 4H), 1.81-1.83 (m, 4H), 3.67-3.69 (m, 4H), 4.22 (q, J=7.2 Hz, 2H), 7.04 (d, J=9.1 Hz, 1H), 7.45 (dd, J=7.8, 4.7 Hz, 1H), 7.59 (dd, J-9.1, 2.2 Hz, 1H), 7.64 (d, J=2.2 Hz, 1H), 8.11 (d, J=7.8 Hz, 1H), 8.75 (d, J=4.7 Hz, 1H).

<Example 138> 2-(4-Azepan-1-yl-3-cyanophenyl)-nicotinic acid

The same operation as in Example 43 was carried out starting from ethyl 2-(4-azepan-1-yl-3-cyanophenyl)-nicotinate (2.5 g, 7.1 mmol), a 1 mol/L aqueous solution of sodium hydroxide (8.5 mL, 8.5 mmol) and ethanol (80 mL) to give the title compound (2.2 g, 96%) as crystals.
Mp. 174-175 ˚C. $^1$H-NMR (DMSO-$d_6$) δ : 1.54-1.56 (m, 4H), 1.81-1.83 (m, 4H), 3.65-3.68 (m, 4H), 7.03 (d, J=9.1 Hz, 1H), 7.42 (dd, J=7.7, 4.7 Hz, 1H), 7.64-7.69 (m, 2H), 8.08 (dd, J=7.7, 1.0 Hz, 1H), 8.71 (dd, J=4.7, 1.0 Hz, 1H), 13.25-13.40 (br, 1H).

<Example 139> Ethyl 2-[3-cyano-4-(2,2-dimethyl-propoxy)phenyl]-5-fluoronicotinate

The same operation as in Example 5a was carried out starting from ethyl 2-chloro-5-fluoronicotinate (1.5 g, 7.7 mmol), 3-cyano-4-(2,2-dimethylpropoxy)phenylboronic acid (2.3 g, 10 mmol), palladium acetate (0.10 g, 0.46 mmol), tris(2-methylphenyl) phosphine (0.28 g, 0.92 mmol), a 10% aqueous solution of sodium carbonate (15 mL, 4 mmol) and ethylene glycol dimethyl ether (75 mL) to give the title compound (1.2 g, 47%) as an oily product.
$^1$H-NMR (DMSO-$d_6$) δ : 1.05 (s, 9H), 1.14 (t, J=7.2 Hz, 3H), 3.87 (s, 2H), 4.20 (q, J=7.2 Hz, 2H), 7.32 (d, J=8.9 Hz, 1H), 7.74 (dd, J=8.9, 2.1 Hz, 1H), 7.82 (d, J=2.1 Hz, 1H), 8.15 (dd, J=8.6, 2.7 Hz, 1H), 8.84 (d, J=2.7 Hz, 1H).

<Example 140> 2-[3-Cyano-4-(2,2-dimethylpropoxy)-phenyl]-5-fluoronicotinic acid

The same operation as in Example 43 was carried out starting from ethyl 2-[3-cyano-4-(2,2-dimethylpropoxy)-phenyl]-5-fluoronicotinate (1.3 g, 3.6 mmol), a 1 mol/L aqueous solution of sodium hydroxide (4.3 mL, 4.3 mmol) and ethanol (40 mL) to give the title compound (0.9 g,75%) as crystals.
Mp. 199-200 ˚C. $^1$H-NMR (DMSO-$d_6$) δ : 1.06 (s, 9H), 3.86 (s, 2H), 7.31 (d, J=8.8 Hz, 1H), 7.79 (dd, J=8.8, 2.1 Hz, 1H), 7.84 (d, J=2.1 Hz, 1H), 8.11 (dd, J=8.7, 2.7 Hz, 1H), 8.80 (d, J=2.7 Hz, 1H), 13.60-13.80 (br, 1H).

<Example 141> Ethyl 2-[4-(N-mothylbutylamino)-3-cyanophenyl]nicotinate

The same operation as in Example 135 was carried out starting from ethyl 2-(3-cyano-4-fluorophenyl)nicotinate (2.0 g, 7.4 mmol), N-methylbutylamine (2.2 mL, 19 mmol) and DMSO (10 mL) to give the title compound (1.8 g, 72%) as an oily product.
$^1$H-NMR (DMSO-$d_6$) δ : 0.91 (t, J=7.3 Hz, 3H), 1.13 (t, J=7.2 Hz, 3H), 1.28-1.33 (m, 2H), 1.58-1.64 (m, 2H), 3.04 (s, 3H), 3.47 (t, J=7.5 Hz, 2H), 4.20 (q, J=7.2 Hz, 2H), 7.08 (d, J=9.0 Hz, 1H), 7.48 (dd, J=7.8, 4.6 Hz, 1H), 7.62 (dd, J=9.0, 2.3 Hz, 1H), 7.68 (d, J=2.3 Hz, 1H), 8.13 (dd, J=7.8, 1.7 Hz, 1H), 8.77 (dd, J=4.6, 1.7 Hz, 1H)

<Example 142> 2-[4-(N-Methylbutylamino)-3-cyano-phenyl]nicotinic acid

The same operation as in Example 43 was carried out starting from ethyl 2-[4-(N-methylbutylamino)-3-cyano-phenyl]nicotinate (1.8 g, 5.4 mmol), a 1 mol/L aqueous solution of sodium hydroxide (6.4 mL, 6.4 mmol) and ethanol (64 mL) to give the title compound (1.2 g,72%) as crystals.
Mp. 172-173 ˚C. $^1$H-NMR (DMSO-$d_6$) δ : 0.92 (t, J=7.3 Hz, 3H), 1.30-1.34 (m, 2H), 1.59-1.65 (m, 2H), 3.04 (s, 3H), 3.46 (t, J=7.2 Hz, 2H), 7.70 (d, J=8.9 Hz, 1H), 7.44 (dd, J=7.7, 4.3 Hz, 1H), 7.69 (d, J=8.9 Hz, 1H), 7.73 (s, 1H), 8.10 (d, J=7.7 Hz, 1H), 8.73 (d, J=4.3 Hz, 1H), 13.25-13.40 (br, 1H).

Example 143

Test for xanthine oxidase inhibitory activity

Evaluation of inhibitory activity of the present invention to xanthine oxidase was conducted using xanthine as a substrate by measuring the amount of uric acid produced by xanthine oxidase which is an oxidizing enzyme therefor. Thus, xanthine oxidase (0.01 unit/L, 20 μL/well; derived from milk; manufactured by Sigma), diethylenetriamine pentaacetate (0.01 mol/L, 20 μL/well), phosphate buffer (20 μL/well), distilled water (100 μL/well) and a diluted solution (20 μL/well) of a test substance were mixed in a 96-well quartz microplate. After preincubating for 5 minutes in a microplate spectrophotometer warmed at 37°C, xanthine (1 mmol/L, 20 μL/well) was added. The changes with elapse of time in OD 292 nm based on the production of uric acid was measured using a microplate spectrophotometer warmed at 37°C whereby the initial reaction velocity was measured. Xanthine oxidase inhibitory activity was calculated by the following formula and concentration of the test substance for 50% suppression ($IC_{50}$ value) was calculated.

Inhibition Rate (%) = {[(Initial Reaction Velocity of the Reaction Control) – (Initial Reaction Velocity upon Addition of Test Substance)] / [Initial Reaction Velocity of the Reaction Control]} × 100

An example of the results is shown in Table 15. The compounds of the present invention showed an excellent inhibitory activity in the test for xanthine oxidase inhibitory activity.

[Table 15.]

| Test Substance (Example No.) | $IC_{50}$ (nM) | Test Substance (Example No.) | $IC_{50}$ (nM) |
|---|---|---|---|
| Example 44 | 91 | Example 81 | 16 |
| Example 46 | 83 | Example 82 | 22 |
| Example 47 | 24 | Example 83 | 42 |
| Example 48 | 38 | Example 84 | 18 |
| Example 49 | 63 | Example 86 | 99 |
| Example 50 | 83 | Example 88 | 47 |
| Example 53 | 58 | Example 90 | 37 |
| Example 56 | 36 | Example 100 | 65 |
| Example 59 | 42 | Example 102 | 53 |
| Example 60 | 49 | Example 104 | 13 |
| Example 61 | 56 | Example 106 | 25 |

| | | | |
|---|---|---|---|
| Example 62 | 27 | Example 112 | 28 |
| Example 63 | 97 | Example 116 | 52 |
| Example 64 | 33 | Example 118 | 12 |
| Example 65 | 34 | Example 120 | 35 |
| Example 69 | 58 | Example 122 | 91 |
| Example 71 | 90 | Example 126 | 26 |
| Example 72 | 28 | Example 130 | 72 |
| Example 73 | 21 | Example 132 | 15 |
| Example 75 | 24 | Example 134 | 18 |
| Example 76 | 17 | Example 138 | 34 |
| Example 77 | 24 | Example 140 | 40 |
| Example 80 | 38 | | |

Example 144

Test using model rats of fructose-loaded hypertriglyceridemia

The test was conducted according to a method of Kusama, et al. (Folia Pharmacologica Japonica, volume 92, pages 175 to 180, 1988). Thus, male rats of SD strain of 6 to 7 weeks age were made in free access to a 75% (w/v) D (-)-fructose solution for three days. During the period of fructose ingestion, a test substance (100 mg/kg) was suspended in 1% methyl cellulose and orally administered once daily. After 2 hours from the final administration, about 500 $\mu$L per rat of blood was collected using a hematocrit capillary from orbital venous plexus under anesthetization with ether and centrifuged (3000 rpm at 4°C for 20 minutes) whereupon the serum was prepared.

(1) Action for lowering of triglyceride in serum

The triglyceride (TG) in the serum was measured by a Triglyceride E-Test Wako (manufactured by Wako Pure Chemical Industries, Ltd.) and each TG lowering rate was determined by the following formula.

$$\text{TG Lowering Rate (\%)} = \{[(\text{TG Value of Control Animal}) - (\text{TG Value of Animal Administered with Test Substance})] / [(\text{TG Value of Control Animal}) - (\text{TG Value of Untreated Animal})]\} \times 100$$

(2) Action for lowering of uric acid in serum

Uric acid value in the serum was measured by a Uric acid C-Test Wako (manufactured by Wako Pure Chemical Industries, Ltd.) using the above serum and each uric acid lowering rate was determined by the following formula.

$$\text{Uric Acid Lowering Rate (\%)} = \{[(\text{Uric Acid Value of Control Animal}) - (\text{Uric Acid Value of Animals Administered with Test Substance})] / [\text{Uric Acid Value of Control Animal}]\} \times 100$$

Table 16 shows an example of the results where the serum triglyceride lowering action and the serum uric acid lowering action of the compounds of the present invention were tested using the model rats of hypertriglyceridemia loaded with fructose. Due to their excellent xanthine oxidase inhibitory action, the compounds of the present invention exhibited serum uric acid lowering action and also serum triglyceride lowering action. There are some cases that the triglyceride lowers due to an adverse action such as a reduction in body weight as a result of a decrease in the amount of feed but, in the compounds of the present invention, no such adverse action was noted.

[Table 16.]

| Example | Inhibition Rate (%) | |
|---|---|---|
| | Action for lowering of triglyceride | Action for lowering of uric acid |
| 47 | 46.3 | 52.6 |
| 56 | 72.1 | 38.7 |
| 59 | 53.2 | 29.3 |
| 62 | 71.1 | 43.6 |
| 72 | 34.2 | 48.5 |
| 75 | 25.7 | 49.5 |
| 77 | 39.6 | 27.8 |
| 78 | 21.6 | 19.4 |
| 83 | 112.2 | 56.6 |
| 84 | 41.4 | 69.2 |
| 100 | 40.8 | 58.2 |
| 102 | 28.4 | 25.2 |

**[Industrial Applicability]**

As will be apparent from the results of the above pharmacological test, the 2-phenylnicotinic acid derivatives according to the present invention exhibit a uric acid lowering action due to an excellent xanthine oxidase inhibitory action and further exhibit a hypolipemic action whereby their utility is very high as a treating or preventive agent for gout and hyperuricemia which are often accompanied by hyperlipemia as a complication.

## Claims

1. A 2-phenylnlcotinic acid derivative represented by the following formula (I) and pharmaceutically acceptable salt and hydrate thereof.

[chem. 4]

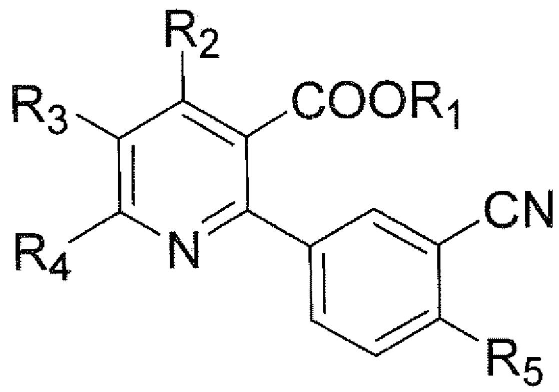

(I)

[In the formula, $R_1$, $R_2$ and $R_4$ are same or different and each is hydrogen or an alkyl group having 1 to 4 carbon (s); $R_3$ is hydrogen or halogen; $R_5$ is an azepanyl group, an amino group which is substituted with one or two alkyl group(s) having 1 to 4 carbon(s) or -O-X; and X is a substituent selected from the following (a) to (h).

(a) an alkyl group having 1 to 10 carbons(s),
(b) an alkyl group having 5 to 8 carbons and forming a saturated hydrocarbon ring having 3 to 6 carbons (which may have a phenyl group),
(c) an alkyl group having 1 to 4 carbon(s) which is substituted with a cycloalkyl group having 3 to 6 carbons,
(d) a phenyl-$C_{1-5}$ alkyl group which is optionally substituted with trifluoromethyl group, an alkyl group having 1 to 4 carbon(s), an alkoxy group having 1 to 4 carbon(s), halogen, methanesulfonyloxy group, nitro group, fluorophenyl group and/or hydroxyl group,
(e) a phenoxy-$C_{1-5}$ alkyl group which is optionally substituted with trifluoromethoxy group, phenoxy group and/or halogen,
(f) an oxazolyl-$C_{1-5}$ alkyl group which is substituted with an alkyl group having 1 to 4 carbon(s) and phenyl group,
(g) a benzoylamino-$C_{1-5}$ alkyl group which is substituted with trifluoromethoxy group or halogen and
(h) an amino alkyl group which is substituted with pyridyl group and an alkyl group having 1 to 4 carbon(s).

**2.** A pharmaceutical agent containing the 2-phenylnicotinic acid derivative or a pharmaceutically acceptable salt or hydrate thereof according to claim 1 as an effective ingredient.

**3.** A xanthine oxidase inhibitor containing the 2-phenylnicotinic acid derivative or a pharmaceutically acceptable salt or hydrate thereof according to claim 1 as an effective ingredient.

**4.** The pharmaceutical agent according to claim 2, wherein said agent is therapeutic or preventive agent for hyperuricemia.

**5.** The pharmaceutical agent according to claim 2, wherein said agent is therapeutic or preventive agent for gout.

**6.** A hypolipemic agent containing the 2-phenylnicotinic acid derivative or a pharmaceutically acceptable salt or hydrate thereof according to claim 1 as an effective ingredient.

**7.** The pharmaceutical agent according to claim 6, wherein said agent is therapeutic or preventive agent for hyperlipemia.

**8.** The pharmaceutical agent according to claim 6, wherein said agent is therapeutic or preventive agent for hypertriglyceridemia.

**9.** The pharmaceutical agent according to claim 2, wherein said agent has a hypouricemic action together with a hypolipemic action.

**INTERNATIONAL SEARCH REPORT**

International application No.
PCT/JP2007/073947

A. CLASSIFICATION OF SUBJECT MATTER
*C07D213/80*(2006.01)i, *A61K31/455*(2006.01)i, *A61K31/55*(2006.01)i, *A61P3/06* (2006.01)i, *A61P19/06*(2006.01)i, *A61P43/00*(2006.01)i, *C07D401/10* (2006.01)i, *C07D413/10*(2006.01)i

According to International Patent Classification (IPC) or to both national classification and IPC

B. FIELDS SEARCHED

Minimum documentation searched (classification system followed by classification symbols)
C07D213/80, A61K31/455, A61K31/55, A61P3/06, A61P19/06, A61P43/00, C07D401/10, C07D413/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2006/022374 A1 (ASTELLAS PHARMA INC., JP), 02 March, 2006 (02.03.06), All references (Family: none) | 1-9 |
| A | WO 2006/046593 A1 (DAIICHI SANKYO CO., LTD.), 04 May, 2006 (04.05.06), All references; particularly, illustrative compound 2-206 (Family: none) | 1-9 |
| A | WO 99/065897 A1 (CHIRON CORP.), 23 December, 1999 (23.12.99), All references; particularly, example 206 (Family: none) | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 March, 2008 (10.03.08) | 18 March, 2008 (18.03.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/073947

C (Continuation). DOCUMENTS CONSIDERED TO BE RELEVANT

| Category* | Citation of document, with indication, where appropriate, of the relevant passages | Relevant to claim No. |
|---|---|---|
| A | WO 2002/007673 A1 (BHATNAGAR P K), 31 January, 2002 (31.01.02), All references; particularly, examples 13 to 18 (Family: none) | 1-9 |
| A | TENENBAUM, A et al., Atherogenic dyslipidemia in metabolic syndrome and type 2 diabetes: therapeuticoptions beyond statins, Cardiovasc Diabetol, 2006.09.26, Vol.5, No.20 | 1-9 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 20060223374 A


### Non-patent literature cited in the description


- Guideline for the management of hyperuricemia and gout. Japanese Society of Gout, 2002
- *Annali di Chimica Applicata,* 1931, vol. 21, 553-558
- **Kusama et al.** *Folia Pharmacologica Japonica,* 1988, vol. 92, 175-180